# EUROPEAN PATENT SPECIFICATION

(11) **EP 4 320 138 B1**
(45) Date of publication and mention of the grant of the patent: **01.04.2026**
(21) Application number: 22721095.2
(22) Date of filing: 08.04.2022
(51) Int. Cl.: C07H 19/073, C07H 19/173, C07H 23/00, C07H 21/04, C07H 21/02

(54) **PSEUDO SOLID PHASE PROTECTING GROUP AND METHODS FOR THE SYNTHESIS OF OLIGONUCLEOTIDES AND OLIGONUCLEOTIDE CONJUGATES**
PSEUDOFESTPHASE-SCHUTZGRUPPE UND VERFAHREN ZUR SYNTHESE VON OLIGONUKLEOTIDEN UND OLIGONUKLEOTIDKONJUGATEN
GROUPE PROTECTEUR DE PSEUDO-PHASE SOLIDE ET PROCÉDÉS DE SYNTHÈSE D'OLIGONUCLÉOTIDES ET DE CONJUGUÉS D'OLIGONUCLÉOTIDES

(30) Priority: 09.04.2021 EP 21167583
(43) Date of publication of application: 14.02.2024
(73) Proprietor: Bachem AG, 4416 Bubendorf (CH)
(72) Inventor: OKADA, Yohei, Tokyo, Tokyo 183-0054 (JP); SUZUKI, Hideaki, Chigasaki Kanagawa, Kanagawa 253-0002 (JP); LUTHER, Anatol, 4416 Bubendorf (CH)
(74) Representative: Jacobi, Markus Alexander
(86) International application number: PCT/EP2022/059528
(87) International publication number: WO 2022/214692

(56) References cited:
- WO-A1-2021/038509
- US-A1- 2012 296 074

## Description

The present invention is a soluble support for producing oligonucleotides and a method for producing oligonucleotides using the soluble support.

The synthesis of nucleic acids commonly relies on solid-phase synthesis, where a growing oligonucleotide chain is built on a solid support by iterative coupling cycles. Different types of coupling chemistry have been developed, among which the phosphoramidite method is most popular. A solid-phase synthesis process using the phosphoramidite method has been optimized and automated. Thus, the solid-phase synthesis process is advantageous in speed and is used most widely. However, the upscaling of the solid-phase synthesis process is difficult for several reasons, including excessive consumption of reagents and raw materials. A further disadvantage of the solid-phase synthesis process is that it is difficult to follow the progress of the reaction in real time and to analyze the structure of an intermediate.

In recent years, a liquid-phase synthesis process using a soluble support, which is also called as a pseudo solid phase protecting group, has been developed. Specifically, synthesis processes which use a long-chain hydrocarbon-containing group as a pseudo solid phase-protecting group have been reported (for example, JP5548852, WO2012/157723, WO2020/017085). Long-chain hydrocarbon-containing groups have also been reported for use in organic synthesis and peptide synthesis (for example, WO2007/122847 and WO2007/034812).

JP5548852 discloses a hydrophobic group bonded nucleoside and a hydrophobic group-bonded oligonucleotide synthetic method. The patent discloses a method for synthesizing a hydrophobic group bonded nucleoside, in which 5'-OH protected nucleoside, 5'-O-(1,1-bis(4-methoxyphenyl)-1-phenylmethyl) thymidine is reacted with succinic anhydride, resulting in 3'-O-succinyl-5'-O-(1,1-bis(4-methoxyphenyl)-1-phenylmethyl) thymidine, and then the resultant is reacted with 3,4,5-tris(octadecyloxy) benzoyl) piperazine, resulting in 3'-O-(4-(3,4,5-tris(octadecyloxy)benzoyl)piperazine-1-ylsuccinyl)-5'-O-(4 and 4'-dimethoxytrityl) thymidine.

In the hydrophobic group bonded nucleoside, a hydrophobic aromatic ring is bonded to a linker via an amide bond and the linker is bonded to the 3'-OH position of a sugar via a succinyl group or the like. Another hydrophobic group bonded nucleoside is disclosed in Matsuno et al. (Organic Letters, 2016, 18, pp. 800-803). Matsuno discloses the hydrophobic group similar to that of JP5548852, and has the structure of carbonyl(4-carbonylbenzyloxy) instead of succinyl group to provide selective cleavage. Such a hydrophobic group can be selectively cleaved via hydrogenation from oligonucleotides bearing 5'-DMTr, 2-CE, and/or 2'-TBS groups.

WO 2012/157723 discloses a pseudo solid phase protecting group bonded to a nucleoside and a method of producing an oligonucleotide using the pseudo solid phaseprotecting group. The pseudo solid phase protecting group is shown as "-L-Y-Z." L is shown as "**CO-L1-L2-CO*" and a succinyl group is exemplified, Y means oxygen atom or NR, and Z is a hydrophobic aromatic ring.

WO2020/017085 discloses another pseudo solid phase protecting group which is easily selectively removed in a short time and particularly without using a toxic heavy metal or dangerous hydrogen gas in a liquid phase synthesis. The linkage to the nucleoside's 3'-hydroxyl group is established via an ester bond.

There is still a need to provide further pseudo solid phase protecting groups, which are suitable for oligonucleotide synthesis. In particular, such a protecting group should be simple and cost-efficient to produce. It should avoid premature cleavage from the growing oligonucleotide chain, but be amenable to complete cleavage under suitable conditions. Such suitable conditions may comprise treatment with a base. The present invention provides such pseudo solid phase protecting groups.

The following terms and expressions are used throughout the present application. Further terms and expressions are explained in the detailed disclosure of the invention. Any and all of these definitions are applicable throughout the present application, unless indicated differently in the context of specific embodiments.

As used herein, the indefinite articles "a" and "an" may mean "at least one".

As used herein, the expression "hydroxyl group" generally refers to a structure of the formula -OH. The expression "hydroxyl moiety" may refer to a hydroxyl group or to a residue of a hydroxyl group, which results from a reaction of the hydroxyl group with another chemical group.

Hence, a hydroxyl moiety may have a structure of the formula -O- or -OH. The expressions "amine group" (e.g. -NH-) and "amine moiety" (e.g. =N- or -NH-) are used analogously.

As used herein, the expressions a "C₁₋₃₀ group", "C1 to C30 group", "C1-C30 group" or "chemical group having 1 to 30 carbon atoms" are used interchangeably. They all refer to a chemical moiety, which comprises between 1 and 30 carbon atoms, but not more or less carbon atoms. In addition, the group may comprise additional atoms, e.g. hydrogen atoms in the case of a C1 to C30 alkyl group. As a further example, a "C1 to C30 hydrocarbon group substituted with a C1-C30 heteroalkyl group" may comprise in total between 2 and 60 carbon atoms, but not more or less carbon atoms.

The term "protecting group" as used herein may be understood in the broadest sense as a group which is introduced into a molecule by chemical modification of a functional group to block said group from reaction in subsequent process steps, e.g. to prevent side reactions at the backbone or the bases of the oligonucleotide.

A molecule, to which a pseudo solid phase protecting group (PSPPG) is bound, may herein be referred to as a "PSPPG protected molecule", e.g. a PSPPG protected nucleoside. A pseudo solid phase protecting group (PSPPG) as used herein, is a protecting group, which enables the separation of said PSPPG protected molecule from other components of a liquid composition. This is often achieved by influencing the solubility of the PSPPG protected molecule, so as to facilitate, e.g., precipitation steps. For the synthesis of oligonucleotides, a PSPPG may comprise hydrophobic moieties, in particular long-chain hydrocarbon moieties and aromatic moieties.

Nucleosides or nucleoside units may be glycosylamines comprising a sugar moiety, commonly a (deoxy)ribose moiety, and a nucleobase. The expression nucleoside unit may be used to refer to nucleoside moieties in the context of a larger moiety, e.g. as part of an oligonucleotide. As used herein, the expressions nucleoside and nucleoside unit encompass naturally occurring nucleosides as well as non-natural compounds, where the ribose moiety and/or the nucleobase has been modified or replaced by a functional equivalent or an abasic site. Examples of natural nucleosides comprise, but are not limited to, adenosine, guanosine, cytidine, ribothymidine, uridine, deoxyuridine, deoxythymidine, inosine, deoxyadenosine, deoxyguanosine, deoxycytidine, and methylated derivatives thereof. Further examples are queusine acheaeosine, wybutosine, lysidine, and N6-threonylcarbamoyladenosine. Non-natural nucleosides may comprise altered ribose moieties, which may, e.g., be "locked" (e.g. comprise a methylene bridge connecting the 2'-oxygen and 4'-carbon), exhibit ethylene bridges, or carry -F, -OMe (-O-CH₃), and/or -methoxyethyloxy (-O-CH₂-CH₂-O-CH₃, aka. MOE, -O-methoxyethyl) substituents. Further, the ribose moiety may be replaced by a functional equivalent, e.g. by another pentose such as arabinose, a hexose such as mannose, morpholine derivatives, or a glycerol moiety. As used herein, the expression nucleobase encompasses both non-natural nucleobases and naturally occurring nucleobases such as, e.g., adenine, guanine, uracil, cytosine, and thymine. Non-natural nucleobases are functional equivalents of natural nucleobases in that they are capable of a specific interaction with a complementary nucleobase. The interaction between complementary nucleobases may be mediated by hydrogen bonds, which is known as Watson-Crick base pairing. Such non-natural nucleobases may be derivatives of purine or pyrimidine, which are capable of a specific interaction with another nucleobase.

Nucleotides or nucleotide units may comprise a nucleoside and a phosphate moiety.

As used herein, the expression oligonucleotide/s is used in a most general way to relate to any oligomers comprising at least two nucleosides linked by a phosphodiester bond or by an analogous structure as shown, e.g., in formulae A-1 and A-2 below, and any isomeric forms and stereoisomers thereof. In a preferred embodiment, an oligonucleotide in the sense of the present invention comprises two or more nucleoside moieties conjugated with each other via a linker comprising or consisting of a structure according to formula A-1 or formula A-2 or a salt thereof: 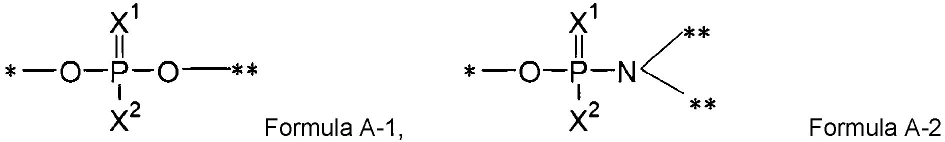 wherein, in formula A-1 and formula A-2:
* and ** each independently from another denotes a point of attachment to a nucleoside moiety (i.e., the antecedent and following nucleoside moiety, respectively) or H (i.e., in case of a terminal end of the oligonucleotide);
X¹ is selected from S and O; and
X² can be any conceivable electron withdrawing group, and is preferably selected from the group consisting of -OH, -OR, -NHR, -NR₂, an C3-C20-(hetero)aromatic moiety, -F, -Cl, -SH, -SeH, and -SR,
where R is any residue, preferably a residue not having more than 30 carbon atoms, preferably a residue selected from the group consisting of a branched, unbranched or cyclic C1-C20 (hetero)alkyl residue, a branched, unbranched or cyclic C2-C20 (hetero)alkenyl residue, a branched, unbranched or cyclic C2-C20 (hetero)alkynyl residue, a C6-C20 aryl residue, a C3-C19 heteroaryl residue, wherein the aforementioned residues are optionally substituted with deuterium, halogen, a branched, unbranched or cyclic C1-C20 (hetero)alkyl residue, a branched, unbranched or cyclic C2-C20 (hetero)alkenyl residue, a branched, unbranched or cyclic C2-C20 (hetero)alkynyl residue, a C1-C20 (hetero)cycloalkyl residue, a C6-C20 aryl residue, and/or a C3-C19 heteroaryl residue.

As used throughout the present invention, the terms "alkyl", "or "heteroalkyl" may be understood in the broadest sense. It may for instance be methyl or ethyl. A heteroalkyl may comprise at least one heteroatom such as, e.g. N, O, S or P. This may also embrace alkoxy (e.g., methoxy (-O-CH₃, -OMe)) and similar residues such as -NHCH₃ or -N(CH₃)₂ or salts thereof. An alkyl may be a branched, unbranched or cyclic C1-C40, C1-C30, or C1-C20 (hetero)alkyl, optionally substituted with deuterium, halogen, a branched, unbranched or cyclic C1-C40, C1-C30, or C1-C20 (hetero)alkyl residue, a branched, unbranched or cyclic C2-C40, C2-C30, or C2-C20 (hetero)alkenyl residue, a branched, unbranched or cyclic C2-C40, C2-C30, C2-C20 (hetero)alkynyl residue, a C1-C20 (hetero)cycloalkyl residue, a C6-C20 aryl residue, and/or with a C3-C19 heteroaryl residue.

As used throughout the present application, the terms "alkenyl" and "heteroalkenyl" may be understood in the broadest sense any may have one, two or more C-C double bonds. A heteroalkenyl may comprise at least one heteroatom such as, e.g. N, O, S or P. Preferably, an alkenyl is a branched, unbranched or cyclic C1-C20 (hetero)alkenyl, optionally substituted with deuterium, halogen, a branched, unbranched or cyclic C1-C20 (hetero)alkyl residue, a branched, unbranched or cyclic C2-C20 (hetero)alkenyl residue, a branched, unbranched or cyclic C2-C20 (hetero)alkynyl residue, a C1-C20 (hetero)cycloalkyl residue, a C6-C20 aryl residue, and/or with a C3-C19 heteroaryl residue.

As used throughout the present invention, the terms "alkynyl" and "heteroalkynyl" may be understood in the broadest sense any may have one, two or more triple C-C bonds. A heteroalkynyl may comprise at least one heteroatom such as, e.g. N, O, S or P. Preferably, an alkynyl is a branched, unbranched or cyclic C1-C20 (hetero)alkynyl, optionally substituted with deuterium, halogen, a branched, unbranched or cyclic C1-C20 (hetero)alkyl residue, a branched, unbranched or cyclic C2-C20 (hetero)alkenyl residue, a branched, unbranched or cyclic C2-C20 (hetero)alkynyl residue, a C1-C20 (hetero)cycloalkyl residue, a C6-C20 aryl residue, and/or with a C3-C19 heteroaryl residue.

As used herein, the terms "aryl" and "heteroaryl" may be understood in the broadest sense. A heteroaryl may comprise at least one heteroatom such as, e.g. N, O, S and/or P, preferably in the cyclic structure. Preferably, an alkenyl is a branched, unbranched or cyclic C1-C20 (hetero)alkyl, optionally substituted with deuterium, halogen, a branched, unbranched or cyclic C1-C20 (hetero)alkyl residue, a branched, unbranched or cyclic C2-C20 (hetero)alkenyl residue, a branched, unbranched or cyclic C2-C20 (hetero)alkynyl residue, a C1-C20 (hetero)cycloalkyl residue, a C6-C20 aryl residue, and/or with a C3-C19 heteroaryl residue.

In some embodiments, X² is selected from the group consisting of -OH, -SH, a C1-C6 alkoxy group, a C1-C6 alkyl sulfide group, a di(C1-C6-alkyl)amino group, and a piperazino group, wherein a nitrogen atom at the 4-position is protected by a protecting group and further optionally substituted.

The type of internucleosidic linkage is not necessarily the same in all positions of an oligonucleotide. For example, the middle of the oligonucleotide strand may comprise phosphodiester bonds, and phosphorothioate bonds may be present at the extremities of the strand.

It is clear from formulae A-1 and A-2 above that each nucleoside comprises two moieties, which may be involved in internucleosidic linkages. Therefore, these hydroxyl or amine moieties are referred to as "backbone moiety/moieties" "or "backbone group/groups" throughout this text. The backbone moieties of a nucleoside can be two hydroxyl moieties or one hydroxyl moiety and one amine moiety.

The hydroxyl moieties are involved in phosphoester type linkages with a phosphor (V) moiety located between adjacent nucleoside units. The amino moieties are involved in a phosphoramidate type linkage with the phosphor (V) moiety. One of the backbone hydroxyl moieties is involved in the internucleosidic linkage to the antecedent nucleoside, the other hydroxyl or amine moiety is involved in the internucleosidic linkage to the following nucleoside. It should be noted that the expressions "antecedent" nucleoside and "following" nucleoside are used arbitrarily in the present context and do not reflect a specific directionality of the oligonucleotide strand. As used herein, the expression "hydroxyl/amine moieties" comprises hydroxyl/amine groups and moieties derived from hydroxyl/amine groups, which are typically involved in ester bonds/amide bonds linking the residue of formulae A-1 or A-2 with the nucleotide moieties.

The backbone hydroxyl and amine groups are typically located at corresponding positions of consecutive nucleoside moieties, which are arranged to form an oligonucleotide strand. Consequently, each nucleoside may comprise a first type of backbone hydroxyl moiety located at a first position of the nucleoside and a second type of backbone hydroxyl/amine moiety located at a second position. The internucleosidic linkages typically involve a first type of backbone hydroxyl moiety derived from an antecedent nucleoside and a second type of backbone hydroxyl/amine moiety derived from the following nucleoside. In RNA and DNA, for example, the backbone hydroxyl moieties are located on the 5' position and 3' positions of the ribose moieties, i.e. each nucleoside comprises a 5' backbone hydroxyl moiety and a 3' backbone hydroxyl moiety.

Each internucleosidic phosphodiester linkage involves the 5' backbone hydroxyl moiety of an antecedent nucleoside and the 3' backbone hydroxyl moiety of the following nucleoside (in DNA and RNA). As a further example, in phosphorodiamidate morpholino oligomers (PMOs), the backbone moieties are the secondary amine moiety of the methylenemorpholine ring ("3' position") and the hydroxyl moiety attached to the methylene group of the methylenemorpholine moiety ("5' position"). Each internucleosidic phosphodiester linkage involves the secondary amine moiety of the antecedent nucleoside and the hydroxyl moiety of the following nucleoside.

Obviously, the very first and the very last nucleoside unit are involved in only one internucleosidic linkage. They may therefore comprise a free backbone hydroxyl group (e.g. the so-called 5'-OH group and the 3'-OH group in case of a phosphoribose backbone as is found in DNA and RNA), or the hydroxyl moiety may be linked to another moiety, e.g. to a capping structure.

Oligonucleotides may be conjugated to additional moieties for various purposes. Conjugation may be via at least one of the terminal backbone hydroxyl moieties or via other functional groups of nucleoside moieties, which may be located at the ends or within the oligonucleotide strand. For example, the 5'-OH group of the antisense strand of siRNA may be replaced by vinyl phosphonate to inhibit cellular degradation and improve potency. A further example is conjugation to N-acetylgalactosamine (GalNAc), which is of interest for targeted oligonucleotide delivery to hepatocytes. GalNac structures, often branched to accommodate 3 or 4 GalNac moieties, may be conjugated, e.g., to the 5'-OH group of an oligonucleotide (cf., e.g., WO2016055601), to the 3'-OH group (cf., e.g., WO2009073809), or monovalent GalNac moieties may be conjugated via a linker to the 2' position of subsequent ribose moieties within the oligonucleotide strand (cf., e.g., WO2019075419).

As used herein, the expression "conjugate of a nucleoside or (oligo)nucleotide" refers to any compound comprising a nucleoside or nucleotide covalently bound to another moiety, e.g. to a moiety comprising a peptide, protein, lipid, carbohydrate, or hydrocarbon moiety.

As used herein, the term "oligonucleotide" and "polynucleotide" may be understood interchangeably. It follows from the above explanations that non-limiting examples of oligonucleotides are deoxynucleic acids (DNA), ribonucleic acids (RNA), locked nucleic acids (LNA), constrained ethyl nucleic acid analogs (cEt), bridged nucleic acids (BNA), tricycloDNA, unlocked nucleic acids (UNA), small interfering RNA (siRNA), microRNA, antisense oligonucleotides (ASO), gapmers, glycerol nucleic acids, phosphorodiamidate morpholino oligomers (PMO), phosphorothioate oligonucleotides, phosphorodithioate oligonucleotides, diastereomerically pure phosphorothioate oligonucleotides, as well as derivatives and analogs thereof.

The expression "oligonucleotide" further comprises conjugates of an oligonucleotide moiety with other moieties such as peptides, carbohydrates, and the like, unless indicated differently in the context of specific embodiments. Particular interesting examples of oligonucleotides are phosphorthioates built from nucleosides comprising a modified ribose moiety, e.g. with 2' substituents selected from -F, -OMe (-O-CH₃), or - methoxyethyloxy (-O-CH₂-CH₂-O-CH₃, aka. MOE, -O-methoxyethyl substituents), and a nucleobase capable of Watson-Crick base pairing with a natural target sequence. The skilled artisan is aware of the fact that the above moieties may have numerous stereoisomers, all of which are encompassed in the above definitions. An "oligonucleotide" as used herein, may be a population of oligonucleotide molecules having essentially the same sequence, which is a mixture of numerous discrete stereoisomers, or which is enriched in one specific stereoisomeric form, or which essentially consists of one specific stereoisomeric form.

It will be understood by a skilled person that an oligonucleotide as used herein may optionally bear any counter ions known in the art, such as anions or cations, such as e.g., chloride ions, acetate ions, carbonate ions, hydrocarbonate ions, sodium ions, potassium ions, magnesium ions, trifluoroacetic acid (TFA) ions, bromide ions, perchlorate ions, ammonium ions and/or cations or anions of residuals of protecting groups. Further, an oligonucleotide may optionally be covalently or non-covalently associated to traces of one or more scavengers, such as, e.g., triisopropylsilane (TIS), dithiothreitol (DTT), anisole, thioanisole or 1,2-ethanedithiol.

In general terms, the present invention relates to a pseudo solid phase protecting group for protecting a nucleoside, a nucleotide, an oligonucleotide, or a conjugate of a nucleoside, a nucleotide or an oligonucleotide, wherein the pseudo solid phase protecting group is represented by formula II: 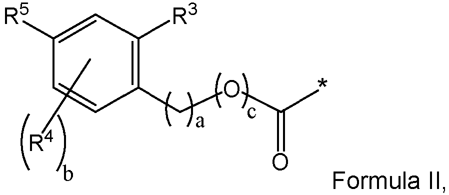 wherein:
* indicates the point of attachment to an oxygen atom of a hydroxyl moiety of the nucleoside, the nucleotide, the oligonucleotide or of the conjugate of a nucleoside, a nucleotide or an oligonucleotide to be protected;
c is 0 or 1;
a is an integer of 1 to 3;
R³ is H;
R⁵ is O-R⁶ or H, where R⁶ is a C8-C40 aliphatic hydrocarbon group;
each of R⁴ is independently O-R⁶, where R⁶ is at each occurrence independently a C8-C40 aliphatic hydrocarbon group;
b is 1 to 3;
with the proviso that if b is 1, at least one of R³ and R⁵ is not H;
and with the further proviso that the sum of all carbon atoms contained in the R³, R⁴, and
R⁵ moieties present is larger than 23 and smaller than 200.

It will be understood that, when the sum of all residues O-R⁶ of the phenyl residue of the generic formulae of the pseudo solid phase protecting group of the present invention is not larger than 4 (in other words at least one of the residues of the phenyl residue is H), the sum of all carbon atoms of the residues O-R⁶ of the phenyl residue is, throughout the present invention, preferably larger than 23 and smaller than 160.

It will be understood that, in those formulae where R³ is H, throughout the present invention, the provisos may also be defined as that if b is 1, R⁵ is not H; and that the sum of all carbon atoms contained in the R³, R⁴, and R⁵ moieties present is larger than 23 and smaller than 160.

It will be understood by those skilled in the art that any residue O-R⁶ is bonded to the core structure of formula II via the oxygen atom (i.e., -O-R⁶, wherein the hyphen indicates that the bond to the core structure of formula II is attached to the oxygen atom). The same rationale applies to other formulas comprising one or more O-R⁶ moieties. Herein, O-R⁶, -O-R⁶, OR⁶ and -OR⁶ are used interchangeably. The hyphen does not represent an additional chemical bond which is not already depicted in the respective formula, e.g. formula II.

The expression "aliphatic hydrocarbon group", as used herein, relates to a moiety, which is non-aromatic and is built from carbon atoms and hydrogen atoms, unless explicitly specified otherwise. This is to say that, unless indicated differently in the context of specific embodiments, an "aliphatic hydrocarbon group" does not comprise any heteroatoms (i.e. atoms of chemical elements other than carbon and hydrogen). If an aliphatic hydrocarbon group is said to "comprise one or more heteroatoms", this means that the one or more heteroatoms may be located in any position of the hydrocarbon group. Preferably, the heteroatoms are selected from O, S, N, Si, or halogen. For example, the hydrocarbon group may comprise -O-, -S-, -COO-, -OCONH-,-CONH- , -Cl, -Br, -I, or -NH₂- moieties. This means that the hydrocarbon group may in such cases take the form -alkyl-O-alkyl.

In some embodiments, R⁵ is H. In some embodiment, R³ is H, and R⁵ is O-R⁶. In some embodiment, R³ is H, and R⁵ is H. Further, b is an integer of 1 to 2 in some embodiments. In some embodiments, a is an integer of 1 to 3 (i.e., a is 1, 2, or 3) and b is an integer of 1 to 2 (i.e., b is 1 or 2). In some embodiments, a is 1. In some embodiments, a is 2 or 3. In some embodiments, a is 1 and b is 1 to 3. In some embodiments, a is 1 and b is 1 or 2. As used herein, a variable "is 1 to 3", if its value is selected from the group consisting of 1, 2, or 3. In some embodiments, a is 1 to 3, b is 1 to 3, and R⁶ is a C8 to C40 alkyl or heteroalkyl group. In some embodiments, a is 1, b is 1 to 3, and R⁶ is a C8 to C40 alkyl or heteroalkyl group.

In some embodiments, if c is 0, a is an integer of 1 to 2 (i.e. a is 1 or 2).

In some preferred embodiments, R⁶ is at each occurrence independently a C8-C40 alkyl or alkenyl group, in particular a C8-C40 alkyl group. In some embodiments, R⁶ is at each occurrence independently a C12-C40 aliphatic hydrocarbon group, which preferably is a C12-C40 alkyl or alkenyl group, in particular a C12-C40 alkyl group. In some embodiments, R⁶ is at each occurrence independently a C12-C30 aliphatic hydrocarbon group, which preferably is a C12-C30 alkyl or alkenyl group, in particular a C12-C30 alkyl group. In some embodiments, R⁶ is at each occurrence independently a C15-C25 aliphatic hydrocarbon group, which preferably is a C15-C25 alkyl or alkenyl group, in particular a C15-C25 alkyl group. In some embodiments, R⁶ is at each occurrence independently a C18-C22 aliphatic hydrocarbon group, which preferably is a C18-C22 alkyl or alkenyl group, in particular a C18-C22 alkyl group. In some embodiments, R⁶ is at each occurrence independently a C18-C22 aliphatic hydrocarbon group, which preferably is a linear C18-C22 alkyl or alkenyl group, in particular a linear C18-C22 alkyl group.

In some embodiments, * indicates the point of attachment to an oxygen atom of a hydroxyl moiety of the nucleoside or the nucleotide to be protected.

In some embodiments, a is 1, b is 1 to 2, and R⁶ is a C8 to C40 alkyl or heteroalkyl group. In some embodiments, a is 1, b is 1 to 2, R⁶ is a C8 to C40 alkyl or heteroalkyl group, R³ is H, and R⁵ is H or O-R⁶. In some embodiments, a is 1, b is 1 to 2, R⁶ is a C8 to C40 alkyl group, R³ is H, and R⁵ is H or O-R⁶. In the foregoing embodiments, the alkyl or heteroalkyl group may for example be a linear, i.e. an unbranched group.

In some embodiments, the sum of all carbon atoms contained in the R³, R⁴, and R⁵ moieties present in the pseudo solid phase protecting group is larger than 23 and smaller than 200, 185, 184, 180, 160, 140, 123, 120, 100, 80, 67, 60, 55, or 40.

It is clear from the above definitions that R⁶ may occur in the compounds according to formula II several times. The different instances of R⁶ groups within the same compound (according to formula I or any other formula) are selected independently from each other, i.e. they may be the same or different at each occurrence.

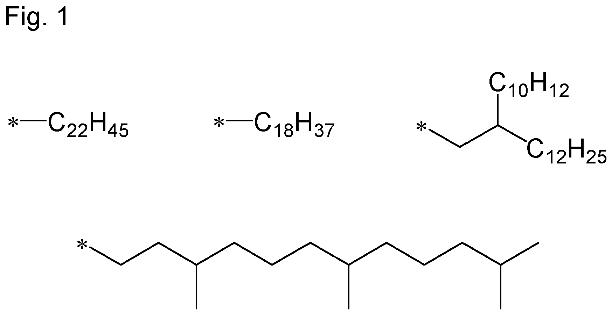

In some embodiments, R⁶ is an aliphatic hydrocarbon group having 8 to 30, 12 to 30, or 12 to 40 carbon atoms. In some embodiments, the R⁶ group has 8 to 30, 12 to 30, or 12 to 40 carbon atoms and is selected from the group consisting of an alkyl, a heteroalkyl, an alkenyl, or an heteroalkenyl. These alkyl, heteroalkyl, alkenyl, or an heteroalkenyl moieties may optionally be substituted. In some embodiments, R⁶ has one of the structures depicted in figure 1, where the asterix denotes the point of attachment to the oxygen atom. In some embodiments, R⁶ is a C8 to C40 alkyl or heteroalkyl group. In the foregoing embodiments, the alkyl or heteroalkyl group may be a linear, i.e. an unbranched group.

In some embodiments of the pseudo solid phase protecting group of the invention, c is 1. In such embodiments, the pseudo solid phase protecting group according to formula II is a pseudo solid phase protecting group according to formula II-1: 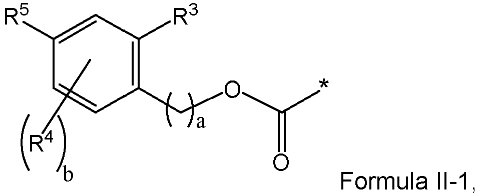 wherein for R³, R⁴, R⁵, R⁶, R⁷, a, b and * the same definitions apply as for formula II. This is to say that all embodiments and definitions set out throughout this text may also apply to pseudo solid phase protecting groups of formula II-1, with the proviso that c is always 1.

In some embodiments of the pseudo solid phase protecting group of the invention, c is 0. In such embodiments, the pseudo solid phase protecting group according to formula II is a pseudo solid phase protecting group according to formula II-2: 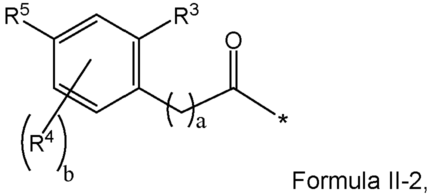 wherein for R³, R⁴, R⁵, R⁶, R⁷, a, b and * the same definitions apply as for formula II. This is to say that all embodiments and definitions set out throughout this text may also apply to pseudo solid phase protecting groups of formula II-2, with the proviso that c is always 0.

In some embodiments, the pseudo solid phase protecting group represented by formula II is a pseudo solid phase protecting group represented by formula II-a: 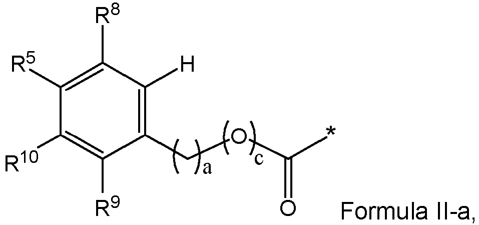 wherein:
* indicates the point of attachment to an oxygen atom of a hydroxyl moiety of the nucleoside, the nucleotide, the oligonucleotide, or of the conjugate of a nucleoside, a nucleotide or an oligonucleotide to be protected;
c is 0 or 1;
a is an integer of 1 to 3;
R⁵ is O-R⁶ or H;
each of R⁸, R⁹, and R¹⁰ is independently O-R⁶ or H;
R⁶ is at each occurrence independently a C8-C40 aliphatic hydrocarbon group;
with the proviso that the pseudo solid phase protecting group according to formula II-a comprises exactly 2 or exactly 3 O-R⁶ moieties; and
with the further proviso that the sum of all carbon atoms contained in the R⁵, R⁸, R⁹, and R¹⁰ moieties is larger than 23 and smaller than 200.

It will be understood that, when the sum of all residues O-R⁶ of the phenyl residue of the generic formulae of the pseudo solid phase protecting group of the present invention is not larger than 3 (in other words at least two of the residues of the phenyl residues are H), the sum of all carbon atoms of the residues O-R⁶ of the phenyl residue is, throughout the present invention, preferably larger than 23 and smaller than 120.

Accordingly, when the pseudo solid phase protecting group according to formula II-a comprises exactly 3 O-R⁶ moieties, the sum of all carbon atoms contained in the R⁵, R⁸, R⁹, and R¹⁰ moieties may be larger than 23 and smaller than 120.

It will be understood that, when the sum of all residues O-R⁶ of the phenyl residue of the generic formulae of the pseudo solid phase protecting group of the present invention is not larger than 2 (in other words at least three of the residues of the phenyl residues are H), the sum of all carbon atoms of the residues O-R⁶ of the phenyl residue is, throughout the present invention, preferably larger than 23 and smaller than 80.

Accordingly, when the pseudo solid phase protecting group according to formula II-a comprises exactly 2 O-R⁶ moieties, the sum of all carbon atoms contained in the R⁵, R⁸, R⁹, and R¹⁰ moieties may be larger than 23 and smaller than 80.

In some embodiments of the pseudo solid phase protecting group represented by formula II-a, R⁶ is at each occurrence independently a C8-C40 alkyl or alkenyl group, in particular a C8-C40 alkyl group. In some embodiments of the pseudo solid phase protecting group represented by formula II-a, R⁶ is at each occurrence independently a C12-C40 aliphatic hydrocarbon group, which preferably is a C12-C40 alkyl or alkenyl group, in particular a C12-C40 alkyl group. In some embodiments of the pseudo solid phase protecting group represented by formula II-a, R⁶ is at each occurrence independently a C12-C30 aliphatic hydrocarbon group, which preferably is a C12-C30 alkyl or alkenyl group, in particular a C12-C30 alkyl group. In some embodiments of the pseudo solid phase protecting group represented by formula II-a, R⁶ is at each occurrence independently a C15-C25 aliphatic hydrocarbon group, which preferably is a C15-C25 alkyl or alkenyl group, in particular a C15-C25 alkyl group. In some embodiments of the pseudo solid phase protecting group represented by formula II-a, R⁶ is at each occurrence independently a C18-C22 aliphatic hydrocarbon group, which preferably is a C18-C22 alkyl or alkenyl group, in particular a C18-C22 alkyl group. In some embodiments of the pseudo solid phase protecting group represented by formula II-a, R⁶ is at each occurrence independently a C18-C22 aliphatic hydrocarbon group, which preferably is a linear C18-C22 alkyl or alkenyl group, in particular a linear C18-C22 alkyl group.

In some embodiments of the pseudo solid phase protecting group represented by formula II-a, a is 1. In some embodiments of the pseudo solid phase protecting group represented by formula II-a, a is 2. In some embodiments of the pseudo solid phase protecting group represented by formula II-a, a is 3. In some embodiments of the pseudo solid phase protecting group represented by the formula II-a, a is 2 or 3.

In some embodiments of the pseudo solid phase protecting group represented by formula II-a, if a is 1 and R⁵ is O-R⁶, R⁹ is H.

In some embodiments of the pseudo solid phase protecting group represented by formula II-a:
- R⁵ is O-R⁶, R⁸ and R¹⁰ are both O-R⁶, and R⁹ is H;
- R⁵ is O-R⁶, R⁸ and R¹⁰ are both H, and R⁹ is O-R⁶; or
- R⁵ is H, R⁸ and R¹⁰ are both O-R⁶, and R⁹ is H.

In some embodiments of the pseudo solid phase protecting group represented by formula II-a:
- R⁵ is O-R⁶, R⁸ and R¹⁰ are both O-R⁶, and R⁹ is H, wherein a is an integer of 1 to 3;
- R⁵ is O-R⁶, R⁸ and R¹⁰ are both H, and R⁹ is O-R⁶, wherein a is an integer of 2 to 3; or
- R⁵ is H, R⁸ and R¹⁰ are both O-R⁶, and R⁹ is H, wherein a is an integer of 1 to 3.

In some embodiments of the pseudo solid phase protecting group represented by formula II-a:
- R⁵ is O-R⁶, R⁸ and R¹⁰ are both O-R⁶, and R⁹ is H, wherein a is an integer of 1 to 3 and R⁶ is at each occurrence independently a C8-C40 aliphatic hydrocarbon group;
- R⁵ is O-R⁶, R⁸ and R¹⁰ are both H, and R⁹ is O-R⁶, wherein a is an integer of 2 to 3 and R⁶ is at each occurrence independently a C12-C40 aliphatic hydrocarbon group; or
- R⁵ is H, R⁸ and R¹⁰ are both O-R⁶ and R⁹ is H, wherein a is an integer of 1 to 3 and R⁶ is at each occurrence independently a C12-C40 aliphatic hydrocarbon group.

In some embodiments of the pseudo solid phase protecting group represented by formula II-a:
- R⁵ is O-R⁶, R⁸ and R¹⁰ are both O-R⁶, and R⁹ is H, wherein a is an integer of 1 to 3, R⁶ is at each occurrence independently a C8-C40 aliphatic hydrocarbon group, and c is 1;
- R⁵ is O-R⁶, R⁸ and R¹⁰ are both H, and R⁹ is O-R⁶, wherein a is an integer of 2 to 3, R⁶ is at each occurrence independently a C12-C40 aliphatic hydrocarbon group, and c is 0 or 1; or
- R⁵ is H, R⁸ and R¹⁰ are both O-R⁶ and R⁹ is H, wherein a is an integer of 1 to 3, R⁶ is at each occurrence independently a C12-C40 aliphatic hydrocarbon group, and c is 0 or 1.

In some embodiments of the pseudo solid phase protecting group represented by formula II-a, if c is 0, a is an integer of 1 to 2 (i.e., a is 1 or 2).

In some embodiments of the pseudo solid phase protecting group represented by the formula II-a, c is 1. In such embodiments, the pseudo solid phase protecting group according to formula II-a is a pseudo solid phase protecting group according to formula II-a-1: 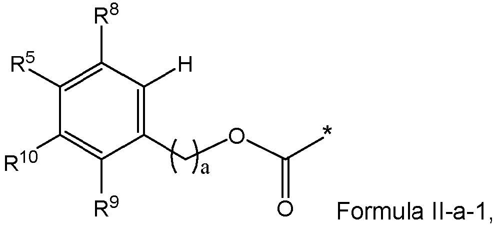 wherein for R⁵, R⁶, R⁸, R⁹, R¹⁰, a, and * the same definitions apply as for formula II-a. This is to say that any embodiments and definitions relating to the pseudo solid phase protecting group of formula II-a may also apply to the pseudo solid phase protecting group of formula II-a-1, with the proviso that c is always 1.

In some embodiments of the pseudo solid phase protecting group represented by the formula II-a, c is 0. In such embodiments, the pseudo solid phase protecting group according to formula II-a is a pseudo solid phase protecting group according to formula II-a-2: 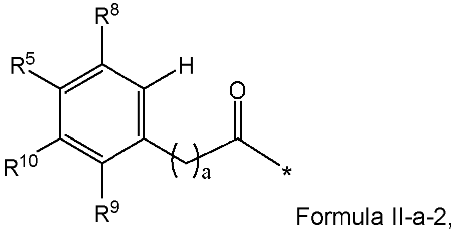 wherein for R⁵, R⁶, R⁸, R⁹, R¹⁰, a, and * the same definitions apply as for formula II-a. This is to say that any embodiments and definitions relating to the pseudo solid phase protecting group of formula II-a may also apply to the pseudo solid phase protecting group of formula II-a-2, with the proviso that c is always 0.

Further preferred embodiments of the pseudo solid phase protecting group according to formula II and formula II-a are shown in formulae III to VII below:

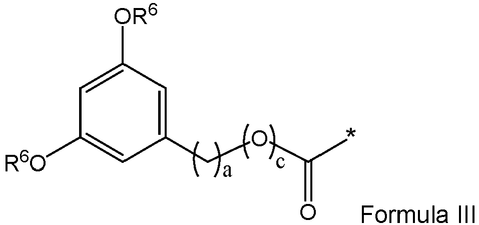

In formula III, * indicates the point of attachment to the oxygen atom of a hydroxyl moiety to be protected; c is 0 or 1; a is an integer of 1 to 3; and the above definitions of R⁶ apply. In some embodiments, each of R⁶ is independently a C12-C40 aliphatic hydrocarbon group, which optionally comprises one or more heteroatoms. In some embodiments, each of R⁶ is independently an aliphatic hydrocarbon group or a heteroalkyl group having 12 to 40 carbon atoms; preferably 12 to 30 carbon atoms. In some embodiments, each of R⁶ is independently an aliphatic hydrocarbon group having 12 to 30 carbon atoms. In some embodiments, R⁶ is chosen from the structures depicted in Fig 1. In some embodiments, each of R⁶ is independently a C12 to C40 alkyl group or a C12 to C40 heteroalkyl group. In some embodiments, each of R⁶ is independently a C12 to C40 alkyl group. In some embodiments, each of R⁶ is independently a linear C12 to C40 alkyl group.

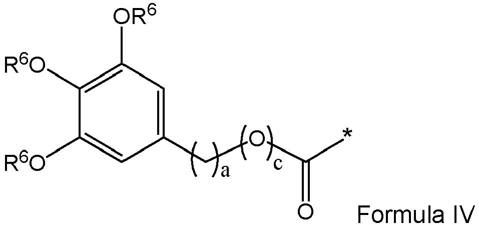

In formula IV, * indicates the point of attachment to the oxygen atom of a hydroxyl moiety to be protected; c is 0 or 1; a is an integer of 1 to 3; and the above definitions of R⁶ apply. In some embodiments, each of R⁶ is independently a C8-C40 aliphatic hydrocarbon group, which optionally comprises one or more heteroatoms. In some embodiments, each of R⁶ is independently an aliphatic hydrocarbon group or a heteroalkyl group having 8 to 40 carbon atoms; preferably 8 to 30 carbon atoms. In some embodiments, each of R⁶ is independently an aliphatic hydrocarbon group having 8 to 30 carbon atoms. In some embodiments, R⁶ is chosen from the structures depicted in Fig 1. In some embodiments, each of R⁶ is independently a C8 to C40 alkyl group or a C8 to C40 heteroalkyl group. In some embodiments, each of R⁶ is independently a C8 to C40 alkyl group. In some embodiments, each of R⁶ is independently a linear C8 to C40 alkyl group.

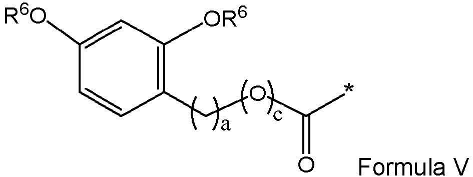

In formula V, * indicates the point of attachment to the oxygen atom of a hydroxyl moiety to be protected; c is 0 or 1; a is an integer of 1 to 3, preferably 2 to 3; and the above definitions of R⁶ apply. In some preferred embodiments, a is an integer of 2 to 3. In some embodiments each of R⁶ is independently a C12-C40 aliphatic hydrocarbon group, which optionally comprises one or more heteroatoms. In some embodiments, each of R⁶ is independently an aliphatic hydrocarbon group or a heteroalkyl group having 12 to 40 carbon atoms; preferably 12 to 30 carbon atoms. In some embodiments, each of R⁶ is independently an aliphatic hydrocarbon group having 12 to 30 carbon atoms. In some embodiments, R⁶ is chosen from the structures depicted in Fig 1. In some embodiments, each of R⁶ is independently a C12 to C40 alkyl group or a C12 to C40 heteroalkyl group. In some embodiments, each of R⁶ is independently a C12 to C40 alkyl group. In some embodiments, each of R⁶ is independently a linear C12 to C40 alkyl group.

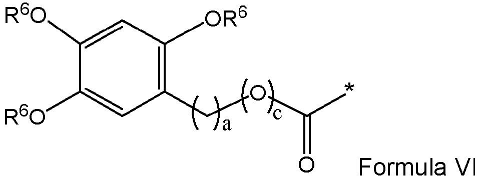

In formula VI, * indicates the point of attachment to the oxygen atom of a hydroxyl moiety to be protected; c is 0 or 1; a is an integer of 1 to 3, preferably of 2 to 3; and the above definitions of R⁶ apply. In some embodiments, each of R⁶ is independently a C8-C40 aliphatic hydrocarbon group, which optionally comprises one or more heteroatoms. In some embodiments, each of R⁶ is independently an aliphatic hydrocarbon group or a heteroalkyl group having 8 to 40 carbon atoms; preferably 8 to 30 carbon atoms. In some embodiments, each of R⁶ is independently an aliphatic hydrocarbon group having 8 to 30 carbon atoms. In some embodiments, R⁶ is chosen from the structures depicted in Fig 1. In some embodiments, each of R⁶ is independently a C8 to C40 alkyl group or a C8 to C40 heteroalkyl group. In some embodiments, each of R⁶ is independently a C8 to C40 alkyl group. In some embodiments, each of R⁶ is independently a linear C8 to C40 alkyl group.

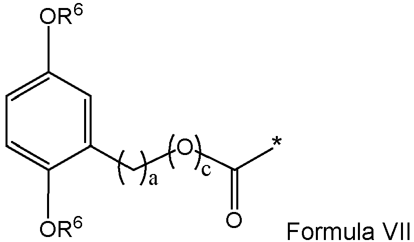

In formula VII, * indicates the point of attachment to the oxygen atom of a hydroxyl moiety to be protected; c is is 0 or 1; a is an integer of 1 to 3; and the above definitions of R⁶ apply. In some embodiments each of R⁶ is independently a C12-C40 aliphatic hydrocarbon group, which optionally comprises one or more heteroatoms. In some embodiments, each of R⁶ is independently an aliphatic hydrocarbon group or a heteroalkyl group having 12 to 40 carbon atoms; preferably 12 to 30 carbon atoms. In some embodiments, each of R⁶ is independently an aliphatic hydrocarbon group having 12 to 30 carbon atoms. In some embodiments, R⁶ is chosen from the structures depicted in Fig 1. In some embodiments, each of R⁶ is independently a C12 to C40 alkyl group or a C12 to C40 heteroalkyl group. In some embodiments, each of R⁶ is independently a C12 to C40 alkyl group. In some embodiments, each of R⁶ is independently a linear C12 to C40 alkyl group.

For the avoidance of doubt, as used herein, the expression "an aliphatic hydrocarbon group or a heteroalkyl group having 12 to 40 carbon atoms" as used herein refers to a C12 to C40 aliphatic hydrocarbon group or to a C12 to C40 heteroalkyl group. In the foregoing embodiments, the alkyl or heteroalkyl group may be a linear, i.e. an unbranched group.

In some embodiments, the pseudo solid phase protecting group represented by any one of formulae II and II-a is a pseudo solid phase protecting group represented by any one of formulae III to VII: 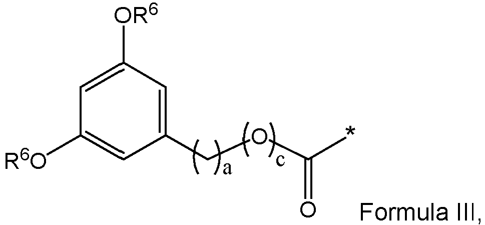 wherein in formula III, * indicates the point of attachment to the oxygen atom of a hydroxyl moiety to be protected; c 0 or 1; a is an integer of 1 to 3; and each of R⁶ is independently a C12-C40 aliphatic hydrocarbon group; 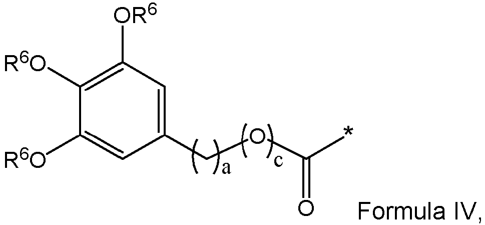 wherein in formula IV, * indicates the point of attachment to the oxygen atom of a hydroxyl moiety to be protected; c is 0 or 1; a is an integer of 1 to 3; and each of R⁶ is independently a C8-C40 aliphatic hydrocarbon group; 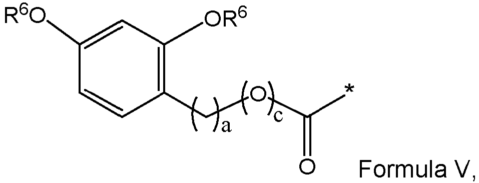 wherein in formula V, * indicates the point of attachment to the oxygen atom of a hydroxyl moiety to be protected; c is 0 or 1; a is an integer of 2 to 3; and each of R⁶ is independently a C12-C40 aliphatic hydrocarbon group; 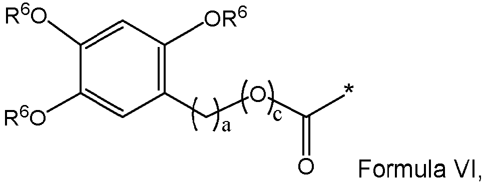 wherein in formula VI, * indicates the point of attachment to the oxygen atom of a hydroxyl moiety to be protected; c is 0 or 1; a is an integer of 2 to 3; and each of R⁶ is independently a C8-C40 aliphatic hydrocarbon group; and 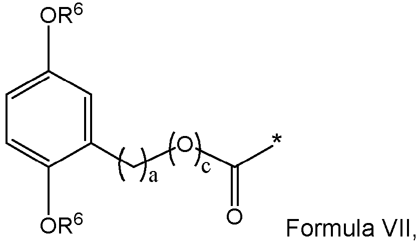 wherein in formula VII, * indicates the point of attachment to the oxygen atom of a hydroxyl moiety to be protected; c is 0 or 1; a is an integer of 1 to 3; and each of R⁶ is independently a C12-C40 aliphatic hydrocarbon group.

In some embodiments, the pseudo solid phase protecting group represented by any one of formulae II and II-a is a pseudo solid phase protecting group represented by any one of the aforementioned formulae III to V:
wherein in formula III, * indicates the point of attachment to the oxygen atom of a hydroxyl moiety to be protected; c is 0 or 1; a is an integer of 1 to 3; and each of R⁶ is independently a C12-C40 aliphatic hydrocarbon group, preferably a C12-C40 alkyl group, in particular a linear C12-C40 alkyl group;
wherein in formula IV, * indicates the point of attachment to the oxygen atom of a hydroxyl moiety to be protected; c is 0 or 1; a is an integer of 1 to 3; and each of R⁶ is independently a C8-C40 aliphatic hydrocarbon group, preferably a C8-C40 alkyl group, in particular a linear C8-C40 alkyl group; and
wherein in formula V, * indicates the point of attachment to the oxygen atom of a hydroxyl moiety to be protected; c is 0 or 1; a is an integer of 2 to 3; and each of R⁶ is independently a C12-C40 aliphatic hydrocarbon group, preferably a C12-C40 alkyl group, in particular a linear C12-C40 alkyl group.

In some embodiments of the pseudo solid phase protecting group represented by any one of formulae III to VII, if c is 0, a is an integer of 1 to 2 (i.e., a is 1 or 2). In some embodiments, in formula IV, c is always 1.

In some embodiments, the pseudo solid phase protecting group of the invention is a pseudo solid phase protecting group for protecting a nucleoside, a nucleotide, or an oligonucleotide. In such embodiments, * in any one of formulae II, 11-1, II-2, II-a, II-a-1, II-a-2, and III to VII indicates the point of attachment to an oxygen atom of a hydroxyl moiety of the nucleoside, the nucleotide, or the oligonucleotide to be protected. In some embodiments, the pseudo solid phase protecting group of the invention is a pseudo solid phase protecting group for protecting a nucleoside, a nucleotide, or an oligonucleotide, wherein the term "oligonucleotide" does not embrace conjugates of an oligonucleotide. In some embodiments, the pseudo solid phase protecting group of the invention is a pseudo solid phase protecting group for protecting a nucleoside. In such embodiments, * in in any one of formulae II, 11-1, II-2, II-a, II-a-1, II-a-2, and III to VII indicates the point of attachment to an oxygen atom of a hydroxyl moiety of the nucleoside to be protected.

The present invention further relates to a compound according to formula I: 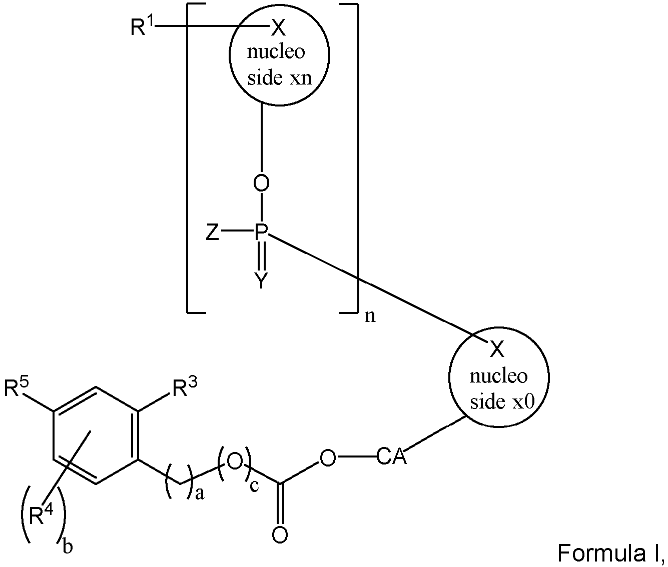 wherein:
R¹ is a protecting group;
X is independently at each position O or N;
n is an integer equal to or larger than 0;
Z is independently at each position H or an electron withdrawing group;
Y is independently for each repetitive unit n missing or O or S;
each of the nucleosides x0 to xn may be the same or different;
CA is a capping moiety or a single bond;
c is 0 or 1;
a is an integer of 1 to 3;
R³ is H;
R⁵ is O-R⁶ or H where R⁶ is a C8-C40 aliphatic hydrocarbon group;
each of R⁴ is independently O-R⁶, where R⁶ is at each occurrence independently a C8-C40 aliphatic hydrocarbon group;
b is 1 to 3;
with the proviso that if b is 1, at least one of R³ and R⁵ is not H;
and with the further proviso that the sum of all carbon atoms contained in the R³, R⁴, and
R⁵ moieties present is larger than 23 and smaller than 200.

The specifications and explanations in respect of the integer a, as well as the residues R³, R⁵, R⁶, R⁴, R⁶, R⁷, and the integer b, which have been laid out with respect to the pseudo solid phase protecting group of the invention, in particular with regard to formula II, are likewise applicable to the compound according to formula I. For example, at least one of R³ and R⁵ may be H, a may be an integer from 1 to 3, b may be an integer from 1 to 2, and R⁶ may be a C1-C30 hydrocarbon group, which is optionally substituted with one or more moieties selected from the group consisting of a C1-C30 heteroalkyl group, a C1-C30 alkyl group, a C1-C30 heteroalkenyl group, and a C1-C30 alkenyl group. In some embodiments of the compound according to formula I, R³ is H. In some embodiments of the compound according to formula I, a is an integer of 1 to 3 and b is an integer of 1 to 2. In some embodiments of the compound according to formula I, if c is 0, a is an integer of 1 to 2 (i.e. a is 1 or 2).

In some embodiments of the compound according to formula I, CA is a single bond. In this case, the oxygen atom depicted next to CA in formula I is bonded via a single bond (the single bond being CA) directly to the nucleoside x0.

The electron withdrawing group Z attached to the phosphorous atom may be any group decreasing the electron density at the P atom. Z may, e.g., be selected from the group consisting of -OH, -OR, -NHR, -NR₂, an C3-C20-(hetero)aromatic moiety, -F, -Cl, -SH, -SeH, -BH₃⁻, -SR, and -SeR, where R is independently a C1-C10 alkyl or a C1-C10 heteroalkyl group. In particular, R may be a C1-C10 alkyl substituted with an electron withdrawing group, e.g. a beta-cyanoethyl group (i.e. a 2-cyanoethyl group). In some embodiments of the compound according to formula I, the electron withdrawing group Z is at each occurrence -OR with R being a 2-cyanoethyl group. Herein, unless indicated differently, a "2-cyanoethyl" protecting group for a hydroxyl moiety is bonded to the oxygen atom to be protected via its 1-position, i.e. as 2-cyanoeth-1-yl group.

In some embodiments of the compound according to formula I, Y is independently for each repetitive unit n O or S. In some embodiments of the compound according to formula I, if Z is H, Y is O (oxygen). In some embodiments of the compound according to formula I, Y is O or S and Z is at each occurrence -OR with R being a 2-cyanoethyl group. In some embodiments of the compound according to formula I, Y is O or S, Z is at each occurrence -OR with R being a 2-cyanoethyl group, and CA is a single bond. As laid out above, the hyphen in -OR is merely used to indicate that the group OR bonds to the core structure of the respective formula, e.g. formula I, via the oxygen atom from which the hyphen originates. The hyphen does not represent an additional chemical bond which is not already depicted in the respective formula, e.g. formula I.

R¹ is a hydroxyl or amine protecting group. R¹ may be a temporary protecting group, which is used during iterative oligomer synthesis to avoid unintended oligomerisation or multiple insertions of a building block. Preferably, R¹ is a triarylmethyl type protecting group, i.e. a protecting group comprising an optionally substituted triarylmethyl residue. The skilled person is well aware of the fact that the properties of such protecting groups may differ depending on their substitution pattern.

Substituents, which stabilize the carbocation leaving group, may facilitate cleavage of the protecting group. For example, the triaryl methyl protecting group may comprise at least one substituent selected from the group consisting of methoxy groups and halogen atoms. Non limiting examples of R¹ include the trityl group (i.e. the triphenylmethyl group), the 4-methyltrityl group, the 4,4'-dimethyltrityl group, the 4,4',4"-trimethyltrityl group, the (p-methoxyphenyl)diphenylmethyl (MMT) group, the di(p-methoxyphenyl)phenylmethyl (DMT) group, the tri(p-methoxyphenyl)methyl ether (TMT) group, the 4,4'-dimethoxy-3"-[N-(imidazolylmethyl)]trityl (IDT) group, the 4,4'-dimethoxy-3"-[N-(imidazolylethyl)carbamoyl]trityl ether (IET) group, the bis(4-methoxyphenyl)-1'-pyrenylmethyl (Bmpm) group, and the 4-(17-tetrabenzo[a,c,g,i]fluorenylmethyl)-4',4"-dimethoxytrityl (Tbf-DMT) group. The DMT group is particularly preferred. The protecting group comprising an optionally substituted triarylmethyl residue may be the same or different for different building blocks used in a method according to the present inventions.

In some embodiments of the compound according to formula I, R¹ is a triarylmethyl type protecting group as defined herein. In some embodiments of the compound according to formula I, R¹ is a triarylmethyl type protecting selected from the group consisting of a trityl group, a MMT group, and a DMT group. In some preferred embodiments of the compound according to formula I, R¹ is a DMT group. In some embodiments of the compound according to formula I, R¹ is a trityl group.

In some embodiments of the compound according to formula I, n is an integer in the range of 0-200, 0-150, 0-120, 0-100, 0-90, 0-80, 0-70, 0-60, 0-50, 0-40, 0-30, 0-20, or 0-10. In some embodiments of the compound according to formula I, the integer n is 0 or 1. In some embodiments of the compound according to formula I, the integer n is 0. It will be understood by a person skilled in the art that, if the integer n is 0, the protecting group R¹ will still be present in the compound of formula I, and will be bonded to the nucleoside x0.

In formula I, each nucleoside is symbolized by a circle, which is connected via a single bond to the oxygen atom of the first backbone hydroxyl moiety, and which comprises a second backbone hydroxyl or amine moiety. X denotes the heteroatom of said second backbone hydroxyl or amine moiety, which is bonded to the phosphor atom of the following internucleosidic linkage group, e.g. phosphate like group. X may be integrated into the nucleoside molecule in any conceivable way. For example, X may be at the 5' position of a ribose moiety (i.e. X is an oxygen atom connected via a single bond to the reminder of the nucleoside moiety), or may be part of a secondary amine group, which forms part of a morpholine ring (i.e. X is a nitrogen atom connected via two single bonds to the reminder of the nucleoside moiety). X may be chosen independently for each repetitive unit n (i.e. for each nucleoside x0 and xn). In some embodiments, X is the same in all positions of the compound according to formula I (i.e. for each nucleoside x0 and xn).

In some embodiments of the compound according to formula I, c is 1. In some embodiments of the compound according to formula I, c is 0.

In some embodiments, the compound of formula I is a compound of formula I-b: 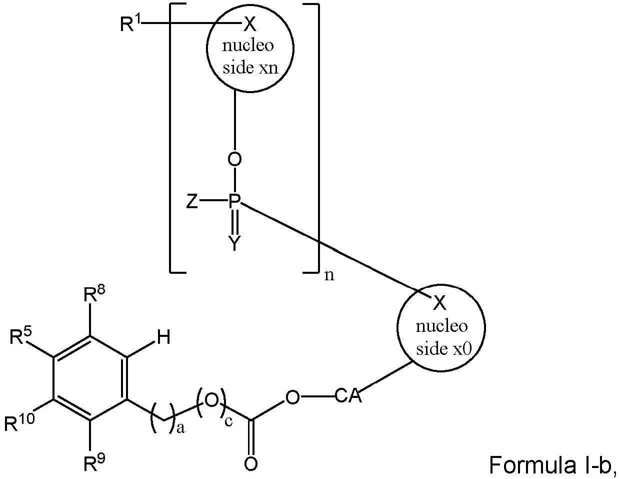 wherein:
R¹ is a protecting group;
X is independently at each position O or N;
n is an integer equal to or larger than 0;
Z is independently at each position H or an electron withdrawing group;
Y is independently for each repetitive unit n missing or O or S;
each of the nucleosides x0 to xn may be the same or different;
CA is a capping moiety or a single bond;
c is 0 or 1;
a is an integer of 1 to 3;
R⁵ is O-R⁶ or H;
each of R⁸, R⁹, and R¹⁰ is independently O-R⁶ or H;
R⁶ is at each occurrence independently a C8-C40 aliphatic hydrocarbon group;
with the proviso that the compound according to formula I-b comprises exactly 2 or exactly 3 O-R⁶ moieties; and
with the proviso that the sum of all carbon atoms contained in the R⁵, R⁸, R⁹, and R¹⁰ moieties is larger than 23 and smaller than 200.

As the compound of formula I-b is a compound of formula I, any embodiments and definitions relating to the compound of formula I may also apply to the compound of formula I-b. Thus, the specifications, explanations, and embodiments in respect of the integer n, X, Y, Z, CA, R¹, x0, and xn, R⁵, R⁶, R⁸, R⁹, and R¹⁰ which have been laid out with respect to the compound of formula I, are likewise applicable to the compound of formula I-b.

In some preferred embodiments of the compound according to formula I-b, none of x0, xn, R¹, CA, and Z comprises an O-R⁶ moiety.

In some preferred embodiments of the compound of formula I-b, R⁶ is at each occurrence independently a C8-C40 alkyl or alkenyl group, in particular a C8-C40 alkyl group. In some embodiments of the compound of formula I-b, R⁶ is at each occurrence independently a C12-C40 aliphatic hydrocarbon group, which preferably is a C12-C40 alkyl or alkenyl group, in particular a C12-C40 alkyl group. In some embodiments of the compound of formula I-b, R⁶ is at each occurrence independently a C12-C30 aliphatic hydrocarbon group, which preferably is a C12-C30 alkyl or alkenyl group, in particular a C12-C30 alkyl group. In some embodiments of the compound of formula I-b, R⁶ is at each occurrence independently a C15-C25 aliphatic hydrocarbon group, which preferably is a C15-C25 alkyl or alkenyl group, in particular a C15-C25 alkyl group. In some embodiments of the compound of formula I-b, R⁶ is at each occurrence independently a C18-C22 aliphatic hydrocarbon group, which preferably is a C18-C22 alkyl or alkenyl group, in particular a C18-C22 alkyl group. In some embodiments of the compound of formula I-b, R⁶ is at each occurrence independently a C18-C22 aliphatic hydrocarbon group, which preferably is a linear C18-C22 alkyl or alkenyl group, in particular a linear C18-C22 alkyl group.

In some embodiments of the compound of formula I-b, a is 1. In some embodiments of the compound of formula I-b, a is 2. In some embodiments of the compound of formula I-b, a is 3. In some embodiments of the compound of formula I-b, a is 2 or 3. In some embodiments, of the compound of formula I-b, if a is 1 and R⁵ is O-R⁶, R⁹ is H. In some embodiments of the compound of formula I-b, if c is 0, a is an integer of 1 to 2 (i.e. a is 1 or 2).

In some embodiments of the compound of formula I-b:
- R⁵ is O-R⁶, R⁸ and R¹⁰ are both O-R⁶, and R⁹ is H;
- R⁵ is O-R⁶, R⁸ and R¹⁰ are both H, and R⁹ is O-R⁶; or
- R⁵ is H, R⁸ and R¹⁰ are both O-R⁶, and R⁹ is H.

In some embodiments of the compound of formula I-b:
- R⁵ is O-R⁶, R⁸ and R¹⁰ are both O-R⁶, and R⁹ is H, wherein a is an integer of 1 to 3;
- R⁵ is O-R⁶, R⁸ and R¹⁰ are both H, and R⁹ is O-R⁶, wherein a is an integer of 2 to 3; or
- R⁵ is H, R⁸ and R¹⁰ are both O-R⁶ and R⁹ is H, wherein a is an integer of 1 to 3.

In some embodiments of the compound of formula I-b:
- R⁵ is O-R⁶, R⁸ and R¹⁰ are both O-R⁶, and R⁹ is H, wherein a is an integer of 1 to 3 and R⁶ is at each occurrence independently a C8-C40 aliphatic hydrocarbon group;
- R⁵ is O-R⁶, R⁸ and R¹⁰ are both H, and R⁹ is O-R⁶, wherein a is an integer of 2 to 3 and R⁶ is at each occurrence independently a C12-C40 aliphatic hydrocarbon group; or
- R⁵ is H, R⁸ and R¹⁰ are both O-R⁶ and R⁹ is H, wherein a is an integer of 1 to 3 and R⁶ is at each occurrence independently a C12-C40 aliphatic hydrocarbon group.

In some embodiments of the compound of formula I-b, c is 1. In some embodiments of the compound of formula I-b, c is 0.

In some embodiments of the compound of formula I-b, Z is independently at each position -OR with R being a 2-cyanoethyl group, Y is independently for each repetitive unit n O or S, CA is a single bond, wherein:
- R⁵ is O-R⁶, R⁸ and R¹⁰ are both O-R⁶, and R⁹ is H, wherein a is an integer of 1 to 3 and R⁶ is at each occurrence independently a C8-C40 aliphatic hydrocarbon group;
- R⁵ is O-R⁶, R⁸ and R¹⁰ are both H, and R⁹ is O-R⁶, wherein a is an integer of 2 to 3 and R⁶ is at each occurrence independently a C12-C40 aliphatic hydrocarbon group; or
- R⁵ is H, R⁸ and R¹⁰ are both O-R⁶ and R⁹ is H, wherein a is an integer of 1 to 3 and R⁶ is at each occurrence independently a C12-C40 aliphatic hydrocarbon group.

In some embodiments of the compound of formula I-b, Z is independently at each position -OR with R being a 2-cyanoethyl group, Y is independently for each repetitive unit n O or S, CA is a single bond, wherein:
- R⁵ is O-R⁶, R⁸ and R¹⁰ are both O-R⁶, and R⁹ is H, wherein a is an integer of 1 to 3, R⁶ is at each occurrence independently a C8-C40 aliphatic hydrocarbon group, and c is 1;
- R⁵ is O-R⁶, R⁸ and R¹⁰ are both H, and R⁹ is O-R⁶, wherein a is an integer of 2 to 3, R⁶ is at each occurrence independently a C12-C40 aliphatic hydrocarbon group, and c is 1 or 0; or
- R⁵ is H, R⁸ and R¹⁰ are both O-R⁶ and R⁹ is H, wherein a is an integer of 1 to 3, R⁶ is at each occurrence independently a C12-C40 aliphatic hydrocarbon group, and c is 1 or 0.

In particularly preferred embodiments of the compound of the invention, X is O. In these embodiments, the compound according to formula I may be alternatively reflected by formula I-a, wherein the definitions of a, b, c, R¹, n, Z, Y, CA, R³, R⁴, R⁵, R⁶, and R⁷ are as set out with respect to formula I:

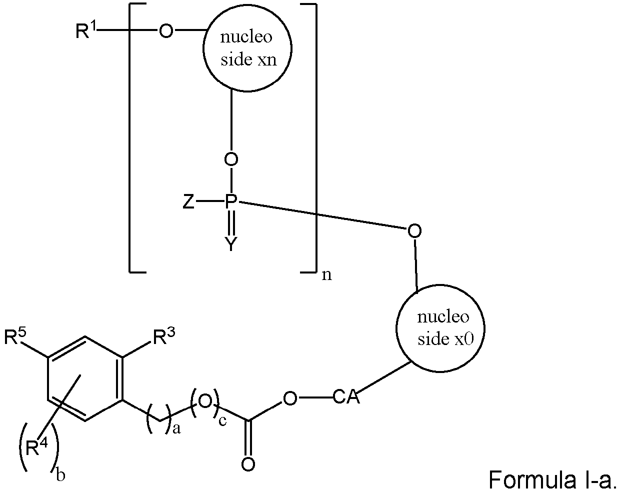

Analogous formulae may be drawn for formulae VIII to XII (vide infra), wherein X is O, wherein the definitions of a, c, R¹, n, Z, Y, CA, and R⁶ are as set out with respect to the respective formula.

Along the same lines, if X is O, the compound according to formula I-b may be alternatively reflected by formula I-c, wherein the definitions of a, c, R¹, n, Z, Y, CA, R⁵, R⁶, R⁸, R⁹, and R¹⁰ are as set out with respect to formula I-b:

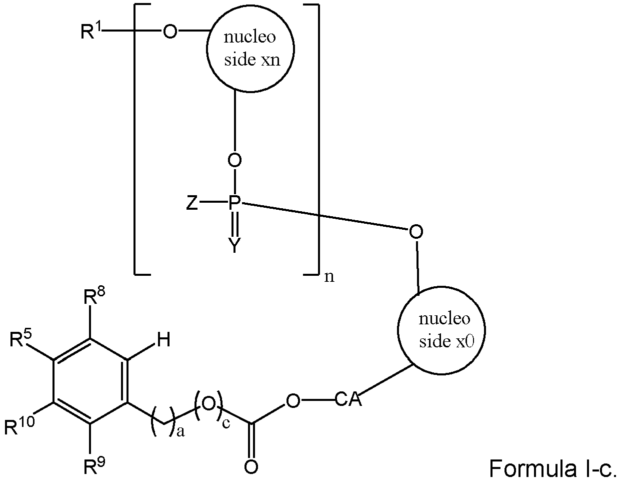

In some embodiments of the compound according to formula I-a, c is 1. In some embodiments of the compound according to formula I-a, c is 0. In some embodiments of the compound according to formula I-c, c is 1. In some embodiments of the compound according to formula I-c, c is 0. In some embodiments of the compound according to formula I-a, if c is 0, a is an integer of 1 to 2 (i.e. a is 1 or 2). In some embodiments of the compound according to formula I-c, if c is 0, a is an integer of 1 to 2 (i.e. a is 1 or 2). Exemplary structures according to formula I, in particular according to any one of formulae I-a, I-b, I-c, and VIII to XII (vide infra) comprise, e.g., the following moieties when bound via a hydroxyl moiety to a pseudo solid phase protecting group according to formula II, in particular according to any one of formulae II-1, II-2, II-a, II-a-1, II-a-2, and III to VII: DNA oligonucleotides, RNA oligonucleotides, LNA oligonucleotides, UNA oligonucleotides, phosphorthioate oligonucleotides, nucleosides connected by phosphite triester moieties, and phosphorodiamidate morpholino oligomers (PMOs), and any conjugates of the foregoing. The following structures illustrate non-limiting examples of such compounds, which may be attached via the respective point of attachment (*) to a pseudo solid phase protecting group according to any one of formulae II, 11-1, 11-2, II-a, II-a-1, II-a-2, and III to VII, so as to obtain a compound according to any one of formulae I, I-a, I-b, I-c, and VIII to XII (vide infra).

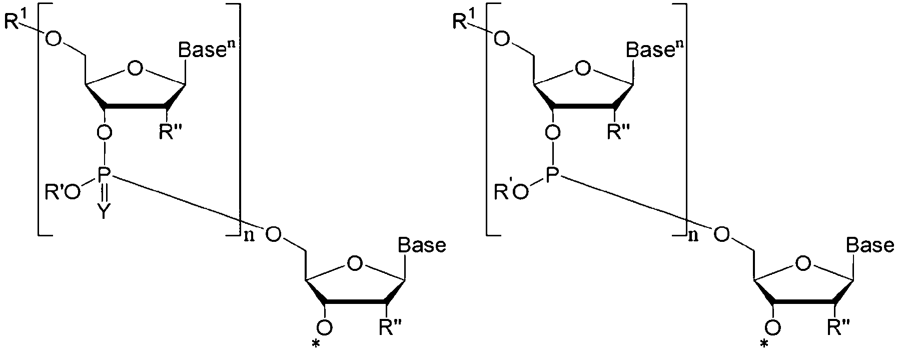

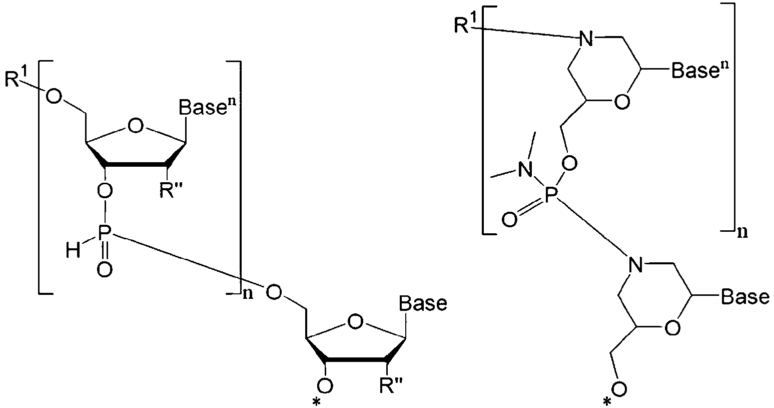

Alternatively, to obtain a compound according to any one of formulae I, I-a, I-b, I-c, and VIII to XII (vide infra), the above compounds may be attached via the point of attachment (*) to a non-nucleosidic moiety, which in turn comprises a hydroxyl moiety protected by a pseudo solid phase protecting group according to any one of formulae II, II-1, II-2, II-a, II-a-1, II-a-2, and III to VII. Said non-nucleosidic moiety may be a capping moiety CA. Depending on the identity of Y, the phosphorous atoms in any one of formulae I, I-a, I-b, I-c, and VIII to XII (vide infra) may have the oxidation number III (where Y is H) or V (where Y is an electron withdrawing group).

As used herein, the expression "capping moiety" refers to any moiety, which is conjugated to a terminal nucleoside of the oligonucleotide strand. The capping moiety is comprised in the final oligonucleotide product obtained after cleavage from the PSPPG. In case the capping moiety is present between the first nucleoside and the pseudo solid phase protecting group (PSPPG), it may be attached to the terminal backbone hydroxyl group. An example for such a strategy is the insertion of a 3' GalNac conjugate as descrl-bed in, e.g., WO2009073809. Typical capping moieties may be peptides, saccharides, or lipids.

In the above exemplary structures, R" may be any conceivable substituent to the ribose 2' position. In particular, R" may be selected from the group consisting of: H, an alkyl, a heteroalkyl, an amine, an amide, an ester, a halogen, a hydroxyl, or a thiol moiety. R" may further represent a linker moiety connected to a tagging moiety or form a bridge to the 4' carbon atom of the ribose ring. The skilled person is aware of the multitude of ribose based nucleoside moieties, which are substituted at the 2' position and can be assembled into oligomers with intervening phosphor based moieties. R' is a C1-C10 alkyl or a C1-C10 heteroalkyl. In particular, R' may be a C1-C10 alkyl substituted with an electron withdrawing group, e.g. R' may be a beta-cyanoethyl group (i.e. a 2-cyanoethyl group).

In some embodiments, the compound according to formula I or I-b is selected from the group consisting of formulae VIII to XII depicted below.

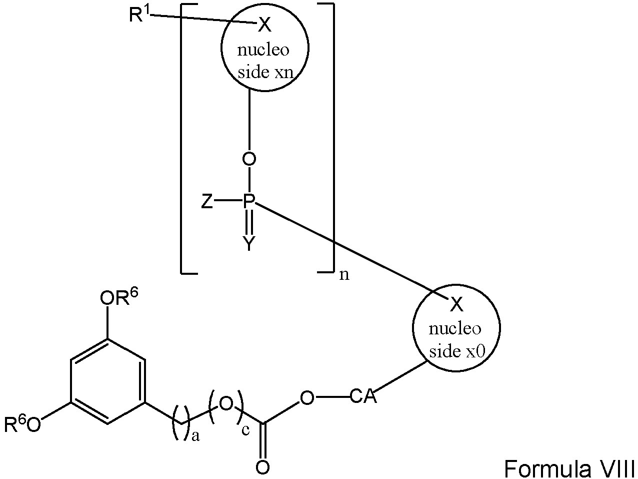

In formula VIII, c is 0 or 1; a is an integer of 1 to 3; R¹ is a protecting group; X is independently at each position O or N; n is an integer equal to or larger than 0; Z is independently at each position H or an electron withdrawing group; Y is independently for each repetitive unit n missing or O or S; each of the nucleosides x0 to xn may be the same or different; CA is a capping moiety or a single bond; and the above definitions of R⁶ apply. In some embodiments, CA is a single bond. In some embodiments, R¹ is a triarylmethyl type protecting group, preferably a protecting group selected from the group consisting of DMT, trityl, and MMT, more preferably a DMT group. In some embodiments, each of R⁶ is independently a C12-C40 aliphatic hydrocarbon group, which optionally comprises one or more heteroatoms. In some embodiments, each of R⁶ is independently an aliphatic hydrocarbon group or a heteroalkyl group having 12 to 40 carbon atoms; preferably 12 to 30 carbon atoms. In some embodiments, each of R⁶ is independently an aliphatic hydrocarbon group having 12 to 30 carbon atoms. In some embodiments, R⁶ is chosen from the structures depicted in Fig 1. In some embodiments, a is 1. In some embodiments, each of R⁶ is each independently a C12 to C40 alkyl group or a C12 to C40 heteroalkyl group. In some embodiments, each of R⁶ is independently a C12 to C40 alkyl group. In some embodiments, X is O and each of R⁶ is independently a C12 to C40 alkyl group or a C12 to C40 heteroalkyl group. In some embodiments, X is O and each of R⁶ is each independently a C12 to C40 alkyl group. In some embodiments, a is 1, X is O, and each of R⁶ is independently a C12 to C40 alkyl group or a C12 to C40 heteroalkyl group. In some embodiments, a is 1, X is O, and each of R⁶ is independently a C12 to C40 alkyl group. In some embodiments, a is an integer of 1 to 3, X is O, and each of R⁶ is each independently a C12 to C40 alkyl group. In some embodiments, a is an integer of 1 to 3, X is O, and each of R⁶ is each independently a linear C12 to C40 alkyl group

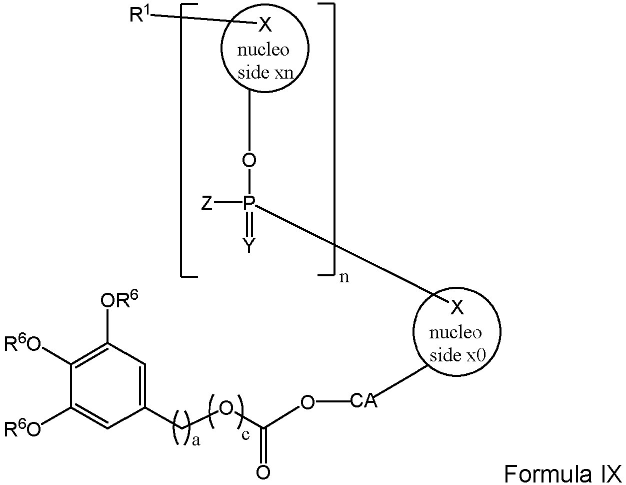

In formula IX, c is 0 or 1; a is an integer of 1 to 3; R¹ is a protecting group; X is independently at each position O or N; n is an integer equal to or larger than 0; Z is independently at each position H or an electron withdrawing group; Y is independently for each repetitive unit n missing or O or S; each of the nucleosides x0 to xn may be the same or different; CA is a capping moiety or a single bond; and the above definitions of R⁶ apply. In some embodiments, CA is a single bond. In some embodiments, R¹ is a triarylmethyl type protecting group, preferably a protecting group selected from the group consisting of DMT, trityl, and MMT, more preferably a DMT group. In some embodiments, each of R⁶ is independently a C12-C40 aliphatic hydrocarbon group, which optionally comprises one or more heteroatoms. In some embodiments, each of R⁶ is independently a C8-C40 aliphatic hydrocarbon group, which optionally comprises one or more heteroatoms.

In some embodiments, each of R⁶ is independently an aliphatic hydrocarbon group or a heteroalkyl group having 8 to 40 carbon atoms; preferably 8 to 30 carbon atoms. In some embodiments, each of R⁶ is independently an aliphatic hydrocarbon group having 8 to 30 carbon atoms. In some embodiments, R⁶ is chosen from the structures depicted in Fig 1. In some embodiments, a is 1. In some embodiments, each of R⁶ is independently a C8 to C40 alkyl group or a C8 to C40 heteroalkyl group. In some embodiments, each of R⁶ is independently a C8 to C40 alkyl group. In some embodiments, X is O and each of R⁶ is independently a C8 to C40 alkyl group or a C8 to C40 heteroalkyl group. In some embodiments, X is O and each of R⁶ is independently a C8 to C40 alkyl group. In some embodiments, a is 1, X is O, and each of R⁶ is independently a C8 to C40 alkyl group or a C8 to C40 heteroalkyl group. In some embodiments, a is 1, X is O, and each of R⁶ is independently a C8 to C40 alkyl group. In some embodiments, a is an integer of 1 to 3, X is O, and each of R⁶ is independently a C8 to C40 alkyl group. In some embodiments, a is an integer of 1 to 3, X is O, and each of R⁶ is independently a linear a C8 to C40 alkyl group.

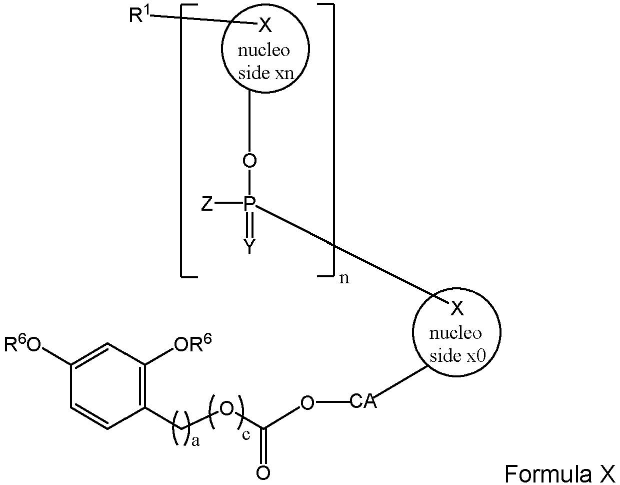

In formula X, c is 0 or 1; a is an integer of 1 to 3, preferably 2 to 3; R¹ is a protecting group; X is independently at each position O or N; n is an integer equal to or larger than 0; Z is independently at each position H or an electron withdrawing group; Y is independently for each repetitive unit n missing or O or S; each of the nucleosides x0 to xn may be the same or different; CA is a capping moiety or a single bond; and the above definitions of R⁶ apply. In some embodiments, a is an integer of 1 to 3. In some preferred embodiments, a is an integer of 2 to 3. In some embodiments, CA is a single bond. In some embodiments, R¹ is a triarylmethyl type protecting group, preferably a protecting group selected from the group consisting of DMT, trityl, and MMT, more preferably a DMT group. In some embodiments, each of R⁶ is independently a C12-C40 aliphatic hydrocarbon group, which optionally comprises one or more heteroatoms. In some embodiments, each of R⁶ is independently an aliphatic hydrocarbon group or a heteroalkyl group having 12 to 40 carbon atoms; preferably 12 to 30 carbon atoms. In some embodiments, each of R⁶ is independently an aliphatic hydrocarbon group having 12 to 30 carbon atoms. In some embodiments, R⁶ is chosen from the structures depicted in Fig 1. In some embodiments, a is 1. In some embodiments, each of R⁶ is independently a C12 to C40 alkyl group or a C12 to C40 heteroalkyl group. In some embodiments, each of R⁶ is independently a C12 to C40 alkyl group. In some embodiments, X is O and each of R⁶ is independently a C12 to C40 alkyl group or a C12 to C40 heteroalkyl group. In some embodiments, X is O and each of R⁶ is independently a C12 to C40 alkyl group. In some embodiments, a is 1, X is O, and each of R⁶ is independently a C12 to C40 alkyl group or a C12 to C40 heteroalkyl group. In some embodiments, a is 1, X is O, and each of R⁶ is independently a C12 to C40 alkyl group. In some embodiments, a is an integer of 1 to 3, X is O, and each of R⁶ is independently a C12 to C40 alkyl group. In some embodiments, a is an integer of 1 to 3, X is O, and each of R⁶ is independently a linear C12 to C40 alkyl group.

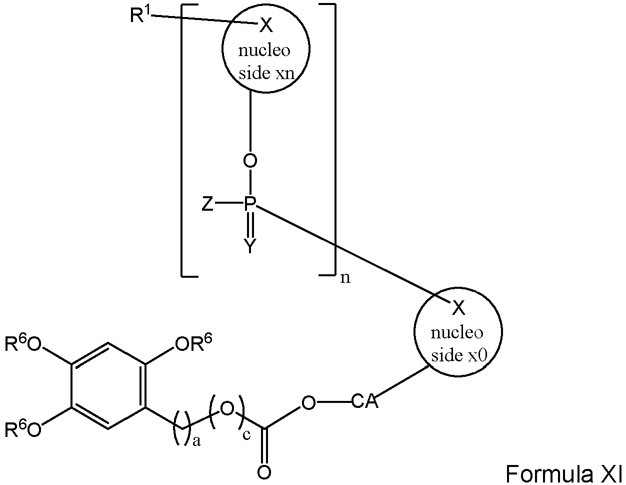

In formula XI, c is 0 or 1; a is an integer of 1 to 3, preferably 2 to 3; R¹ is a protecting group; X is independently at each position O or N; n is an integer equal to or larger than 0; Z is independently at each position H or an electron withdrawing group; Y is independently for each repetitive unit n missing or O or S; each of the nucleosides x0 to xn may be the same or different; CA is a capping moiety or a single bond; and the above definitions of R⁶ apply. In some preferred embodiments, a is an integer of 2 to 3. In some embodiments, CA is a single bond. In some embodiments, R¹ is a triarylmethyl type protecting group, preferably a protecting group selected from the group consisting of DMT, trityl, and MMT, more preferably a DMT group. In some embodiments, each of R⁶ is independently a C12-C40 aliphatic hydrocarbon group, which optionally comprises one or more heteroatoms. In some embodiments, each of R⁶ is independently a C8-C40 aliphatic hydrocarbon group, which optionally comprises one or more heteroatoms. In some embodiments, each of R⁶ is independently an aliphatic hydrocarbon group or a heteroalkyl group having 8 to 40 carbon atoms; preferably 8 to 30 carbon atoms. In some embodiments, each of R⁶ is independently an aliphatic hydrocarbon group having 8 to 30 carbon atoms. In some embodiments, R⁶ is chosen from the structures depicted in Fig 1. In some embodiments, a is 1. In some embodiments, each of R⁶ is independently a C8 to C40 alkyl group or a C8 to C40 heteroalkyl group. In some embodiments, each of R⁶ is independently a C8 to C40 alkyl group. In some embodiments, X is O and each of R⁶ is each independently a C8 to C40 alkyl group or a C8 to C40 heteroalkyl group. In some embodiments, X is O and each of R⁶ is independently a C8 to C40 alkyl group. In some embodiments, a is 1, X is O, and each of R⁶ is independently a C8 to C40 alkyl group or a C8 to C40 heteroalkyl group. In some embodiments, a is 1, X is O, and each of R⁶ is independently a C8 to C40 alkyl group. In some embodiments, a is an integer of 1 to 3, X is O, and each of R⁶ is independently a C8 to C40 alkyl group. In some embodiments, a is an integer of 1 to 3, X is O, and each of R⁶ is independently a linear C8 to C40 alkyl group.

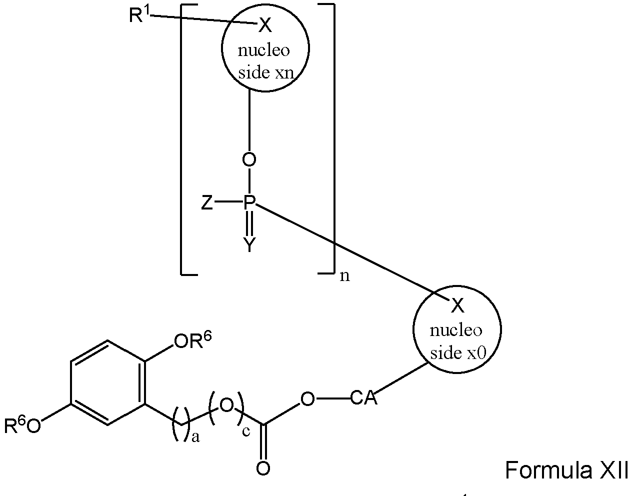

In formula XII, a is an integer of 1 to 3; R¹ is a protecting group; X is independently at each position O or N; n is an integer equal to or larger than 0; Z is independently at each position H or an electron withdrawing group; Y is independently for each repetitive unit n missing or O or S; each of the nucleosides x0 to xn may be the same or different; CA is a capping moiety or a single bond; and the above definitions of R⁶ apply. In some embodiments, CA is a single bond. In some embodiments, R¹ is a triarylmethyl type protecting group, preferably a protecting group selected from the group consisting of DMT, trityl, and MMT, more preferably a DMT group. In some embodiments, each of each of R⁶ is independently a C12-C40 aliphatic hydrocarbon group, which optionally comprises one or more heteroatoms. In some embodiments, each of R⁶ is independently an aliphatic hydrocarbon group or a heteroalkyl group having 12 to 40 carbon atoms; preferably 12 to 30 carbon atoms. In some embodiments, each of R⁶ is independently an aliphatic hydrocarbon group having 12 to 30 carbon atoms. In some embodiments, R⁶ is chosen from the structures depicted in Fig 1. In some embodiments, a is 1. In some embodiments, each of R⁶ is independently a C12 to C40 alkyl group or a C12 to C40 heteroalkyl group. In some embodiments, each of R⁶ is independently a C12 to C40 alkyl group. In some embodiments, X is O and each of R⁶ is each independently a C12 to C40 alkyl group or a C12 to C40 heteroalkyl group. In some embodiments, X is O and each of R⁶ is independently a C12 to C40 alkyl group. In some embodiments, a is 1, X is O, and each of R⁶ is independently a C12 to C40 alkyl group or a C12 to C40 heteroalkyl group. In some embodiments, a is 1, X is O, and each of R⁶ is independently a C12 to C40 alkyl group. In some embodiments, a is an integer of 1 to 3, X is O, and each of R⁶ is independently a C12 to C40 alkyl group. In some embodiments, a is an integer of 1 to 3, X is O, and each of R⁶ is independently a linear C12 to C40 alkyl group.

In some embodiments the compound according to formula I is a compound represented by any one formulae VIII to XII, wherein c is 1. In some embodiments the compound according to formula I is a compound represented by any one formulae VIII to XII, wherein c is 0.

In some embodiments, the compound according to formula I is a compound according to a formula selected from the group consisting formula VIII, formula IX, and formula XII. In some embodiments, the compound according to formula I is a compound according to a formula selected from the group consisting formula VIII, formula IX, and formula XII, and R¹ is a triarylmethyl type protecting group, e.g. DMT, trityl, or MMT. In some embodiments, the compound according to formula I is a compound according to a formula selected from the group consisting formula VIII, formula IX, and formula X. In some embodiments, the compound according to formula I is a compound according to a formula selected from the group consisting formula VIII, formula IX, and formula X, and R¹ is a triarylmethyl type protecting group, e.g. DMT, trityl, or MMT.

In some preferred embodiments, the compound represented by any one of formula I and I-b is a compound according to any one of formulae VIII to XII: 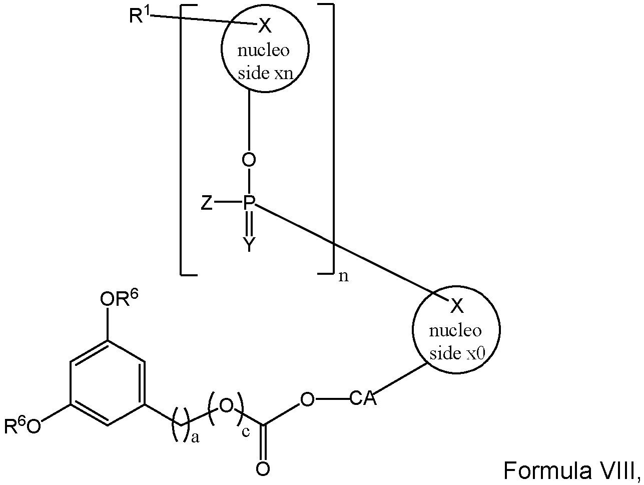 wherein in formula VIII, c is 0 or 1; a is an integer of 1 to 3; each of R⁶ is independently a C12-C40 aliphatic hydrocarbon group; R¹ is a protecting group; X is independently at each position O or N; n is an integer equal to or larger than 0; Z is independently at each position H or an electron withdrawing group; Y is independently for each repetitive unit n missing or O or S; each of the nucleosides x0 to xn may be the same or different; and CA is a capping moiety or a single bond; 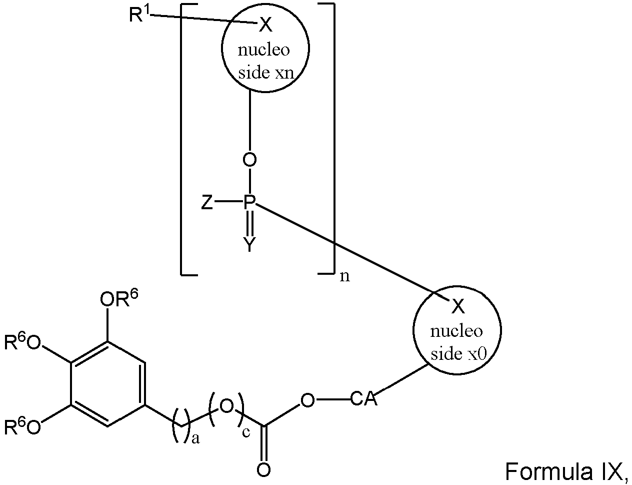
wherein in formula IX, c is 0 or 1; a is an integer of 1 to 3; each of R⁶ is independently a C8-C40 aliphatic hydrocarbon group, preferably a C12-C40 aliphatic hydrocarbon group;
X is independently at each position O or N; n is an integer equal to or larger than 0; Z is independently at each position H or an electron withdrawing group; Y is independently for each repetitive unit n missing or O or S; each of the nucleosides x0 to xn may be the same or different; and CA is a capping moiety or a single bond; 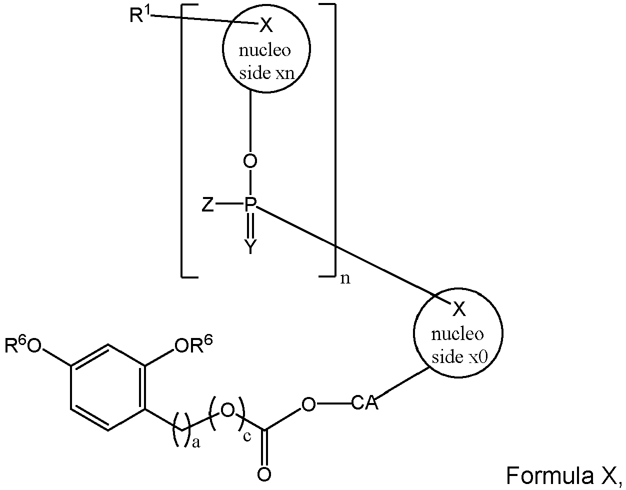
wherein in formula X, c is 0 or 1; a is an integer of 2 to 3; each of R⁶ is independently a C12-C40 aliphatic hydrocarbon group; R¹ is a protecting group; X is independently at each position O or N; n is an integer equal to or larger than 0; Z is independently at each position H or an electron withdrawing group; Y is independently for each repetitive unit n missing or O or S; each of the nucleosides x0 to xn may be the same or different; and CA is a capping moiety or a single bond; 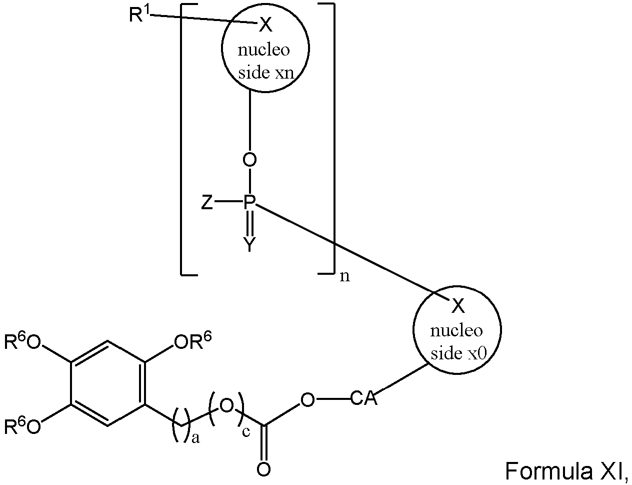
wherein in formula XI, c is 0 or 1; a is an integer of 2 to 3; each of R⁶ is independently a C8-C40 aliphatic hydrocarbon group, preferably a C12-C40 aliphatic hydrocarbon group;
R¹ is a protecting group; X is independently at each position O or N; n is an integer equal to or larger than 0; Z is independently at each position H or an electron withdrawing group;
Y is independently for each repetitive unit n missing or O or S ; each of the nucleosides x0 to xn may be the same or different; and CA is a capping moiety or a single bond; and 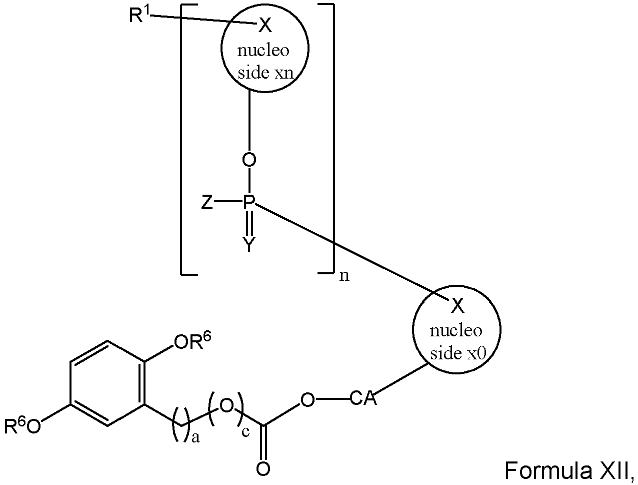
wherein in formula XII, c is 0 or 1; a is an integer of 1 to 3; each of R⁶ is independently a C12-C40 aliphatic hydrocarbon group, which optionally comprises one or more heteroatoms; R¹ is a protecting group; X is independently at each position O or N; n is an integer equal to or larger than 0; Z is independently at each position H or an electron withdrawing group; Y is independently for each repetitive unit n missing or O or S; each of the nucleosides x0 to xn may be the same or different; and CA is a capping moiety or a single bond.

In some embodiments of the compound according to any one of formulae VIII to XII, if c is 0, a is an integer of 1 to 2 (i.e. a is 1 or 2). In some embodiments, in formula IX, c is always 1. In some embodiments, in formula IX, c is always 0.

In some preferred embodiments, the compound represented by formula I is a compound according to any one of formulae VIII, IX, and X.

In some embodiments, the compound of formula I, in particular the compound of formula I-b, is a compound of the following formula I-b-1: 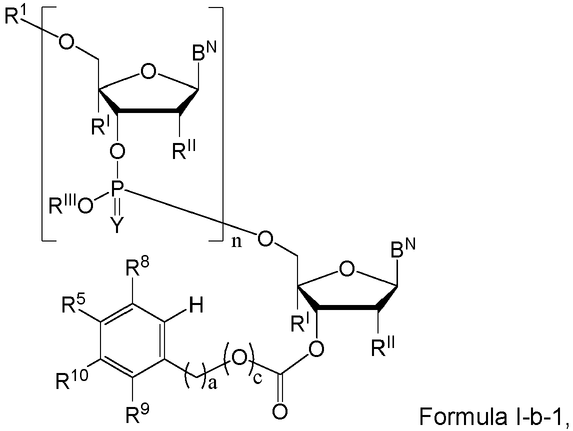 wherein in formula I-b-1:
the integers a and c, as well as R⁵, R⁸, R⁹, R¹⁰, and R⁶ are defined as for formula I-b;
R¹ is a triarylmethyl type protecting group;
n is an integer equal to or larger than 0;
Y is independently for each repetitive unit n O or S;
B^{N} is a nucleobase, which may be the same or different at each occurrence;
R^{III} is a hydroxyl protecting group, which may be the same or different at each occurrence;
R^{II} is at each occurrence independently selected from the group consisting of H, F,
-O-(C1-C5 alkyl), -O-(C1-C5 alkyl)-O-(C1-C5 alkyl), -O-Si(C1-C5 alkyl)₃, and -O-CH₂-O-Si(C1-C5 alkyl)₃; and
R^{I} is at each occurrence independently H or R^{II} and R^{I} together form a methylene or ethylene bridge of the chemical structure +-CH₂-O-++, +-CH(CH₃)-O-++, or +-CH₂-CH₂-O-++, where + is the point of attachment to the 4'-carbon atom (i.e. the carbon atom to which RI is bonded) and ++ is the point of attachment to the 2'-carbon atom (i.e. the carbon atom to which R^{II} is bonded).

The term "the integers a and c, as well as R⁵, R⁸, R⁹, R¹⁰, and R⁶ are defined as for formula I-b" will be understood to mean that any definitions and embodiments relating to the integers a and c and/or R⁵, R⁸, R⁹, R¹⁰ R⁶ in the context of a compound of formula I-b may also apply to the integers a and c and/or R⁵, R⁸, R⁹, R¹⁰, R⁶ in the context of a compound of formula I-b-1.

In some embodiments of the compound of formula I-b-1, c is 1 or 0, wherein:
- R⁵ is O-R⁶, R⁸ and R¹⁰ are both O-R⁶, and R⁹ is H, wherein a is an integer of 1 to 3 and R⁶ is at each occurrence independently a C8-C40 aliphatic hydrocarbon group;
- R⁵ is O-R⁶, R⁸ and R¹⁰ are both H, and R⁹ is O-R⁶, wherein a is an integer of 2 to 3 and R⁶ is at each occurrence independently a C12-C40 aliphatic hydrocarbon group; or
- R⁵ is H, R⁸ and R¹⁰ are both O-R⁶ and R⁹ is H, wherein a is an integer of 1 to 3 and R⁶ is at each occurrence independently a C12-C40 aliphatic hydrocarbon group.

In some embodiments of the compound of formula I-b-1, c is 1 or 0, wherein:
- R⁵ is O-R⁶, R⁸ and R¹⁰ are both O-R⁶, and R⁹ is H, wherein a is an integer of 1 to 3 and R⁶ is at each occurrence independently a linear C18-C22 alkyl group;
- R⁵ is O-R⁶, R⁸ and R¹⁰ are both H, and R⁹ is O-R⁶, wherein a is an integer of 2 to 3 and R⁶ is at each occurrence independently a linear C18-C22 alkyl group; or
- R⁵ is H, R⁸ and R¹⁰ are both O-R⁶ and R⁹ is H, wherein a is an integer of 1 to 3 and R⁶ is at each occurrence independently a linear C18-C22 alkyl group.

In some embodiments of the compound of formula I-b-1, c is 1. In some embodiments of the compound of formula I-b-1, c is 0.

In some embodiments of the compound of formula I-b-1:
n is an integer in the range of 0-200, 0-150, 0-120, 0-100, 0-90, 0-80, 0-70, 0-60, 0-50, 0-40, 0-30, 0-20, 0-10, or 0-1;
Y is independently for each repetitive unit n O or S;
B^{N} is selected independently at each occurrence from the group consisting of adenine, guanine, cytosine, thymine, and uracil;
R^{III} is for each repetitive unit n a 2-cyanoethyl group; and
R^{II} is at each occurrence independently selected from the group consisting of H, F, -O-CH₃ (i.e. methoxy), -O-CH₂-CH₂-O-CH₃ (i.e. 2-methoxyethyl-1-oxy),
-O-Si(CH₃)₃ (i.e. trimethylsilyloxy, TMS), -O-Si(CH₃)₂(C(CH₃)₃) (i.e. *tert-*butyl(dimethyl)silyloxy, TBDMS or TBS), and -O-CH₂-O-Si(C(CH₃)₃)₃ (i.e. ((triisopropylsilyl)oxy)-methyloxy, TOM); and
R^{I} is H at each occurrence.

In some embodiments of the compound of formula I-b-1, c is 1. In some embodiments of the compound of formula I-b-1, c is 0.

When stating that a nucleobase such as B^{N} "is selected independently at each occurrence from the group consisting of adenine, guanine, cytosine, thymine, and uracil", said nucleobases may optionally be protected. In particular, the exocyclic amino group of adenine, guanine, and cytosine may be protected. Examples of suitable protecting groups are listed in Table 1 (vide infra).

In some embodiments of the compound of formula I-b-1:
n is an integer of 0 or 1;
Y is independently for each repetitive unit n O or S;
B^{N} is selected independently at each occurrence from the group consisting of adenine, guanine, cytosine, thymine, and uracil;
R^{III} is for each repetitive unit n a 2-cyanoethyl group;
R^{II} is at each occurrence independently selected from the group consisting of H, -O-CH₃ (i.e. methoxy), and -O-Si(CH₃)₂(C(CH₃)₃) (i.e. tert-butyl(dimethyl)silyloxy, TBDMS or TBS); and
R^{I} is H at each occurrence.

In some embodiments of the compound of formula I-b-1:
n is an integer of 0 or 1;
Y is independently for each repetitive unit n O;
B^{N} is selected independently at each occurrence from the group consisting of adenine, in which the exocyclic amino group is benzoyl-protected, guanine, in which the exocyclic amino group is isobutyryl-protected, cytosine, in which the exocyclic amino group is benzoyl- or acetyl-protected, thymine, and uracil;
R^{III} is for each repetitive unit n a 2-cyanoethyl group;
R^{II} is at each occurrence independently selected from the group consisting of H, -O-CH₃ (i.e. methoxy), and -O-Si(CH₃)₂(C(CH₃)₃) (i.e. tert-butyl(dimethyl)silyloxy, TBDMS or TBS); and
R^{I} is H at each occurrence.

In some embodiments of the compound according to formula I-b-1, if c is 0, a is an integer of 1 to 2 (i.e. a is 1 or 2).

In some embodiments, the compound of formula I, in particular the compound of formula I-b-1, is a compound according to any one of the following formulae VIII-1, IX-1, and X-1: 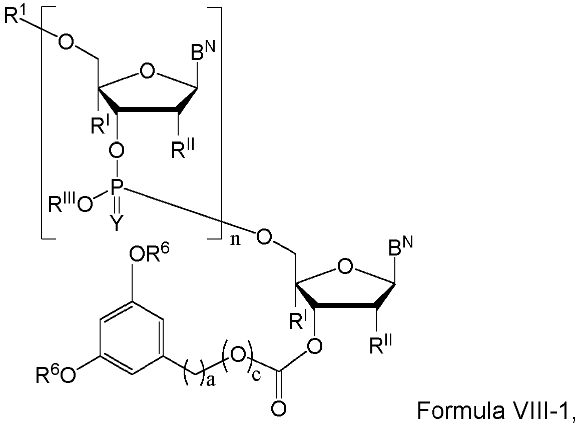 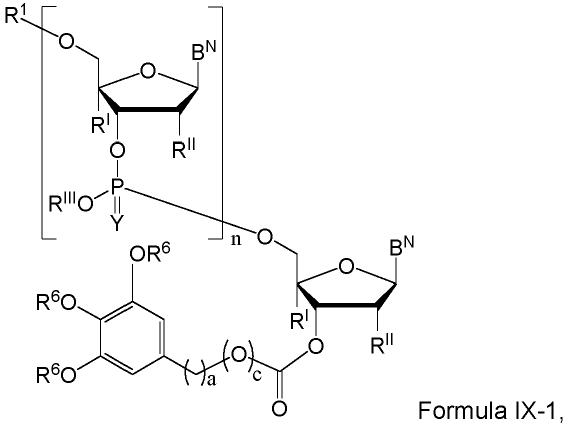 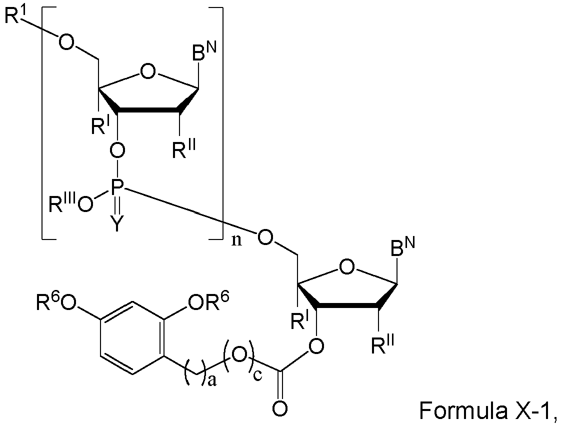 wherein
in formula VIII-1:
   the integers a and c as well as R⁶ are defined as for formula VIII;
in formula IX-1:
   the integers a and c as well as R⁶ are defined as for formula IX;
in formula X-1:
   the integers a and c as well as R⁶ are defined as for formula X; and
   wherein in formulae VIII-1, IX-1, and X-1, the integer n as well as R¹, Y, B^{N}, R^{I}, R^{II}, and
   R^{III} are defined as for formula I-b-1.

Thus, any definitions and embodiments relating to any one of a, c and R⁶ in the context of formula VIII may also apply to the integers a and c as well as to R⁶ in the context of formula VIII-1. Any definitions and embodiments relating to any one of a, c, and R⁶ in the context of formula IX may also apply to the integers a and c as well as to R⁶ in the context of formula IX-1. Any definitions and embodiments relating to any one of a, c, and R⁶ in the context of formula X may also apply to the integers a and c as well as to R⁶ in the context of formula X-1. Along the same lines, any definitions and embodiments relating to any one of n, R¹, Y, B^{N}, R^{I}, R^{II}, and R^{III} in the context of formula I-b-1 may also apply to the integer n as well as to R¹, Y, B^{N}, R^{I}, R^{II}, and R^{III} in the context of any one of formula VIII-1, IX-1, and X-1.

In some embodiments of the compound of formula VIII-1, IX-1, or X-1, c is 1. In some embodiments of the compound of formula VIII-1, IX-1, or X-1, c is 0. In some embodiments of the compound of formula VIII-1, IX-1, or X-1, if c is 0, a is an integer of 1 to 2 (i.e. a is 1 or 2).

In another aspect, the present application relates to a method for the synthesis of oligonucleotides, comprising steps a) through f):
a) providing a nucleoside, nucleotide, oligonucleotide, or conjugate of a nucleoside, nucleotide or oligonucleotide bound to a pseudo solid phase protecting group according to any one of formulae II, II-1, II-2, II-a, II-a-1, II-a-2, and III to VII, wherein the nucleoside, nucleotide, oligonucleotide, or conjugate thereof comprises a backbone hydroxyl or amine moiety, which is protected by a protecting group R¹;
b) cleaving the protecting group R¹ from the compound provided in the previous step, thereby generating a free backbone hydroxyl or amine group;
c) reacting the free backbone hydroxyl or amine group generated in step b) with a phosphorus moiety of a nucleoside or oligonucleotide building block, which building block further comprises a backbone hydroxyl or amine moiety protected by a protecting group R¹, thereby producing a covalent linkage between the oxygen or nitrogen atom of said free backbone hydroxyl or amine group and the phosphorus atom of said building block;
d) optionally modifying the phosphorus moiety;
e) optionally reiterating the sequence of steps b) to c) or b) to d); and
f) cleaving the oligonucleotide from the pseudo solid phase protecting group.

The above definitions and explanations relating to the pseudo solid phase protecting groups and compounds of the present invention are likewise applicable to the methods of the present invention and *vice versa.*

As will be understood from what has been laid out above, said "backbone hydroxyl or amine moiety" of the compound provided in step a) is preferably a terminal hydroxyl or amine moiety such as, for example, a DMT-protected 5'-OH group of a ribose-based nucleoside moiety (i.e. 5'-ODMT).

In some embodiments of the method comprising the aforementioned steps a) through f):
said "backbone hydroxyl or amine moiety" of the compound provided in step a) is a backbone hydroxyl moiety,
said "backbone hydroxyl or amine group" of steps b) and c) is a backbone hydroxyl group, and/or
said "backbone hydroxyl or amine moiety" of the nucleoside or oligonucleotide building block of step c) is a backbone hydroxyl moiety.

In some embodiments of the method comprising the aforementioned steps a) through f):
said "backbone hydroxyl or amine moiety" of the compound provided in step a) is a terminal backbone hydroxyl moiety,
said "backbone hydroxyl or amine group" of steps b) and c) is a terminal backbone hydroxyl group, and/or
said "backbone hydroxyl or amine moiety" of the nucleoside or oligonucleotide building block of step c) is a terminal backbone hydroxyl moiety.

In some embodiments of the method comprising the aforementioned steps a) through f), step a) is: providing a nucleoside, nucleotide or oligonucleotide, bound to a pseudo solid phase protecting group according to any one of formulae II, II-1, II-2, II-a, II-a-1, II-a-2, and III to VII, wherein the nucleoside, nucleotide or oligonucleotide comprises a backbone hydroxyl or amine moiety, which is protected by a protecting group R¹. In these embodiments, the term "oligonucleotide" does not comprise conjugates thereof.

It should be understood that the methods of the present invention may be performed in various ways and that the sequence of steps performed may not necessarily correspond to the sequence in which said steps are given in the claims, unless indicated differently. Therefore, any numbering of steps, e.g. a) through f) or i) through iii), is merely introduced for the ease of reference and is not intended to reflect a mandatory sequence of said steps, unless indicated differently.

As has been set out above, R¹ may be a triarylmethyl type protecting group such as, e.g., trityl, methoxytrityl (i.e. (p-methoxyphenyl)diphenylmethyl, MMT), and dimethoxyltrityl (i.e., di(p-methoxyphenyl)phenylmethyl, DMT). R¹ may be the same or different for different iterations of steps b) and c) of the present method. In some embodiments of the method of the invention, the protecting group R¹ is selected from the group consisting of a trityl group, an MMT group, and a DMT group, and may be the same or different for each iteration of step b) and c). In some embodiments of the method of the invention, the protecting group R¹ is a DMT group. In some embodiments of the method of the invention, R¹ is a trityl group whenever R¹ is a protecting group for an amine moiety and R¹ is a DMT group whenever R¹ is a protecting group for a hydroxyl moiety. Preferably, step a) involves providing the compound in an aprotic solvent or a mixture of aprotic solvents. While such solvents are not particularly limited, halogenated hydrocarbon solvents such as dichloromethane,1,2-dichloroethane, chloroform, carbon tetrachloride and the like, aromatic hydrocarbon solvents such as benzene, toluene, xylene (o-, m-, or p-xylene), mesitylene, and the like, aliphatic hydrocarbon solvents such as pentane, hexane, cyclohexane, heptane, octane, and the like, ether solvents such as diethyl ether, tetrahydrofuran, cyclopentyl methyl ether, and the like, and ester solvents such as ethyl acetate, isopropyl acetate, and the like may for example be used.

Step a) of the methods described herein comprises providing a nucleoside, nucleotide or oligonucleotide, or conjugate thereof bound to a pseudo solid phase protecting group as disclosed herein. This may be understood in the broadest possible sense.

For example, if said nucleoside, nucleotide, oligonucleotide, or conjugate of a nucleoside, nucleotide or oligonucleotide provided in step a) is bound to a pseudo solid phase protecting group according to formula II, wherein c is 1, step a) may involve a method according to the following steps i) to iii):
i) Providing a nucleoside, nucleotide, oligonucleotide, or conjugate thereof comprising a first free backbone hydroxyl moiety, a second backbone hydroxyl or amine moiety, which is protected by a protecting group R¹, and optionally further protecting groups, which are orthogonal to R¹;
ii) Reacting the first free hydroxyl moiety with an activated carbonic acid derivative; and
iii) Reacting the product of step ii) with a compound of formula XIII 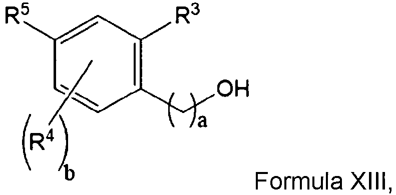 wherein in formula XIII:
   a is an integer of 1 to 3;
   R³ is H;
   R⁵ is selected from the group consisting of H, and O-R⁶, where R⁶ is a C8-C40 aliphatic hydrocarbon group;
   each of R⁴ is independently O-R⁶, where R⁶ is a C8-C40 aliphatic hydrocarbon group;
   b is 1 to 3;
   with the proviso that if b is 1, at least one of R³ and R⁵ is not H;
   and with the further proviso that the sum of all carbon atoms contained in the R³, R^{4,} and R⁵ moieties present is larger than 23 and smaller than 200.

Thus, further aspects of the invention pertain to a method for preparing the compound according to formula I, wherein c is 1, comprising the following steps i) to iii):
i) Providing a nucleoside or oligonucleotide comprising a first free backbone hydroxyl moiety, a second backbone hydroxyl or amine moiety, which is protected by a protecting group R¹, and optionally further protecting groups, which are orthogonal to R¹;
ii) Reacting the first free hydroxyl moiety with an activated carbonic acid derivative; and
iii) Reacting the product of step ii) with a compound of formula XIII 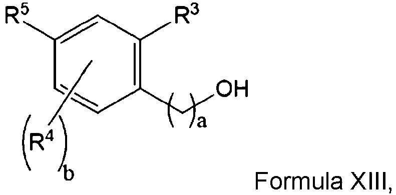 wherein:
   a is an integer of 1 to 3;
   R³ is H;
   R⁵ is O-R⁶ or H where R⁶ is a C8-C40 aliphatic hydrocarbon group;
   each of R⁴ is independently O-R⁶, where R⁶ is at each occurrence independently a C8-C40 aliphatic hydrocarbon group;
   b is 1 to 3;
   with the proviso that if b is 1, at least one of R³ and R⁵ is not H;
   and with the further proviso that the sum of all carbon atoms contained in the R³, R⁴, and R⁵ moieties present is larger than 23 and smaller than 200.

As used herein, two protecting groups are considered "orthogonal", if they may be removed independently from each other, i.e. using different sets of reaction conditions. Nucleosides and oligonucleotides with an acid-labile backbone protecting group such as, e.g., DMT and acid resistant protecting groups for the phosphate and nucleobase moieties are easily available to the skilled artisan.

The activated carbonic acid derivative used in step ii) may be phosgene or an activated diester of carbonic acid such as, e.g., disuccinimidyl carbonate or an activated diamide of carbonic acid such as, e.g., 1,1'-carbonyldiimidazole. In this context, the activated carbonic acid derivative is a compound, in which a carbonyl carbon atom is bonded to two leaving groups (e.g. two -Cl residues in phosgene or two N-hydroxysuccinimide residues in disuccinimidyl carbonate or two imidazole residues in 1,1'-carbonyldiimidazole), both of which may be substituted by nucleophilic attack of a free hydroxyl group. The skilled artisan will understand that the first such leaving group may be substituted by said first free hydroxyl moiety in step ii), and the second such leaving group may be substituted by the hydroxyl group of the compound of formula XIII in step iii), so as to arrive at a desired carbonic acid diester.

Preferably, in step i), the second backbone moiety is a hydroxyl moiety. Preferably, the reaction is carried out in an aprotic solvent.

It will be understood by a person skilled in the art, that any definitions and embodiments relating to the integers a and b as well as to the residues R³, R⁴, R⁵, R⁶, and R⁷ in the context of the pseudo solid phase protecting group of formula II may also apply to the compound of formula XIII. Likewise, any definitions and embodiments pertaining to the protecting group R¹ in the context of the compound of formula I may also apply to the protecting group R¹ of step i).

In some embodiments of the method comprising the aforementioned steps i) to iii), the method is a method for preparing the compound according to formula I-b, wherein c is 1, and the compound of formula XIII is a compound o formula XIII-b: 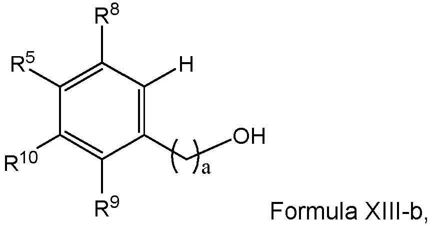 wherein in formula XIII-b, the integer a as well as the residues R⁵, R⁸, R⁹, R¹⁰, and R⁶ are defined as for formula I-b. This is to mean that any definitions and embodiments relating to the integer a as well as the residues R⁵, R⁸, R⁹, R¹⁰, and R⁶ in the context of formula I-b may also apply to the compound of formula XIII-b.

In some embodiments of the method comprising the aforementioned steps i) to iii), the method is a method for preparing the compound according to any one of formulae VIII to XII, preferably VIII to X, wherein c is 1, and wherein:
- the compound of formula XIII is a compound o formula XIII-c, if a compound of formula VIII is to be prepared: 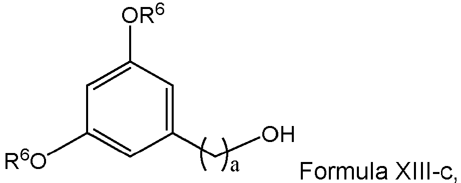 wherein in formula XIII-c, the integer a as well as the residues R⁶ are defined as for formula VIII;
- the compound of formula XIII is a compound o formula XIII-d, if a compound of formula IX is to be prepared: 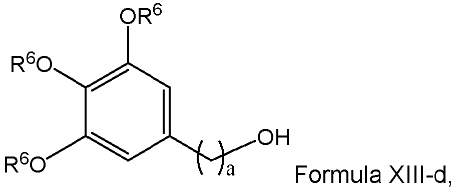 wherein in formula XIII-d, the integer a as well as the residues R⁶ are defined as for formula IX;
- the compound of formula XIII is a compound o formula XIII-e, if a compound of formula X is to be prepared: 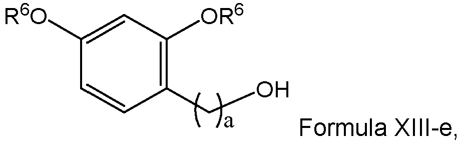 wherein in formula XIII-e, the integer a as well as the residues R⁶ are defined as for formula X;
- the compound of formula XIII is a compound o formula XIII-f, if a compound of formula XI is to be prepared: 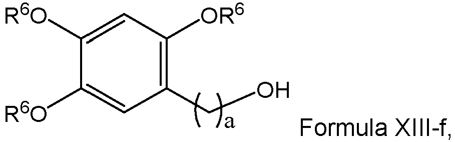 wherein in formula XIII-f, the integer a as well as the residues R⁶ are defined as for formula XI; and
- the compound of formula XIII is a compound o formula XIII-g, if a compound of formula XII is to be prepared: 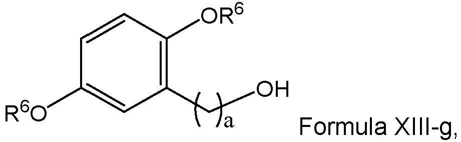 wherein in formula XIII-g, the integer a as well as the residues R⁶ are defined as for formula XII.

In some embodiments of the method comprising the aforementioned steps i) to iii), step i) is: Providing a nucleoside or oligonucleotide comprising a first free backbone hydroxyl moiety, a second backbone hydroxyl or amine moiety, which is protected by a protecting group R¹, and optionally further protecting groups, which are orthogonal to R¹. In such embodiments, the term "oligonucleotide" does preferably not embrace conjugates thereof. In some embodiments of the method comprising the aforementioned steps i) to iii), step i) is: Providing a nucleoside comprising a first free backbone hydroxyl moiety, a second backbone hydroxyl or amine moiety, which is protected by a protecting group R¹, and optionally further protecting groups, which are orthogonal to R¹.

In some embodiments of the method comprising the aforementioned steps i) to iii), at least steps ii) and iii) are performed in an aprotic non-nucleophilic solvent. Examples of suitable solvents for this purpose may be halogenated hydrocarbon solvents such as dichloromethane,1,2-dichloroethane, chloroform, carbon tetrachloride and the like, aromatic hydrocarbon solvents such as benzene, toluene, xylene (o-, m-, or p-xylene), mesitylene, and the like, aliphatic hydrocarbon solvents such as pentane, hexane, cyclohexane, heptane, octane, and the like.

For example, if said nucleoside, nucleotide, oligonucleotide, or conjugate of a nucleoside, nucleotide or oligonucleotide provided in step a) is bound to a pseudo solid phase protecting group according to formula II, wherein c is 0, step a) may involve a method according to the following steps I) and II):
I) Providing a nucleoside, nucleotide, oligonucleotide, or conjugate thereof comprising a first free backbone hydroxyl moiety, a second backbone hydroxyl or amine moiety, which is protected by a protecting group R¹, and optionally further protecting groups, which are orthogonal to R¹, and
II) Reacting the compound provided in step I) with a compound of formula XIV, under conditions suitable to form an ester bond between the oxygen atom of said first free backbone hydroxyl moiety of the compound provided in step I) and the carboxyl or carbonyl carbon atom of formula XIV to which R^{L} is bonded: 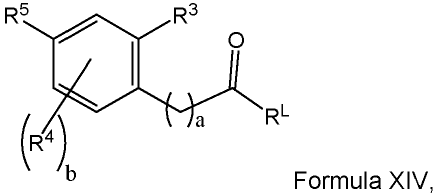 wherein in formula XIV:
   R^{L} is selected from the group consisting of -OH and a leaving group capable of being substituted by a hydroxyl moiety during ester bond formation;
   a is an integer of 1 to 3;
   R³ is H;
   R⁵ is selected from the group consisting of H, and O-R⁶, where R⁶ is a C8-C40 aliphatic hydrocarbon group;
   each of R⁴ is independently O-R⁶, where R⁶ is a C8-C40 aliphatic hydrocarbon group;
   b is 1 to 3;
   with the proviso that if b is 1, at least one of R³ and R⁵ is not H;
   and with the further proviso that the sum of all carbon atoms contained in the R³, R⁴, and R⁵ moieties present is larger than 23 and smaller than 200.

It will be understood by a person skilled in the art, that any definitions and embodiments relating to the integers a and b as well as to the residues R³, R⁴, R⁵, R⁶, and R⁷ in the context of the pseudo solid phase protecting group of formula II may also apply to the compound of formula XIV. Likewise, any definitions and embodiments pertaining to the protecting group R¹ in the context of the compound of formula I may also apply to the protecting group R¹ of step I).

Thus, further aspects of the invention pertain to a method for preparing the compound according to formula I, wherein c is 0, comprising the following steps I) and II):
I) Providing a nucleoside or oligonucleotide comprising a first free backbone hydroxyl moiety, a second backbone hydroxyl or amine moiety, which is protected by a protecting group R¹, and optionally further protecting groups, which are orthogonal to R¹; and
II) Reacting the compound provided in step I) with a compound of formula XIV, under conditions suitable to form an ester bond between the oxygen atom of said first free backbone hydroxyl moiety of the compound provided in step I) and the carboxyl or carbonyl carbon atom of formula XIV to which R^{L} is bonded: 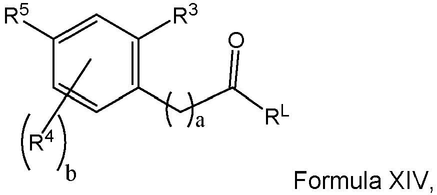 wherein in formula XIV:
   R^{L} is selected from the group consisting of -OH and a leaving group capable of being substituted by a hydroxyl moiety during ester bond formation;
   a is an integer of 1 to 3;
   R³ is H;
   R⁵ is O-R⁶ or H where R⁶ is a C8-C40 aliphatic hydrocarbon group;
   each of R⁴ is independently O-R⁶, where R⁶ is at each occurrence independently a C8-C40 aliphatic hydrocarbon group;
   b is 1 to 3;
   with the proviso that if b is 1, at least one of R³ and R⁵ is not H;
   and with the further proviso that the sum of all carbon atoms contained in the R³, R⁴, and R⁵ moieties present is larger than 23 and smaller than 200.

A person skilled in the art is familiar with leaving groups R^{L} suitable to be substituted by a hydroxyl moiety during ester bond formations and knows which (reaction) conditions are suitable for this purpose. Non-limiting examples of suitable leaving groups R^{L} comprise halogen atoms such as Cl, Br, and F, wherein Cl may be preferred. If R^{L} is a halogen atom, preferably Cl, the ester bond forming reaction of step II) may be performed in the presence of a non-nucleophilic base such as pyridine, collidine, trimethylamine (TEA), and diisopropylethylamine (DIPEA).

If R^{L} is -OH (i.e. a hydroxyl group), the carboxyl group of the compound of formula XIV may be activated towards ester bond formation. In such cases, the ester bond forming reaction of step II) may be performed in the presence of one or more activating agents, also referred to as coupling agents, and optionally one or more additives.

The coupling agent may for example be a carbodiimide coupling agent as known to those skilled in the art. Examples of suitable carbodiimide coupling agents comprise N,N'-diisopropylcarbodiimide (DIC), N,N'-dicyclohexylcarbodiimide (DCC), and N-(3-dimethylaminopropyl)-N'-ethylcarbodiimide (EDC), or mixtures thereof. As known to those skilled in the art, such carbodiimide coupling agents may be used alongside coupling additives such as, e.g., 4-(dimethylamino)pyridine (DMAP), 2-cyano-2-(hydroxyimino)acetate (Oxyma), 1-hydroxybenzotriazole (HOBt), 1-hydroxy-7-azabenzotriazole (HOAt), 5-(hydroxyimino)-1,3-dimethylpyrimidine-2,4,6(1H,3H,5H)-trione (Oxyma-B), ethyl 1-hydroxy-1,2,3-triazole-4-carboxylate (HOCt), N-hydroxysuccinimide (HOSu), and mixtures thereof. Mixtures of DCC and DMAP or DIC and DMAP are for example commonly used to achieve ester bond formation between carboxylic acids and alcohols.

As an alternative to the carbodiimide coupling agents, so-called aminium / uronium salts are commonly used as coupling reagents. In this case, the addition of a non-nucleophilic base such as pyridine, collidine, TEA, and/or DIPEA is common. Further additives such as DMAP may optionally be added as well. Examples of aminium / uronium salts comprise (benzotriazolyl)tetramethyluronium tetrafluoroborate (TBTU), N-[(7-aza-1H-benzotriazol-1-yl)(dimethylamino)-methylene]-N-methylmethanaminium tetrafluoroborate N-oxide (TATU), 2-(1H-benzotriazol-1-yl)-1,1,3,3-tetramethyluronium hexafluorophosphate (HBTU), N-[(7-aza-1H-benzotriazol-1-yl)(dimethylamino)-methylene]-N-methylmethanaminium hexafluorophosphate N-oxide (HATU), and 1-cyano-2-ethoxy-2-oxoethylidenaminooxy)dimethylamino-morpholino-carbenium-hexafluorophosphate (COMU). For example, a mixture of COMU, DIPEA, and DMAP may be used.

Instead of aminium / uranium salts, phosphonium salts such as Benzotriazol-1-yl-oxy-tris-pyrrolidino-phosphonium-hexafluorphosphat (PyBOP) may be used. As another activating agent, MSNT (1-(mesitylene-2-sulfonyl)-3-nitro-1,2,4-triazole) may be used, e.g. alongside N-methylimidazole as additive.

In some embodiments of the method comprising the aforementioned steps I) and II), the method is a method for preparing the compound according to formula I-b, wherein c is 0, and the compound of formula XIV is a compound o formula XIV-b: 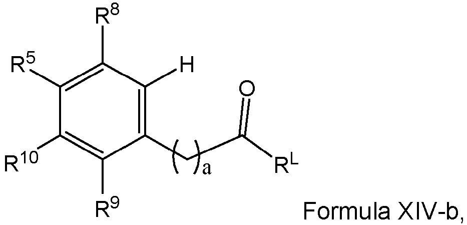 wherein in formula XIV-b,
the integer a as well as the residues R⁵, R⁸, R⁹, R¹⁰, and R⁶ are defined as for formula I-b; and
R^{L} Is defined as for formula XIV.

Thus, any definitions and embodiments relating to the integer a as well as the residues R⁵, R⁸, R⁹, R¹⁰, and R⁶ in the context of formula I-b may also apply to the compound of formula XIV-b.

In some embodiments of the method comprising the aforementioned steps I) and II), the method is a method for preparing the compound according to any one of formulae VIII to XII, preferably VIII to X, wherein c is 0, and wherein:
- the compound of formula XIV is a compound o formula XIV-c, if a compound of formula VIII is to be prepared: 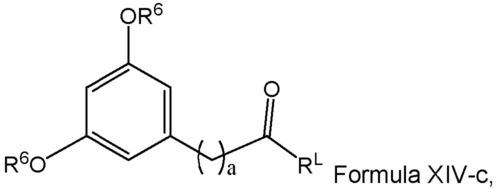 wherein in formula XIV-c, the integer a as well as the residues R⁶ are defined as for formula VIII and R^{L} is defined as for formula XIV;
- the compound of formula XIV is a compound o formula XIV-d, if a compound of formula IX is to be prepared: 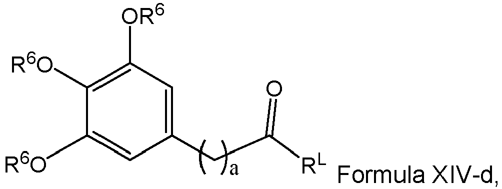 wherein in formula XIV-d, the integer a as well as the residues R⁶ are defined as for formula IX and R^{L} is defined as for formula XIV;
- the compound of formula XIV is a compound o formula XIV-e, if a compound of formula X is to be prepared: 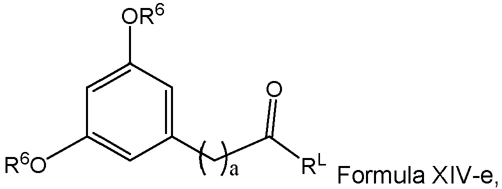 wherein in formula XIV-e, the integer a as well as the residues R⁶ are defined as for formula X and R^{L} is defined as for formula XIV;
- the compound of formula XIV is a compound o formula XIV-f, if a compound of formula XI is to be prepared: 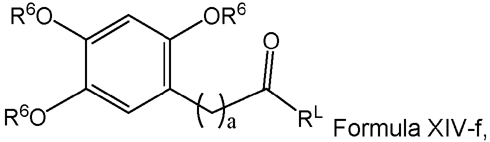 wherein in formula XIV-f, the integer a as well as the residues R⁶ are defined as for formula XI and R^{L} is defined as for formula XIV; and
- the compound of formula XIV is a compound o formula XIV-g, if a compound of formula XII is to be prepared: 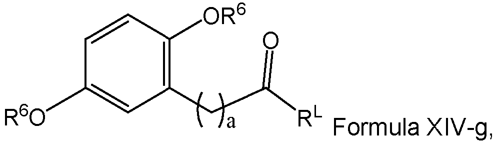 wherein in formula XIV-g, the integer a as well as the residues R⁶ are defined as for formula XII and R^{L} is defined as for formula XIV.

In some embodiments of the method comprising the aforementioned steps I) and II), step I) is: Providing a nucleoside or oligonucleotide comprising a first free backbone hydroxyl moiety, a second backbone hydroxyl or amine moiety, which is protected by a protecting group R¹, and optionally further protecting groups, which are orthogonal to R¹. In such embodiments, the term "oligonucleotide" does preferably not embrace conjugates thereof. In some embodiments of the method comprising the aforementioned steps I) and II), step I) is: Providing a nucleoside comprising a first free backbone hydroxyl moiety, a second backbone hydroxyl or amine moiety, which is protected by a protecting group R¹, and optionally further protecting groups, which are orthogonal to R¹.

In some embodiments of the method comprising the aforementioned steps I) and II), R^{L} in any one of formulae XIV, XIV-b, XIV-c, XIV-d, XIV-e, XIV-f, and XIV-g, is -OH (i.e. a hydroxyl group).

In some embodiments of the method comprising the aforementioned steps I) and II), at least steps II) is performed in an aprotic non-nucleophilic solvent. Examples of suitable solvents for this purpose may be halogenated hydrocarbon solvents such as dichloromethane,1,2-dichloroethane, chloroform, carbon tetrachloride and the like, aromatic hydrocarbon solvents such as benzene, toluene, xylene (o-, m-, or p-xylene), mesitylene, and the like, aliphatic hydrocarbon solvents such as pentane, hexane, cyclohexane, heptane, octane, and the like.

Step b) of the method comprising the aforementioned steps a) through f) may involve removing the protecting group R¹ by subjecting the compound provided in step a) to acidic conditions. Preferably, this is achieved by adding a proton donor to a solution of the compound provided in step a). The skilled person will select the proton donor depending on the properties or the protecting group R¹. Examples of suitable proton donors comprise trifluoroacetic acid, cyanopyridinium trifluoroacetate and trifluoroethanol, triethylamonium trifluoroacetate, cyanoacetic acid, acetic acid, dichloroacetic acid, trichloroacetic acid, phosphoric acid, mesylic acid, tosic acid, and hydrochloric acid. For example, 2-10% (v/v) DCA in toluene or DCM may be used. Optionally, the cleavage is carried out in the presence of a carbocation scavenger.

As used herein, the expression "carbocation scavenger" relates to a nucleophilic compound, which may be used to bind a carbocation formed, or to formally donate a hydride ion to said carbocation, thereby preventing unwanted side reactions of the carbocation. Examples of carbocation scavengers are alcohols and phenols [e.g. methanol, ethanol, phenol, cresol, water], phenol ethers [e.g. anisole, 1,3-dimethoxybenzene (dimethylanisole), 1,3,5-trimethoxybenzene]; thioethers [e.g. dimethylsulfide, methyl ethyl sulfide, thioanisole-, silanes -e.g. triisopropylsilane (TIS), triethylsilane (TES)], N-heterocycles [e.g. pyrrole, 3-methylpyrrole, 2,4-dimethylpyrrole indole, 2-methylindole, succinimide, phthalimide], thiols and thiophenols [e.g. 1,2-ethanedithiol (EDT), 1,4-dithioerythrol (DTE), 1,4-dithiothreitol (DTT), 3,6-dioxa-1,8-octanedithiol (DODT), 1,4-benzenedimethanthiol (BDMT), 1,4-butanedithiol, 2-mercaptoethanol, cysteine, thiophenol, p-thiocresol], and polyalkylbenzenes [e.g. 1,3,5-trimethylbenzene, pentamethylbenzene].

Step b) may further comprise a step of adding a base to the solution after completion of the cleavage reaction so as to neutralize the composition before adding the subsequent protected building block in step c). Preferred bases are N,N-diisopropylethylamine, pyridine, 4-cyanopyridine, trimethylamine, sodium carbonate and potassium carbonate. The skilled person will carefully limit the exposure to base in this step so as to avoid premature cleavage from the pseudo solid phase protecting group (PSPPG). Step b may involve separation of the PSPPG-protected compound from the deprotecting reagents and from any byproducts of the deprotection reaction. In later iterations of the coupling cycle, i.e. after steps c), d) and b) or c) and b) have been carried out in succession, it is also possible to remove residual reagents, scavengers and byproducts. In both cases, step b) may involve the addition of polar solvents to effect precipitation of the PSPPG-protected compound. At the same time, the process solvent may be removed, e.g. by evaporation. These operations may be used to induce precipitation of the PSPPG-protected compound. The polar solvents used for precipitation include, but are not limited to, water, acetonitrile, propionitrile, methanol, ethanol, 1-propanol, 2-propanol, preferably acetonitrile and methanol, and even more preferably acetonitrile.

A mixture of these solvents may also be used. Precipitation may be followed by solid-liquid separation, e.g. by filtration or centrifugation and dissolution in a suitable process solvent or a mixture of solvents. Therefore, in some embodiments, step b) comprises cleaving the protecting group R¹ from the compound provided in the previous step, thereby generating a free backbone hydroxyl or amine moiety; precipitating the compound thus obtained by addition of a polar solvent; separating the precipitate from the surrounding liquid; and dissolving the precipitate by addition of solvent.

The method steps c), i.e. the coupling of the building block to the growing oligonucleotide strand, and d), e.g. the oxidation or sulfurization of a phosphorous (III) moiety, depend on the coupling chemistry chosen and will be routinely carried out by the skilled artisan. For example, with preactivated moieties used for PMO synthesis, step c) may involve simply incubating the preactivated building block with the product of step b) in an inert solvent (i.e. a solvent which does not interfere in the desired coupling reaction). As a further example, in the case of H-phosphonate oligonucleotide synthesis, the coupling between the building block and the free backbone moiety may be achieved in the presence of an activating agent such as pivaloyl chloride. As a further example, in the case of phosphoramidite oligonucleotide synthesis, a weak acid such as tetrazole is needed to activate the reaction. Therefore, step c) may optionally comprise the addition of an activating agent. Step c) may further comprise the removal of unreacted building blocks, either by separation or by addition of a quenching moiety, which reacts with any residual building blocks.

As used herein, unless indicated differently, the expression "nucleoside or oligonucleotide building block" refers to mono- or oligomeric building blocks allowing to assemble an oligonucleotide strand. The skilled person will readily understand that basically any coupling chemistry compatible with the use of a base-labile pseudo solid phase protecting group may be used in the methods of the present invention. Consequently, the chemical nature of the phosphorous moiety may vary. The phosphorous moiety may be attached to the nucleoside or oligonucleotide via a backbone hydroxyl group. In some embodiments, the building block may comprise a H-phosphonate moiety attached to a backbone hydroxyl group of the nucleoside or nucleotide building block.

In other embodiments, the building block may be a phosphorous V reagent as taught in, e.g., WO 2019200273. In other embodiments, the building block may be a methylmorpholine based nucleoside or oligonucleotide according to formula B, where the backbone hydroxyl group is attached to an activated phosphor moiety.

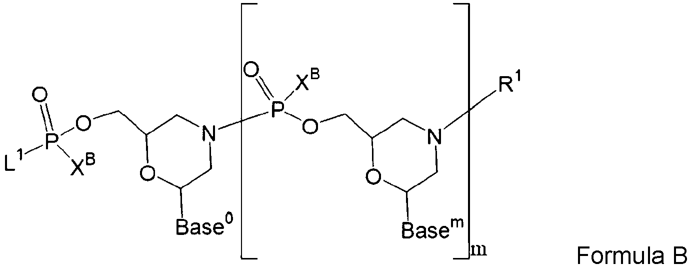

In formula B, m is an integer equal to or larger than 0; R¹ is a protecting group as described before, preferably a trityl protecting group; L¹ is a leaving group, e.g. a halogen atom (preferably a chlorine atom, CI), a methanesulfonyloxy group, or a p-toluenesulfonyloxy group; and X^{B} is a di-(C1-C6-alkyl)amino group, preferably a dimethylamino group, or a 1-piperazinyl group, wherein the 4-position nitrogen atom is protected by a protecting group and optionally further substituted. Each of the bases 0 to m may be the same or different and comprise the same or different protecting groups. Preferably, each base (i.e. Base⁰ and any Base^{m}) is a nucleobase selected from the group consisting of adenine, guanine, cytosine, thymine, and uracil, wherein the exocyclic amino group in adenine, guanine, and cytosine is protected. Compounds according to Formula B may be prepared by well-known methods as are described, e.g. in WO 9109033.

In yet other embodiments of the method comprising the aforementioned steps a) through f), the building block of step c) is be a phosphoramidite building block of the general structure given in formula C: 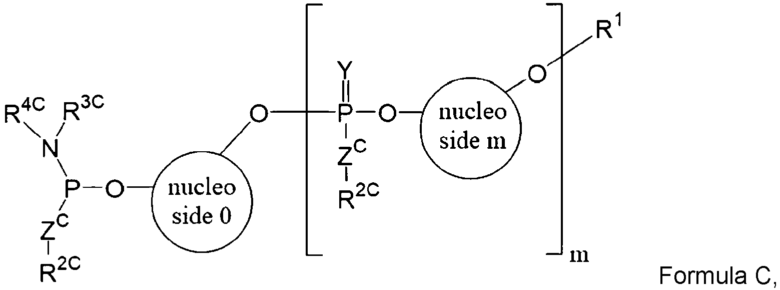 wherein:
R¹ is a protecting group as described before;
m is an integer equal to or larger than 0;
Y is selected independently for each repetitive unit m from the group consisting of S and O;
Z^{C} is selected independently for each position (i.e. for the terminal position and for each repetitive unit m) from the group consisting of O and S;
R^{2C} is a protecting group, which may be the same or different for each position (i.e. for the terminal position and each repetitive unit m);
R^{3C} and R^{4C} are protecting groups, which may be the same or different; and
each of the nucleosides 0 to m may be the same or different.

Compounds according to Formula C may be prepared according to well-known methods and are commercially available.

In some embodiments R^{3C} and R^{4C} are both C1-C5-alkyl groups, e.g. both isopropyl groups. In some embodiments, R^{2C} is at each occurrence a 2-cyanoethyl group. In some embodiments, Z^{C} is at each occurrence O. In some preferred embodiments, R^{3C} and R^{4C} are each independently of each other a C1-C5-alkyl group, preferably R^{3C} and R^{4C} are both isopropyl groups, Z^{C} is at each occurrence O, and R^{2C} is at each occurrence a 2-cyanoethyl group.

In some embodiments, different types of building blocks with different coupling chemistry may be used in different coupling cycles. For example, phosphorothioate bonded nucleosides may be introduced using P(V) chemistry, and phorphodiester bonded nucleosides may be introduced using P(III) chemistry.

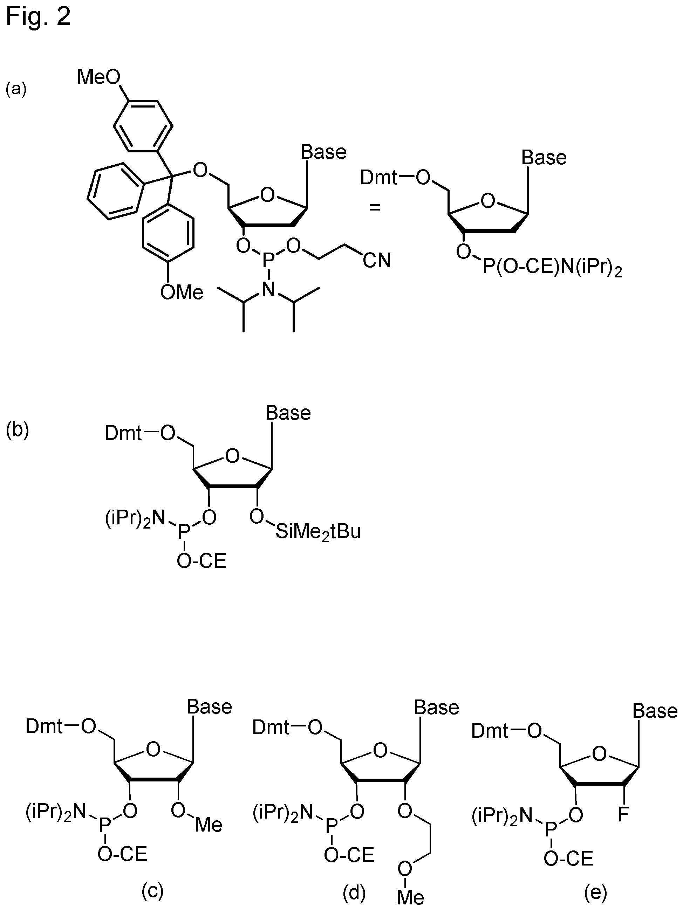

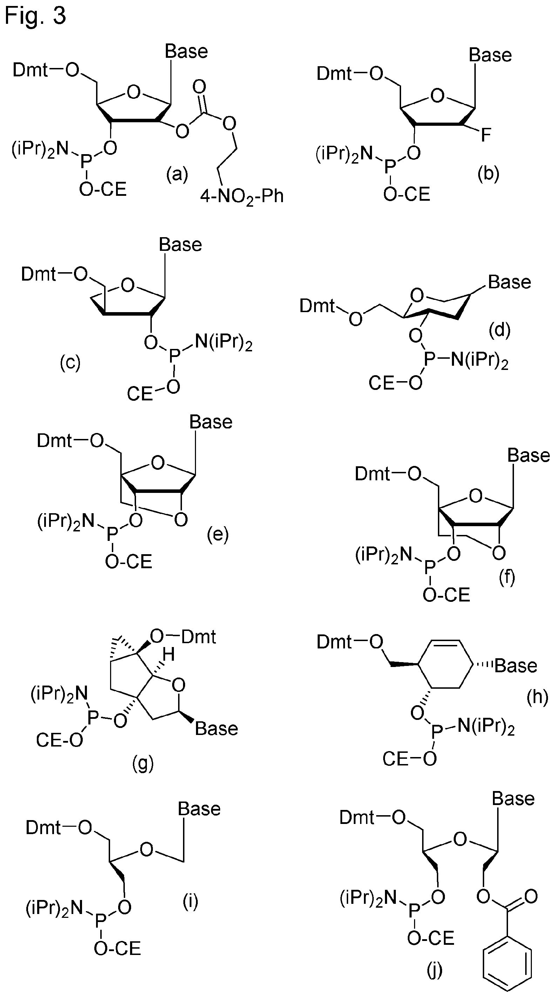

In some embodiments, m is an integer between 0 and 2 (i.e. m is 0, 1, or 2), i.e. the building blocks are mononucleoside, dinucleotide, or trinucleotide building blocks. Specific, non-limiting examples of mononucleoside phosphoamidite building blocks are given in figures 2 and 3. It should be noted that the nucleosides 0 to m and x0 to xn in formulae I, I-b, I-a, I-c, VIII to XII, and C may differ from each other with respect to, e.g., the identity and substitution pattern of the ribose or ribose analog moiety (aka. ribose surrogate), with respect to the nucleobase, and with respect to the presence and identity of any protecting groups.

A typical example for a suitable protecting group R^{2C} is the 2-cyanoethyl group; and a typical example for both R^{3C} and R^{4C} is the isopropyl group. Alternatively, R^{2C} and R^{3C} may form an ethylene bridge and the phosphoramidite may take the form of a chiral group of formula D, where ** denotes the point of attachment to the backbone hydroxyl group.

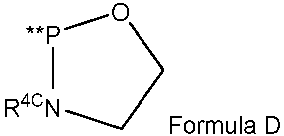

The building blocks used in the methods of the present invention may exhibit further protecting groups, e.g. to block the exocyclic amino groups of the nucleobases from side reactions.

In some embodiments, the nucleoside or oligonucleotide phosphoramidite building block of formula C is a building block of the following formula C-1: 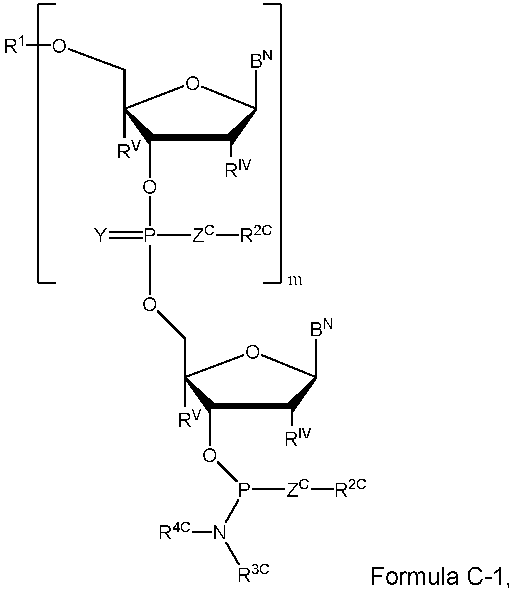 wherein in formula C-1:
m is an integer equal to or larger than 0, preferably 0, 1, or 2;
R' is a triarylmethyl type protecting group, preferably a DMT group;
B^{N} is a nucleobase which may be the same or different at each occurrence, preferably a nucleobase which is at each occurrence independently selected from the group consisting of adenine, guanine, cytosine, thymine, and uracil;
Y is selected independently for each repetitive unit m from the group consisting of S and O;
Z^{C} is selected independently for each position (i.e. for the terminal position and for each repetitive unit m) from the group consisting of O and S;
R^{2C} is a protecting group, which may be the same or different for each position (i.e. for the terminal position and each repetitive unit m);
R^{3C} and R^{4C} are protecting groups, which may be the same or different;
R^{IV} is independently at each occurrence selected from the group consisting of H, F, -O-(C1-C5 alkyl), -O-(C1-C5 alkyl)-O-(C1-C5 alkyl), -O-Si(C1-C5 alkyl)₃, and -O-CH₂-O-Si(C1-C5 alkyl)₃; and
R^{V} is independently at each occurrence H or R^{V} and R^{IV} together form a methylene or ethylene bridge of the chemical structure +-CH₂-O-++, +-CH(CH₃)-O-++, or +-CH₂-CH₂-O-++, where + is the point of attachment to the 4'-carbon atom (i.e. the carbon atom to which R^{V} is bonded) and ++ is the point of attachment to the 2'-carbon atom (i.e. the carbon atom to which R^{IV} is bonded).

In some embodiments, in the nucleoside or oligonucleotide phosphoramidite building block of formula C-1:
m is an integer equal to or larger than 0, preferably 0, 1, or 2;
R' is a triarylmethyl type protecting group, preferably a DMT group;
B^{N} is a nucleobase which may be the same or different at each occurrence, preferably a nucleobase which is at each occurrence independently selected from the group consisting of adenine, guanine, cytosine, thymine, and uracil;
Y is selected independently for each repetitive unit m from the group consisting of S and O;
Z^{C} is at each occurrence O;
R^{2C} is at each occurrence a 2-cyanoethyl group;
R^{3C} and R^{4C} are each independently of each other a C1-C5-alkyl group, preferably R^{3C} and R^{4C} are both isopropyl groups;
R^{IV} is independently at each occurrence selected from the group consisting of H, F, -O-CH₃ (i.e. methoxy), -O-CH₂-CH₂-O-CH₃ (i.e. 2-methoxyethyl-1-oxy), -O-Si(CH₃)₃ (i.e. trimethylsilyloxy, TMS), -O-Si(CH₃)₂(C(CH₃)₃) (i.e. *tert-*butyl(dimethyl)silyloxy, TBDMS or TBS), and -O-CH₂-O-Si(C(CH₃)₃)₃ (i.e. ((triisopropylsilyl)oxy)-methyloxy, TOM); and
R^{V} is H at each occurrence.

In some embodiments, in the nucleoside or oligonucleotide phosphoramidite building block of formula C-1:
m is 0;
R' is a DMT group;
B^{N} is a nucleobase which may be the same or different at each occurrence, preferably a nucleobase which is at each occurrence independently selected from the group consisting of adenine, guanine, cytosine, thymine, and uracil;
Y is selected independently for each repetitive unit m from the group consisting of S and O;
Z^{C} is at each occurrence O;
R^{2C} is at each occurrence a 2-cyanoethyl group;
R^{3C} and R^{4C} are both isopropyl groups;
R^{IV} is independently at each occurrence selected from the group consisting of H, -O-CH₃ (i.e. methoxy), and -O-Si(CH₃)₂(C(CH₃)₃) (i.e. tert-butyl(dimethyl)silyloxy, TBDMS or TBS); and
R^{V} is H at each occurrence.

In some embodiments, in the nucleoside or oligonucleotide phosphoramidite building block of formula C-1:
m is 0;
R¹ is a DMT group;
B^{N} is at each occurrence independently selected from the group consisting of adenine, guanine, cytosine, thymine, and uracil;
Y is selected independently for each repetitive unit m from the group consisting of S and O;
Z^{C} is at each occurrence O;
R^{2C} is at each occurrence a 2-cyanoethyl group;
R^{3C} and R^{4C} are both isopropyl groups;
R^{IV} is independently at each occurrence selected from the group consisting of H, -O-CH₃ (i.e. methoxy), and -O-Si(CH₃)₂(C(CH₃)₃) (i.e. tert-butyl(dimethyl)silyloxy, TBDMS or TBS); and
R^{V} is H at each occurrence.

As laid out above, it will be understood that the nucleobases adenine, guanine, cytosine, thymine, and uracil may optionally be protected (i.e. carry one or more protecting groups). In particular, the exocyclic amino group in adenine, guanine, and cytosine may be protected. Examples of suitable protecting groups are provided in Table 1 (vide infra)

In some embodiments, in the nucleoside or oligonucleotide phosphoramidite building block of formula C-1:
m is 0;
R¹ is a DMT group;
B^{N} is at each occurrence independently selected from the group consisting of adenine, in which the exocyclic amino group is benzoyl-protected, guanine, in which the exocyclic amino group is isobutyryl-protected, cytosine, in which the exocyclic amino group is benzoyl- or acetyl-protected, thymine, and uracil;
Y is selected independently for each repetitive unit m from the group consisting of S and O;
Z^{C} is at each occurrence O;
R^{2C} is at each occurrence a 2-cyanoethyl group;
R^{3C} and R^{4C} are both isopropyl groups;
R^{IV} is independently at each occurrence selected from the group consisting of H, -O-CH₃ (i.e. methoxy), and -O-Si(CH₃)₂(C(CH₃)₃) (i.e. *tert*-butyl(dimethyl)silyloxy, TBDMS or TBS); and
R^{V} is H at each occurrence.

Suitable nucleoside derivatives are commercially available. Table 1 gives a non-limiting overview of commonly used protecting groups, e.g. for nucleoside building blocks.

**Table 1: non-limiting overview of commonly used protecting groups, e.g. for nucleoside building blocks**

| Protecting group | Often used for |
|---|---|
| Bz = benzoyl; iBu = isobutyryl; | exocyclic amino group of adenine |
| PAC = phenoxyacetyl | |
| Ac = acetyl or Bz = benzoyl | exocyclic amino group of cytosine |
| iBu = isobutyryl; | exocyclic amino group of guanine |
| i-Pr-PAC = 4-isopropylphenoxyacetyl; | |
| dimethylformamidino | |
| TBDMS = t-butyldimethylsilyl; | protection of 2' OH-group |
| TOM = tri(isopropyl)silyloxymethyl | |
| TMS = trimethylsilyl | |

In some embodiments of the method of the present invention, the compound provided in step a) comprises a backbone hydroxyl moiety, which is protected by a protecting group R¹, and the building block used in step c) comprises a backbone hydroxyl moiety, which is protected by a protecting group R'. Further, the building block used in step c) may be a phosphoramidite building block.

Step d) of the method may involve the oxidation of the P(III) moiety to a P(V) moiety. Oxidation, as used herein, is a chemical reaction which increases the oxidation number of a moiety subjected to said reaction. Oxidation may therefore comprise the introduction of a =O moiety or of a =S moiety. The oxidation of a H-phosphonate moiety or of a phosphite triester moiety may therefore result in the formation of any internucleosidic linkage according to formula A-1 or A-2 above, e.g. in the formation of a phosphodiester, a phosphorothioate, a phosphoroamidate, a phosphotriester (aka. phosphate triester), or a thiophosphate triester bond. The corresponding methods and reagents are well known and readily available to the skilled person. For example, phosphotriester bonds may be obtained by reacting a phosphite triester moiety with aqueous iodine in the presence of a weak base such as pyridine, collidine, or lutidine. Sulfurization may be achieved, e.g. using a reagent such as 3-(dimethylaminomethylidene)amino-3H-1,2,4-dithiazole-3-thione (DDTT), 3H-1,2-benzodithiol-3-one 1,1-dioxide (Beaucage reagent), or N,N,N'N'-Tetraethylthiuram disulfide (TETD).

In some embodiments of the method of the invention comprising the aforementioned steps a) through f), the phosphorus moiety of the nucleoside or oligonucleotide building block used in step c) is a phosphorus (III) moiety; the method comprises a step d) of oxidizing or sulfurizing the phosphorus (III) atom to a phosphorus (V) atom, thereby creating the desired type of internucleoside linkage; and step e) comprises optionally either reiterating the sequence of steps b) through d) or reiterating the sequence of steps b) and c) before executing step d). Herein, the terms "internucleoside linkage" and "internucleosidic linkage" may be used interchangeably.

In some embodiments of the method of the invention comprising the aforementioned steps a) through f), step c) comprises reacting the free backbone hydroxyl group resulting from step b) with a phorsphoramidite nucleoside or oligonucleotide building block so as to form a phosphite triester bond, and step d) involves oxidizing or sulfurizing the phosphite triester bond. It will be understood that in both cases (i.e. oxidation or sulfutization), the P(III) atom of said phosphite triester bond will be converted to a P(V) atom, wherein sulfurization is accompanied by the introduction of a sulfur atom into the respective internucleoside linkage group, while oxidation is accompanied by introduction of an oxygen atom into the respective internucleosidic linkage group.

In order to avoid the formation of difficult-to-remove deletion sequences, the methods of the invention may comprise a further step of blocking (also referred to as "capping") unreacted free hydroxyl or amine groups after step c) or step d). The hydroxyl or amine groups may typically be blocked (i.e. capped) by acetylation. The means of acetylation hydroxyl or amine groups during oligonucleotide synthesis are known to those skilled in the art. For example, acetic anhydride, optionally in the presence of a base such as TEA, DIPEA, pyridine, lutidine or collidine, may be used.

It should be noted that the order of steps indicated in the claims and the description of the present methods is not necessarily binding, unless indicated differently. In some embodiments of the method comprising the aforementioned steps a) through f) these steps are executed in order from a) through f). This is to say that in these embodiments, steps a), b), c), d), e), and f) are performed in this order. This does however not exclude performing further steps in between any of steps a) trough f). Moreover, the present methods may comprise further steps. For example, the assembled olignonucleotide moiety may be conjugated to another moiety before executing cleavage step f) of the present methods. Further, the skilled artisan may use intervening separation steps, in particular precipitation and washing steps, e.g. when the composition of solvent changes between process steps, or in order to remove traces of reagents from a previous method step. Additionally, an isolated iteration of step b) may be performed after step e) or after step f) to remove the terminal (e.g. 5'-terminal) R¹-protecting group introduced via the nucleoside or oligonucleotide building block used in the final iteration of step c).

For example, the pseudo-solid phase protecting group with the growing oligonucleotide strand attached to it may be precipitated by increasing the polarity of the solvent after each iteration of step d). This may be achieved by adding a polar solvent or a mixture of polar solvents. Examples of polar solvents comprise water, nitrile solvents, e.g. acetonitrile, and propionitrile, and alcohols such as methanol, ethanol, 1-propanol, and 2-propanol.

Step f) of cleaving the oligonucleotide from the pseudo solid phase protecting group may involve incubation with a base such as ammonia or a source of hydroxide ions. In some cases, sodium hydroxide in aqueous alcoholic solvents or in cyclic ethers (e.g. tetrahydrofuran, 2-methyl-tetrahydrofuran) may be used. In some embodiments of the method comprising the aforementioned steps a) through f), step f) comprises incubation with a base, wherein said base is preferably selected from the group consisting of ammonia, a C1-C6-alkyl amine (i.e. a primary alkyl amine comprising between one and 6 carbon atoms, e.g. methylamine or tert-butylamine), and a source of hydroxide ions. In this context, the term "incubation" means incubating the conjugate of the pseudo solid phase protecting group and the oligonucleotide with said base, preferably in a solution comprising said base. Thus, in some embodiments of the method comprising the aforementioned steps a) through f), step f) comprises incubating the conjugate of the pseudo solid phase protecting group and the oligonucleotide in a solution comprising a base, wherein said base is preferably selected from the group consisting of ammonia, a C1-C6-alkyl amine (in particular methylamine or tert-butylamine), and a source of hydroxide ions. In some embodiments of the method comprising the aforementioned steps a) through f), step f) comprises incubating the conjugate of the pseudo solid phase protecting group and the oligonucleotide in a solution, preferably an aqueous solution, comprising a source of hydroxide ions. In some embodiments of the method comprising the aforementioned steps a) through f), step f) comprises incubating the conjugate of the pseudo solid phase protecting group and the oligonucleotide in a solution, preferably an aqueous solution, comprising ammonia. As used herein, the term "a source of hydroxide ions" refers to a salt which comprises hydroxide ions, wherein sodium hydroxide, potassium hydroxide, and ammonium hydroxide are preferred examples and sodium hydroxide is particularly preferred. A person skilled in the art knows that an aqueous solution of ammonia may also be referred to as an ammonium hydroxide solution since it comprises ammonium ions and hydroxide ions. The skilled artisan understands that a solution comprising a source of hydroxide anions may comprise said source of hydroxide ions in solvated form. The term "aqueous solution" refers to a solution comprising water and optionally one or more water-miscible organic solvents such as acetonitrile, tetrahydrofuran, ethanol, and the like.

In some embodiments of the method comprising the aforementioned steps a) through f), wherein the pseudo solid phase protecting group bound to said nucleoside, nucleotide, oligonucleotide, or conjugate of a nucleoside, nucleotide or oligonucleotide provided in step a) is a pseudo solid phase protecting group according to any one of formulae II, II-1, II-2, II-a, II-a-1, II-a-2, and III to VII, wherein c is 1, step f) comprises incubating the conjugate of the pseudo solid phase protecting group and the oligonucleotide in a solution, preferably an aqueous solution, comprising a source of hydroxide ions and/or a C1-C6-alkyl amine (in particular methylamine or tert-butylamine), preferably a source of hydroxide ions. In some embodiments of the method comprising the aforementioned steps a) through f), wherein the pseudo solid phase protecting group bound to said nucleoside, nucleotide, oligonucleotide, or conjugate of a nucleoside, nucleotide or oligonucleotide provided in step a) is a pseudo solid phase protecting group according to any one of formulae II, II-1, II-2, II-a, II-a-1, II-a-2, and III to VII, wherein c is 1, step f) comprises incubating the conjugate of the pseudo solid phase protecting group and the oligonucleotide in an aqueous solution of sodium hydroxide, potassium hydroxide or ammonium hydroxide, preferably sodium hydroxide. In some embodiments of the method comprising the aforementioned steps a) through f), wherein the pseudo solid phase protecting group bound to said nucleoside, nucleotide, oligonucleotide, or conjugate of a nucleoside, nucleotide or oligonucleotide provided in step a) is a pseudo solid phase protecting group according to any one of formulae II, II-1, II-2, II-a, II-a-1, II-a-2, and III to VII, wherein c is 1, step f) comprises incubating the conjugate of the pseudo solid phase protecting group and the oligonucleotide in an aqueous solution of a C1-C6-alkyl amine, preferably tert-butylamine. For example, a 1:1 (v/v) mixture of water and tert-butylamine may be used.

In some embodiments of the method comprising the aforementioned steps a) through f), wherein the pseudo solid phase protecting group bound to said nucleoside, nucleotide, oligonucleotide, or conjugate of a nucleoside, nucleotide or oligonucleotide provided in step a) is a pseudo solid phase protecting group according to any one of formulae II, 11-1, II-2, II-a, II-a-1, II-a-2, and III to VII, wherein c is 0, step f) comprises incubating the conjugate of the pseudo solid phase protecting group and the oligonucleotide in a solution, preferably an aqueous solution, comprising ammonia and/or a C1-C6-alkyl amine (e.g. methylamine or tert-butylamine), preferably ammonia. In some embodiments of the method comprising the aforementioned steps a) through f), wherein the pseudo solid phase protecting group bound to said nucleoside, nucleotide, oligonucleotide, or conjugate of a nucleoside, nucleotide or oligonucleotide provided in step a) is a pseudo solid phase protecting group according to any one of formulae II, 11-1, 11-2, II-a, II-a-1, II-a-2, and III to VII, wherein c is 0, step f) comprises incubating the conjugate of the pseudo solid phase protecting group and the oligonucleotide in an aqueous ammonia solution. Such an aqueous ammonia solution may for example comprise 28% ammonia (w/w) in water, wherein w/w indicates that 28% refers to the weight-% of NH₃ in water.

The methods of the invention are not particularly limited with respect to the temperature, at which they are carried out, and may normally take place at ambient temperature, e.g. at a temperature of between 15 and 35°C. However, it is to be understood that the reactions may be carried out at any suitable temperature, e.g. at a temperature of between 0°C and 90°C.

Some embodiments of the invention relate to a method for the synthesis of oligonucleotides, comprising steps a) through f):
a) providing a nucleoside or oligonucleotide of the following formula S-1: 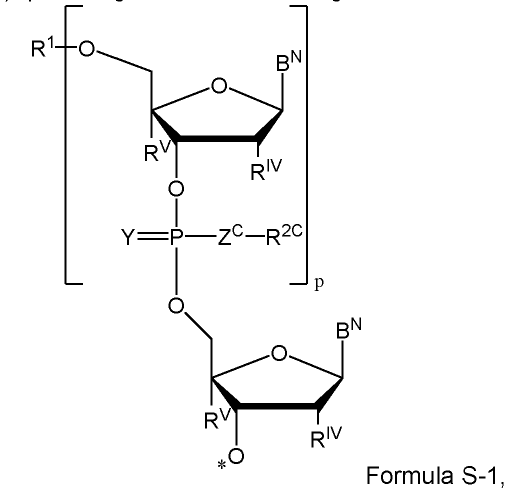 wherein in Formula S-1:
   * denotes the point of attachment of a pseudo solid phase protecting group according to any one of formulae II, 11-1, 11-2, II-a, II-a-1, II-a-2, and III to VII;
   p is an integer equal to or larger than 0; and
   R¹, B^{N}, Y, Z^{C}, R^{2C}, R^{IV}, and R^{V} are defined as for formula C-1;
b) cleaving the protecting group R¹ from the compound provided in the previous step, thereby generating a free backbone hydroxyl or amine group;
c) reacting the free backbone hydroxyl or amine group generated in step b) with a phosphorus moiety of a nucleoside or oligonucleotide building block of formula C-1: 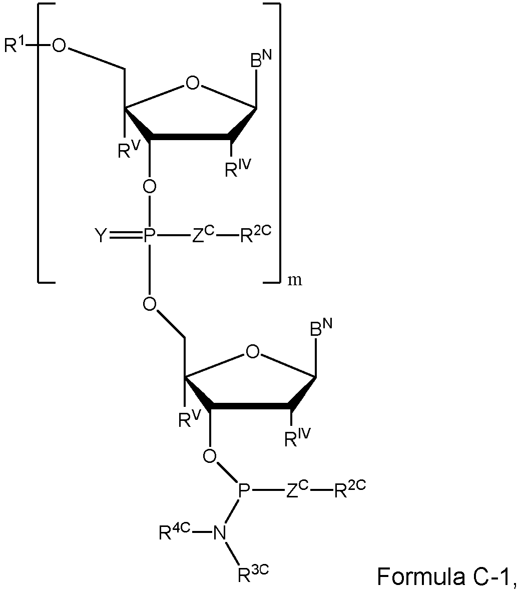 wherein in formula C-1:
   m, R¹, B^{N}, Y, Z^{C}, R^{2C}, R^{3C}, R^{4C}, R^{IV}, and R^{V} are defined as stated above;
d) optionally modifying the phosphorus moiety;
e) optionally reiterating the sequence of steps b) to c) or b) to d); and
f) cleaving the oligonucleotide from the pseudo solid phase protecting group.

In some embodiments, the integer p of formula S-1 is an integer in the range of 0-200, 0-150, 0-120, 0-100, 0-90, 0-80, 0-70, 0-60, 0-50, 0-40, 0-30, 0-20, 0-10, or 0-1.

In some embodiments, the method comprising the aforementioned steps a) through f), further comprises the following step g):
g) isolating the support-cleaved oligonucleotide.

In this context, the term "support-cleaved oligonucleotide" refers to the oligonucleotide cleaved from the pseudo solid phase protecting group, preferably as obtained in step f).

The means of isolating oligonucleotides upon chemical oligonucleotide synthesis form part of the common knowledge of those skilled in the art. Typically, such a step of isolating an oligonucleotide comprises one or more purification steps and one or more steps aiming at obtaining the oligonucleotide in solid form. For oligonucleotide purification, chromatographic methods may typically be used, in particular ion exchange (especially anion exchange) chromatography and reversed phase (RP) HPLC, e.g. in form of hydrophobic interaction HPLC. These techniques are known to those skilled in the art. Additionally, a step of isolating an oligonucleotide may comprise ultrafiltration and/or desalting steps. For example, a solution obtained after cleaving oligonucleotides from the pseudo solid phase protecting group (PSPPG) may be submitted to ultrafiltration and/or desalting, ion exchange chromatography, and another round of ultrafiltration and/or desalting. Alternatively, the PSPPG-cleaved oligonucleotides may be submitted to reversed phase (RP) HPLC, e.g. in form of hydrophobic interaction HPLC. The latter method may preferably be performed, if the oligonucleotides still carry the 5'-terminal hydroxyl protecting group, e.g. the DMT group. Said 5'-protecting group may even be removed on the RP-HPLC column by passing an acidic solution through the column. If the 5'-terminal protecting group has been removed prior to purification, e.g. prior to cleaving the oligonucleotide from PSPPG, ion exchange chromatography may be preferred. Purification is typically followed by one or more steps aiming at obtaining the oligonucleotide in solid form. Lyophilization and spray drying may for example be used. In some cases, it may be desirable to obtain the oligonucleotide in form of a salt with certain counter ions. In such cases salt exchange may be performed, typically prior to lyophilization or spray drying.

The following Figures, Claims and Examples, including the experiments conducted and the results achieved, are provided for illustrative purposes only and are not to be construed as limiting the scope of the claims.

### Examples

### Analytical Methods

The oligonucleotides synthesized were analyzed by high performance liquid chromatography (HPLC) coupled to MS-spectrometry using the following parameters.
Method A: Solid phase: YMC-Triart C18, 12 nm, 1.9 µm, 2.1x100 mm; Mobile phase A: H₂O:HFIP:TEA (1000:1.5:0.2); Mobile phase B:THF:HFIP:TEA (1000:1.5:0.2), Flow rate: 0.48 mL/min; Gradient (mobile phase B %): 0-0.5 min; 65%, 0.5-1.0min; 65 to 72 %, 1.0-3.0 min; 72%, 3.0-5.0 min; 72 to 80%, 5.0-6.0 min; 80 to 85%, 6-6.5 min; 80 to 90%
Column temperature: 40°C; Detection wavelength: 254 nm.
Method B: Solid phase: Column YMC-Triart C18, 12 nm, 1.9 µm, 2.1x100 mm; Mobile Phase A: H₂O:TFA(1000:1); Moblie Phase B: MeCN:TFA(1000:1); Flow rate 0.9 mL/min; Gradient (mobile phase B %): 0-1 min; 1%, 1-15 min; 50%, 15.01-18 min; 90%; Column temperature: 60°C; Detection wavelength: 263 nm.
Method C: Solid phase: YMC-Triart C18, 12 nm, 1.9 µm, 2.1x100 mm; Mobile Phase A: H₂O:TFA(1000:1); Mobile Phase B: MeCN:TFA(1000:1); Flow rate 0.3 mL/min; Gradient (mobile phase B %): 0-1 min; 1%, 1-15 min; 50%, 15.01-18 min; 90%; Column temperature: 60°C; Detection wavelength: 263 nm.
MS method: Ion source: Dual ESI, Ionization mode: positive or negative, Vcap: 3500 V, Gas temp: 325°C, Gas Flow: 8 L/min, Nebulizer: 50 psig.

### Example 1: Synthesis of DMTr-dA(Bz)-Kc (1).

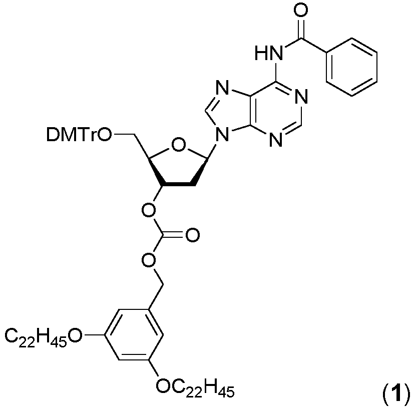

To a solution of DMTr-dA (0.30 mmol, 197 mg) and 1,1'-carbonyldiimidazole (0.60 mmol, 97.3 mg) in CH₂Cl₂ (10 mL) stirred at room temperature (rt, i.e. approximately 20 °C) was added 4-dimethylaminopyridine (0.60 mmol, 73.3 mg). The resulting reaction mixture was stirred at rt for 14 h, diluted with NH₄Cl aq and extracted with CH₂Cl₂. The combined organic layers were dried over Na₂SO₄, filtered and concentrated in vacuo to give 205 mg (91%) DMTr-dA-1H-imidazole-1-carboxyl. To a solution of DMTr-dA-1H-imidazole-1-carboxyl (ca. 0.30 mmol) and DBU (0.23 mmol, 34.3 µL) in CH₂Cl₂ (15 mL) stirred at rt was added 3,5-bis(docosyloxy)benzenemethanol, C₅₁H₉₆O₃ (0.15 mmol, 114 mg). The resulting reaction mixture was stirred at rt for 16 h and concentrated in vacuo. The precipitate was filtered and washed with MeOH to give DMTr-dA-Kc in 99% yield (215 mg) as white solid. Throughout this text, the terms "DMT" and "DMTr" are used interchangeably to refer to the di(p-methoxyphenyl)phenylmethyl group.

1H NMR (CDCl₃, 500 MHz) δ 9.01 (1H, d), 8.71 (1H, s), 8.17 (1H, s), 8.02 (2H, d), 7.60 (1H, t), 7.52 (2H, t), 7.39 (2H, d), 7.28 (4H, d), 7.25 (2H, t), 7.20 (1H ,t), 6.79 (4H, d), 6.51 (3H, dt), 6.44 (1H, s), 5.48 (1H, d), 5.11 (2H, s), 4.40 (1H, s), 3.93 (4H, t), 3.77 (6H, s), 3.46 (2H, m), 3.10 (1H, m), 2.75 (1H, dd), 1.77 (4H, quint), 1.44 (4H, m), 1.39-1.19 (72H, m), 0.88 (6H, t); HPLC (method A) : Rt=4.30 min; MS (ESI⁻): m/z calcd for [C₉₀H₁₂₇N₅O₁₀ - H]⁻: 1439.0, found: 1439.0.

### Example 2: Synthesis of DMTr-dT-Kc (2).

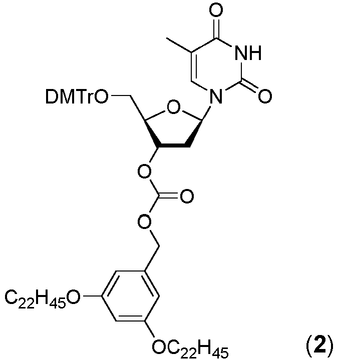

To a solution of DMTr-dT (0.30 mmol, 163 mg) and 1,1'-carbonyldiimidazole (0.60 mmol, 97.3 mg) in CH₂Cl₂ (10 mL) stirred at rt was added 4-dimethylaminopyridine (0.60 mmol, 73.3 mg). The resulting reaction mixture was stirred at rt for 14 h, diluted with NH₄Cl aq and extracted with CH₂Cl₂. The combined organic layers were dried over Na₂SO₄, filtered and concentrated in vacuo to give DMTr-dT-1H-imidazole-1-carboxyl quantitatively (197 mg). To a solution of DMTr-dT-1H-imidazole-1-carboxyl (ca. 0.30 mmol) and DBU (0.23 mmol, 34.3 µL) in CH₂Cl₂ (15 mL) stirred at rt was added 3,5-bis(docosyloxy)benzenemethanol, C₅₁H₉₆O₃ (0.15 mmol, 114 mg). The resulting reaction mixture was stirred at rt for 16 h and concentrated in vacuo. The precipitate was filtered and washed with MeOH to give DMTr-dT-Kc quantitatively (203 mg) as a white solid.

1H NMR (CDCl₃, 500 MHz) δ 7.96 (1H, s), 7.60 (1H, d), 7.38 (2H, dd), 7.30 (2H, m), 7.27 (2H, m), 7.26 (1H, m), 7.25 (2H, m), 6.84 (4H, d), 6.48 (2H, d), 6.44 (1H, dd), 6.42 (1H, t), 5.36 (1H, d), 5.06 (2H, s), 4.22 (1H, d), 3.92 (4H, t), 3.79 (6H, s), 3.51 (1H, dd), 3.44 (1H, dd), 2.56 (1H, dd), 2.44 (1H, m), 1.75 (4H, quint), 1.43 (4H, quint), 1.37 (3H, s), 1.36-1.21 (72H, m), 0.88 (6H, t); HPLC (Method A): Rt=4.39 min; MS (ESI⁻): m/z calcd for [C₈₃H₁₂₆N₂O₁₁ - H]⁻: 1325.9, found: 1325.9.

### Example 3: Synthesis of DMTr-dG(Bu)-Kc (3).

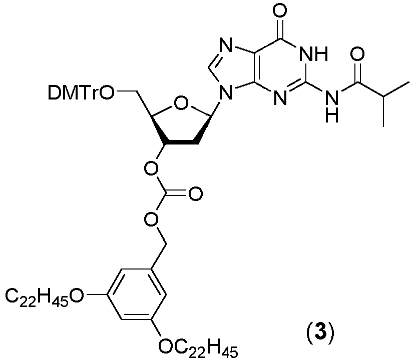

To a solution of DMTr-dG(iBu) (0.30 mmol, 192 mg) and 1,1'-carbonyldiimidazole (0.60 mmol, 97.3 mg) in CH₂Cl₂ (10 mL) stirred at rt was added 4-dimethylaminopyridine (0.60 mmol, 73.3 mg). The resulting reaction mixture was stirred at rt for 14 h, diluted with NH₄Cl aq and extracted with CH₂Cl₂. The combined organic layers were dried over Na₂SO₄, filtered and concentrated in vacuo to give DMTr-dG(iBu)-1H-imidazole-1-carboxyl quantitatively (225 mg). To a solution of DMTr-dG(iBu)-1H-imidazole-1-carboxyl (ca. 0.30 mmol) and DBU (0.23 mmol, 34.3 µL) in CH₂Cl₂ (15 mL) stirred at rt was added 3,5-bis(docosyloxy)benzenemethanol, C₅₁H₉₆O₃ (0.15 mmol, 114 mg). The resulting reaction mixture was stirred at rt for 16 h and concentrated in vacuo. The precipitate was filtered and washed with MeOH to give DMTr-dG(iBu)-Kc quantitatively (225 mg) as white solid.

1H NMR (CDCl₃, 500 MHz) δ 11.9 (1H, s), 7.76 (1H, s), 7.58 (1H, s), 7.49 (2H, d), 7.35 (4H, d), 7.25 (2H, t), 7.21 (1H, d), 6.79 (4H, dd), 6.48 (2H, d), 6.42 (1H, t), 6.14 (1H, dd), 5.51 (1H, d), 5.07 (2H, m), 4.26 (1H, q), 3.91 (4H, t), 3.77 (3H, s), 3.76 (3H, s), 3.47 (1H, dd), 3.26 (1H, m), 3.24 (1H, dd), 2.56 (1H, dd), 1.75 (5H, m), 1.42 (5H, m), 1.37-1.21 (71H, m), 0.97 (3H, d), 0.88 (6H, t), 0.81 (3H, d); HPLC (method A): Rt=3.76 min; MS (ESI⁻): m/z calcd for [C₈₇H₁₃₁N₅O₁₁ - H]⁻: 1421.0, found: 1421.0.

### Example 4: Synthesis of DMTr-dC(Bz)-Kc (4).

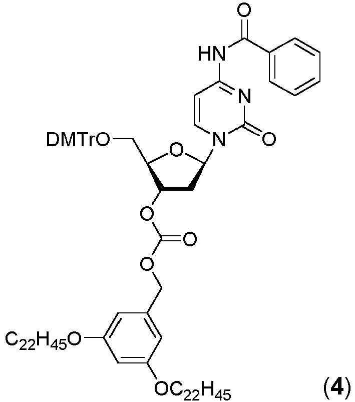

To a solution of DMTr-dC(Bz) (0.30 mmol, 192 mg) and 1,1'-carbonyldiimidazole (0.60 mmol, 97.3 mg) in CH₂Cl₂ (10 mL) stirred at rt was added 4-dimethylaminopyridine (0.60 mmol, 73.3 mg). The resulting reaction mixture was stirred at rt for 14 h, diluted with NH₄Cl aq and extracted with CH₂Cl₂. The combined organic layers were dried over Na₂SO₄, filtered and concentrated in vacuo to give DMTr-dC(Bz)-1H-imidazole-1-carboxyl quantitatively (227 mg). To a solution of DMTr-dC(Bz)-1H-imidazole-1-carboxyl (ca. 0.30 mmol) and DBU (0.23 mmol, 34.3 µL) in CH₂Cl₂ (15 mL) stirred at rt was added 3,5-bis(docosyloxy)benzenemethanol, C₅₁H₉₆O₃ (0.15 mmol, 114 mg). The resulting reaction mixture was stirred at rt for 16 h and concentrated in vacuo. The precipitate was filtered and washed with MeOH to give DMTr-dC(Bz)-Kc quantitatively (216 mg) as white solid.

1H NMR (CDCl₃, 500 MHz) δ 8.66 (1H, s), 8.15 (1H, d), 7.88 (2H, d), 7.61 (1H, t), 7.51 (2H, t), 7.37 (2H, d), 7.28 (6H, m), 7.24 (1H, t), 6.86 (4H, dd), 6.50 (2H, d), 6.43 (1H, s), 6.31 (1H, t), 5.33 (1H, t), 5.09 (2H, s), 4.36 (1H, d), 3.93 (4H, t), 3.79 (3H, s), 3.78 (3H, s), 3.49 (2H, s), 2.93 (1H, dd), 2.38 (1H, quint), 1.76 (4H, quint), 1.44 (4H, quint), 1.39-1.19 (73H, m), 0.88 (6H, t); HPLC (method A): Rt=4.38 min; MS (ESI⁻): m/z calcd for [C₈₉H₁₂₉N₃O₁₁ - H]⁻: 1415.0, found: 1415.0.

### Example 5: Synthesis of DMTr-dT-Ka (5).

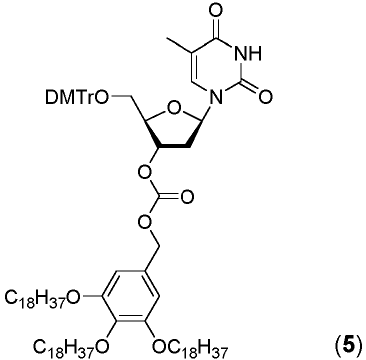

To a solution of DMTr-dT (0.30 mmol, 163 mg) and 1,1'-carbonyldiimidazole (0.60 mmol, 97.3 mg) in CH₂Cl₂ (10 mL) stirred at rt was added 4-dimethylaminopyridine (0.60 mmol, 73.3 mg). The resulting reaction mixture was stirred at rt for 14 h, diluted with NH₄Cl aq and extracted with CH₂Cl₂. The combined organic layers were dried over Na₂SO₄, filtered, and concentrated in vacuo to give DMTr-dT-1H-imidazole-1-carboxyl quantitatively (197 mg). To a solution of DMTr-dT-1H-imidazole-1-carboxyl (ca. 0.30 mmol) and DBU (0.23 mmol, 34.3 µL) in CH₂Cl₂ (15 mL) stirred at rt was added 3,4,5-tris(octadecyloxy)benzenemethanol, C₆₁H₁₁₆O₄ (0.15 mmol, 137 mg). The resulting reaction mixture was stirred at rt for 16 h and concentrated in vacuo. The precipitate was filtered and washed with MeOH to give DMTr-dT-Ka quantitatively (240 mg) as white solid.

1H NMR (CDCl₃, 500 MHz) δ 8.75 (1H, s), 7.61 (1H, s), 7.38 (2H, d), 7.27 (7H, m), 6.84 (4H, d), 6.56 (2H, s), 6.46 (1H, dd), 5.37 (1H, d), 5.05 (2H, s), 4.23 (1H, m), 3.96 (3H, t), 3.93 (2H, t), 3.79 (6H, s), 3.52 (1H, dd), 3.45 (1H, dd), 2.57 (1H, dd), 2.45 (1H, m), 1.79 (3H, quint), 1.73 (2H, quint), 1.46 (6H, m), 1.37 (3H, s), 1.35-1.17 (86H, m), 0.88 (9H, t); HPLC (method A): Rt=4.92 min; MS (ESI⁻): m/z calcd for [C₉₃H₁₄₆N₂O₁₂ - H]⁻: 1482.1, found: 1482.1.

### Example 6: Synthesis of DMTr-rU(TBS)-Kc (6).

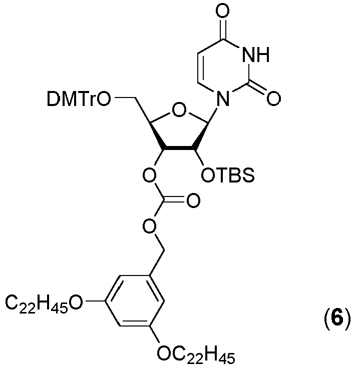

To a solution of DMTr-rU (0.30 mmol, 198 mg) and 1,1'-carbonyldiimidazole (0.60 mmol, 97.3 mg) in CH₂Cl₂ (10 mL) stirred at rt was added 4-dimethylaminopyridine (0.60 mmol, 73.3 mg). The resulting reaction mixture was stirred at rt for 14 h, diluted with NH₄Cl aq and extracted with CH₂Cl₂. The combined organic layers were dried over Na₂SO₄, filtered and concentrated in vacuo to give DMTr-rU-1H-imidazole-1-carboxyl quantitatively (238 mg). To a solution of DMTr-rU-1H-imidazole-1-carboxyl (ca. 0.30 mmol) and DBU (0.23 mmol, 34.3 µL) in CH₂Cl₂ (15 mL) stirred at rt was added 3,5-bis(docosyloxy)benzenemethanol, C₅₁H₉₆O₃ (0.15 mmol, 114 mg). The resulting reaction mixture was stirred at rt for 16 h and concentrated in vacuo. The precipitate was filtered and washed with MeOH to give DMTr-rU-Kc in 94% yield (204 mg) as white solid.

1H NMR (CDCl₃, 500 MHz) δ 8.74 (1H, d), 7.92 (1H, d), 7.36 (2H, dd), 7.29 (2H, t), 7.25 (6H, m), 6.84 (4H, dd), 6.49 (2H, d), 6.42 (1H, t), 5.95 (1H, d), 5.30 (1H, dd), 5.15 (1H, t), 5.10 (1H, d), 5.04 (1H, d), 4.54 (1H, t), 4.31 (1H, m), 3.91 (4H, t), 3.79 (3H, s), 3.78 (3H, s), 3.78 (1H, m), 3.59 (1H, dd), 3.45 (1H, dd), 1.75 (4H, quint), 1.43 (4H, quint), 1.38-1.12 (70H, m), 0.88 (6H, t), 0.86 (9H, s), 0.09 (3H, s), 0.07 (3H, s); HPLC (method A): Rt=4.83 min; MS (ESI⁻): m/z calcd for [C₈₈H₁₃₈N₂O₁₂Si - H]⁻: 1442.0, found: 1442.0.

### Example 7: Synthesis of dT-Kc (7).

To a solution of DMTr-dT-Kc **2** (0.15 mmol) in CH₂Cl₂ (20 mL) stirred at rt was added dichloroacetic acid (7.46 mmol, 616.2 µl). The resulting reaction mixture was stirred at rt for 1 h, diluted with MeOH, neutralized with triethylamine (7.54 mmol, 1045.2 µl), and concentrated in vacuo. The precipitate was filtered and washed with MeOH to give dT-Kc quantitatively (179 mg) as a white solid.

1H NMR (CDCl₃, 500 MHz) δ 7.93 (1H, s), 7.41 (1H, d), 6.49 (2H, d), 6.42 (1H, t), 6.16 (1H, dd), 5.30 (1H, dt), 5.09 (2H, s), 4.19 (1H, q), 3.93 (4H, t), 3.93 (1H, m), 2.54 (1H, m), 2.44 (2H, m), 1.93 (3H, d), 1.76 (4H, quint), 1.44 (4H, quint), 1.38-1.21 (73H, m), 0.88 (6H, t); HPLC (Method A): Rt=3.49 min; MS (ESI⁻): m/z calcd for [C₆₂H₁₀₈N₂O₉ - H]⁻: 1023.8, found: 1023.8.

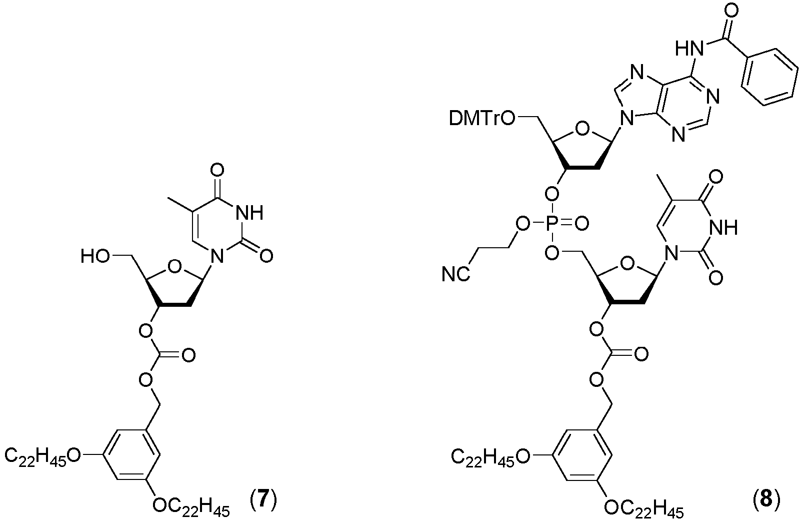

### Example 8: Synthesis of DMTr-dA(Bz)-dT-Kc (8).

To a solution of dT-Kc **7** (1.00 mmol) and DMTr-dA(Bz) phosphoramidite (2.00 mmol, 1.72 g) in CH₂Cl₂ (100 mL) stirred at rt was added 0.25 M 5-benzylmercapto-1H-tetrazole solution in CH₃CN (2.00 mmol, 8 mL). After checking completion of coupling by TLC (hexane:EtOAc = 1:1), a solution of Ac₂O (1.31 mol, 124 µL), pyridine (1.54 mmol, 124 µL) and 1-methylimidazole (1.56 mmol, 124 µL) in CH₂Cl₂ (12 mL) was added. After stirring at rt for 10 min, a solution of 70% t-butyl hydroperoxide solution (0.96 mmol, 130.8 µL) in CH₂Cl₂ (13.5 mL) was added. The resulting reaction mixture was stirred at rt for 30 min and concentrated in vacuo. The precipitate was filtered and washed with MeOH to give DMTr-dA(Bz)-dT-Kc quantitatively (2.19 g) as cream white solid. HPLC (Method A): Rt=4.81 min; MS (ESI⁻): m/z calcd for [C₁₀₃H₁₄₅N₈O₁₇P - C₂F₃O₂]⁻: 1910.0, found: 1910.0.

### Example 9: Synthesis of dA(Bz)-dT-Kc (9).

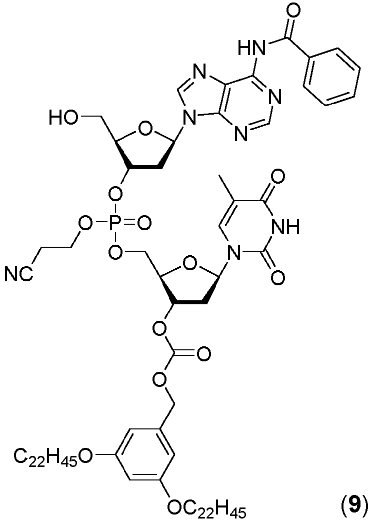

To a solution of DMTr-dA(Bz)-dT-Kc **8** (0.50 mmol) in CH₂Cl₂ (20 mL) stirred at rt was added dichloroacetic acid (7.46 mmol, 616.2 µl). The resulting reaction mixture was stirred at rt for 1 h, diluted with MeOH, neutralized with triethylamine (7.54 mmol, 1045.2 µl), and concentrated in vacuo. The precipitate was filtered and washed with MeOH to give dA(Bz)-dT-Kc in 99% yield (737 mg) as white solid. HPLC (Method A): Rt=3.11 min; MS (ESI⁻): m/z calcd for [C₈₆H₁₂₉N₈O₁₅P + C₂F₃O₂]⁻: 1607.9, found: 1607.9.

### Example 10: Synthesis of dA-dT (10).

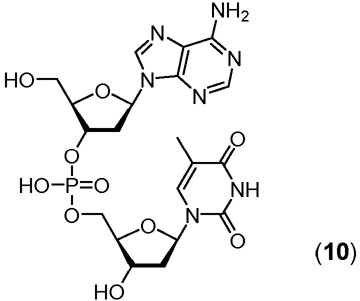

The dA(Bz)-dT-Kc **9** (0.0669 mmol) was added to aqueous 1 M NaOH/tetrahydrofuran solution (13.4 mL, 1:3 v/v). After stirring at 80 °C for 90 min, the resulting reaction mixture was cooled to room temperature. The aqueous layer was separated, neutralized with 6N HCl (1.1 mL), diluted with water, and lyophilized to give crude dA-dT (211 mg).

HPLC (Method B): Rt=2.72 min; MS (ESI⁺): m/z calcd for [C₂₀H₂₆N₇O₁₀P + H]⁺: 556.2, found: 556.2.

### Example 11: Synthesis of dT-Ka (11).

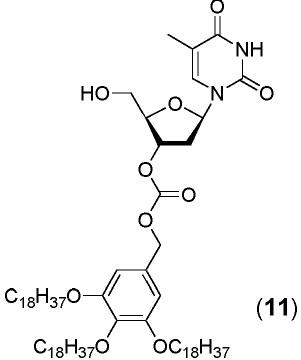

To a solution of DMTr-dT-Ka **5** (0.15 mmol) in CH₂Cl₂ (20 mL) stirred at rt was added dichloroacetic acid (7.46 mmol, 616.2 µl). The resulting reaction mixture was stirred at rt for 1 h, diluted with MeOH, neutralized with triethylamine (7.54 mmol, 1045.2 µl), and concentrated in vacuo. The precipitate was filtered and washed with MeOH to give dT-Ka quantitatively (180 mg) as a white solid. 1H NMR (CDCl₃, 500 MHz) δ 8.23 (1H, s), 7.42 (1H, d), 6.57 (2H, s), 6.16 (1H, dd), 5.30 (1H, dt), 5.06 (2H, s), 4.19 (1H, q), 3.97 (4H, t), 3.94 (4H, t), 2.54 (2H, m), 2.44 (1H, ddd), 1.93 (3H, d), 1.80 (4H, quint), 1.73 (2H, quint), 1.61 (2H, s), 1.47 (6H, quint), 1.39-1.20 (82H, m), 0.88 (9H, t); HPLC (Method A): Rt=4.04 min; MS (ESI⁻): m/z calcd for [C₇₂H₁₂₈N₂O₁₀ - H]⁻: 1179.9, found: 1180.0.

### Example 12: Synthesis of DMTr-dA(Bz)-dT-Ka (12).

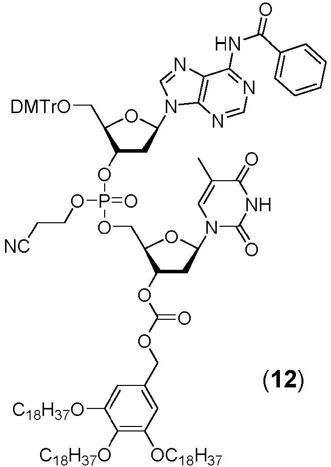

To a solution of dT-Ka **11** (1.00 mmol), DMTr-dA(Bz) phosphoramidite (2.00 mmol, 1.72 g) and in CH₂Cl₂ (100 mL) stirred at rt was added 0.25 M 5-benzylmercapto-1H-tetrazole solution in CH₃CN (2.00 mmol, 8 mL). After checking completion of coupling by TLC (hexane:EtOAc = 1:1), a solution of Ac₂O (1.31 mol, 124 µL), pyridine (1.54 mmol, 124 µL) and 1-methylimidazole (1.56 mmol, 124 µL) in CH₂Cl₂ (12 mL) was added. After stirring at rt for 10 min, a solution of 70% t-butyl hydroperoxide solution (0.96 mmol, 130.8 µL) in CH₂Cl₂ (13.5 mL) was added. The resulting reaction mixture was stirred at rt for 30 min and concentrated in vacuo. The precipitate was filtered and washed with MeOH to give DMTr-dA(Bz)-dT-Ka quantitatively (2.10 g) as creamy white solid. HPLC (Method A): Rt=3.98 min; MS (ESI⁻): m/z calcd for [C₁₁₃H₁₆₆N₈O₁₈P - H]⁻: 1952.2, found: 1952.2.

### Example 13: Synthesis of dA(Bz)-dT-Ka (13).

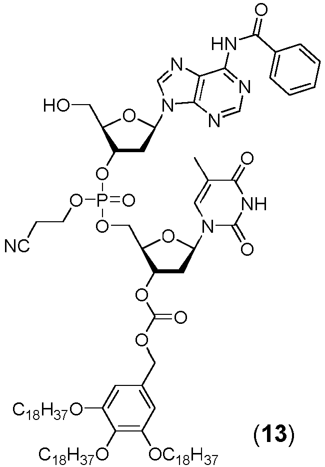

To a solution of DMTr-dA(Bz)-dT-Ka **12** (0.5 mmol) in CH₂Cl₂ (20 mL) stirred at rt was added dichloroacetic acid (7.46 mmol, 616.2 µl). The resulting reaction mixture was stirred at rt for 1 h, diluted with MeOH, neutralized with triethylamine (7.54 mmol, 1045.2 µl), and concentrated in vacuo. The precipitate was filtered and washed with MeOH to give dA(Bz)-dT-Ka in 86% yield (702 mg) as white solid. HPLC (Method A): Rt=3.35 min; MS (ESI⁻): m/z calcd for [C₉₂H₁₄₈N₈O₁₆P - H]⁻: 1650.1, found: 1650.1.

### Example 14: Synthesis of rU(TBS)-Kc (14).

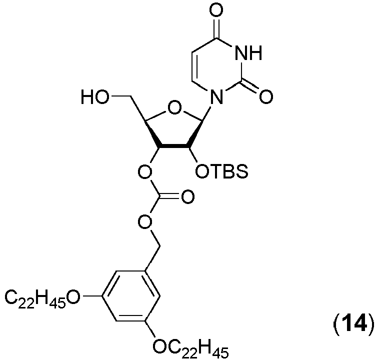

To a solution of DMTr-rU(TBS)-Kc **6** (0.15 mmol) in CH₂Cl₂ (20 mL) stirred at rt was added dichloroacetic acid (7.46 mmol, 616.2 µl). The resulting reaction mixture was stirred at rt for 1 h, diluted with MeOH, neutralized with triethylamine (7.54 mmol, 1045.2 µl), and concentrated *in vacuo.* The precipitate was filtered and washed with MeOH to give rU(TBS)-Kc quantitatively (179 mg) as a white solid. ¹H NMR (CDCl₃, 500 MHz) *δ* 9.29 (1H, s), 7.58 (1H, d), 6.49 (2H, d), 6.42 (1H, t), 5.76 (1H, dd), 5.53 (1H, d), 5.15 (1H, dd), 5.11 (1H, d), 5.03 (1H, d), 4.77 (1H, t), 4.29 (1H, m), 3.92 (4H, t), 3.77 (1H, dd), 3.37 (1H, s), 1.76 (4H, quint), 1.44 (4H, quint), 1.38-1.19 (73H, m), 0.88 (6H, t), 0.84 (9H, s), 0.06 (3H, s), 0.02 (3H, s); HPLC (Method A): Rt=4.01 min; MS (ESI⁻): m/z calcd for [C₆₇H₁₂₀N₂O₁₀Si - H]⁻: 1139.9, found: 1139.9.

### Example 15: Synthesis of DMTr-rA(Bz, TBS)-rU(TBS)-Kc (15).

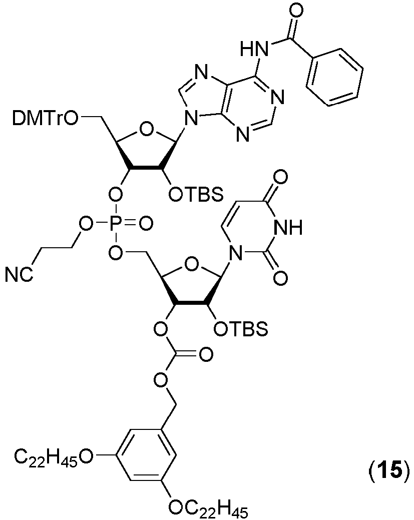

To a solution of rU(TBS)-Kc **14** (0.5 mmol), DMTr-rA(Bz, TBS) phosphoramidite (1.00 mmol, 988.2 mg) in CH₂Cl₂ (100 mL) stirred at rt was added 0.25 M 5-benzylmercapto-1H-tetrazole solution in CH₃CN (2.00 mmol, 8 mL). After checking completion of coupling by TLC (hexane:EtOAc = 1:1), a solution of Ac₂O (1.31 mol, 124 µL), pyridine (1.54 mmol, 124 µL) and 1-methylimidazole (1.56 mmol, 124 µL) in CH₂Cl₂ (12 mL) was added. After stirring at rt for 10 min, a solution of 70% t-butyl hydroperoxide solution (0.96 mmol, 130.8 µL) in CH₂Cl₂ (13.5 mL) was added. The resulting reaction mixture was stirred at rt for 30 min and concentrated *in vacuo.* The precipitate was filtered and washed with MeOH to give DMTr-rA(Bz, TBS)-rU(TBS)-Kc in 82% yield (841 mg) as creamy white solid.HPLC (Method A): Rt=4.76 min; MS (ESI⁻): m/z calcd for [C₁₁₄H₁₇₁N₈O₁₉PSi₂ - C₂F₃O₂]⁻: 2156.2, found: 2156.2.

### Example 16: Synthesis of rA(Bz, TBS)-rU(TBS)-Kc (16).

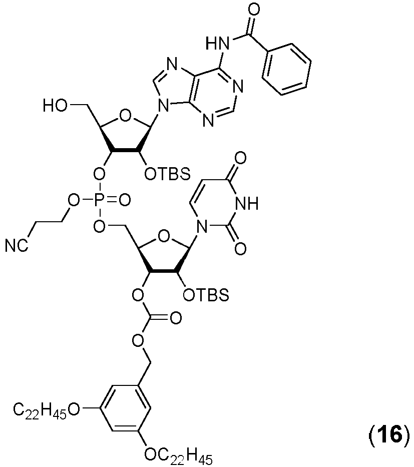

To a solution of DMTr-rA(Bz, TBS)-rU(TBS)-Kc 15 (0.5 mmol) in CH₂Cl₂ (20 mL) stirred at rt was added dichloroacetic acid (7.46 mmol, 616.2 µl). The resulting reaction mixture was stirred at rt for 1 h, diluted with MeOH, neutralized with triethylamine (7.54 mmol, 1045.2 µl), and concentrated *in vacuo.* The precipitate was filtered and washed with MeOH to give rA(Bz, TBS)-rU(TBS)-Kc in 61% yield (531 mg).HPLC (Method A): Rt=4.17 min; MS (ESI⁻): m/z calcd for [C₉₃H₁₅₃N₈O₁₇PSi₂ - C₂F₃O₂]⁻: 1854.0, found: 1854.0.

### Example 17: Synthesis of rA-rU (17).

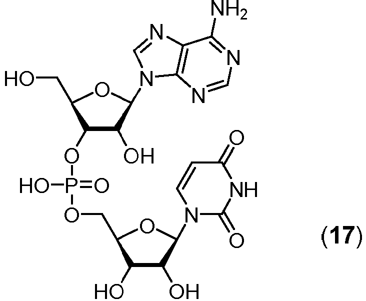

To a solution of rA(Bz, TBS)-rU(TBS)-Kc **16** (5.7 µmol) in THF (1 mL) stirred at rt was added tetrabutylammonium fluoride monohydrate (36.5 mmol, 9.6 mg). The resulting reaction mixture was stirred at rt for 30 min and 1 M NaOH (0.344 mL) was added. The resulting reaction mixture was stirred at 80 °C for 90 min and cooled to room temperature. The aqueous layer was separated and analyzed by HPLC (method C): Rt=2.69 min; LC-MS to find rA-rU. MS (ESI⁺): m/z calcd for [C₁₉H₂₄N₇O₁₂P + H]⁺: 574.1, found: 574.1.

### Example 18: Exemplary syntheses of pseudo solid phase protecting groups

### methyl 2-(3,5-bis(docosyloxy)phenyl)acetate

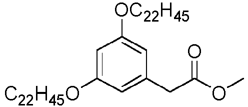

To a solution of methyl 2-(3,5-dihydroxyphenyl)acetate (2.5 mmol) and 1-bromodocosane (3 eq) in DMF/THF (1/1, 100 mL) stirred at rt was added K₂CO₃ (6 eq). The resulting reaction mixture was stirred at 80 °C for 18 h, diluted with MeCN (100 mL) at 60 °C, and filtered. The collected solid was washed with MeCN (60 °C) and dried in vacuo to give methyl 2-(3,5-bis(docosyloxy)phenyl)acetate quantitatively as white solid. ¹H NMR (CDCl₃, 500 MHz) δ 6.40 (2H, d), 6.36 (1H, t), 3.91 (4H, t), 3.69 (3H, s), 3.54 (2H, s), 1.75 (4H, quint), 1.43 (4H, quint), 1.38-1.19 (72H, m), 0.88 (6H, t).

### methyl 3-(2,4-bis(docosyloxy)phenyl)propanoate

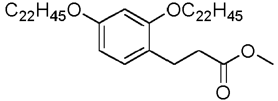

To a solution of methyl 3-(2,4-dihydroxyphenyl)propionate (5 mmol) and 1-bromodocosane (3 eq) in DMF/THF (1/1, 100 mL) stirred at rt was added K₂CO₃ (6 eq). The resulting reaction mixture was stirred at 80 °C for 18 h, diluted with MeCN at 60 °C, and filtered. The collected solid was washed with MeCN (60 °C) and concentrated in vacuo to give methyl 3-(2,4-bis(docosyloxy)phenyl)propanoate quantitatively as white solid. ¹H NMR (CDCl₃, 300 MHz) *δ* 7.01 (1H, d), 6.41 (1H, d), 6.36 (1H, dd), 3.91 (4H, dt), 3.65 (3H, s), 2.86 (2H, t), 2.58 (2H, t), 1.76 (4H, m), 1.44 (4H, m), 1.38-1.19 (72H, m), 0.88 (6H, t).

### 2-(3,5-bis(docosyloxy)phenyl)acetic acid

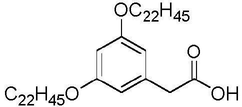

To a solution of methyl 2-(3,5-bis(docosyloxy)phenyl)acetate (2.5 mmol) EtOH/THF (1/1, 50 mL) stirred at rt was added aq. KOH (1 M, 6 eq). The resulting reaction mixture was stirred at 90 °C for 5 h, neutralized with 1 N aq. HCl at rt, and filtered. The collected solid was washed with water/acetone and dried in vacuo to give 2-(3,5-bis(docosyloxy)phenyl)acetic acid in 47% (930 mg) over 2 steps from 2-(3,5-dihydroxyphenyl)acetate (2.5 mmol) as a white solid. ¹H NMR (CDCl₃, 500 MHz) *δ* 6.32 (2H, s), 6.23 (1H, s), 3.80 (4H, m), 3.27 (2H, m), 1.65 (4H, m), 1.34 (4H, m), 1.31-1.19 (73H, m), 0.88 (6H, t).

### 3-(2,4-bis(docosyloxy)phenyl)propanoic acid

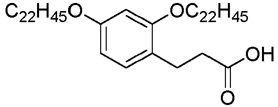

To a solution of methyl 3-(2,4-bis(docosyloxy)phenyl)propanoate (5.0 mmol) EtOH/THF (1/1, 50 mL) stirred at rt was added aq.KOH (1 M, 6 eq). The resulting reaction mixture was stirred at 90 °C for 5 h, neutralized with 1 N aq. HCl at rt, and filtered. The collected solid was washed with water/acetone and concentrated in vacuo to give 3-(2,4-bis(docosyloxy)phenyl)propanoic acid in 49% (1.96 g) over 2 steps from methyl 3-(2,4-dohydroxyphenyl)propionate (5 mmol) as a white solid. ¹H NMR (CDCl₃, 500 MHz) *δ* 7.03 (1H, d), 6.41 (1H, d), 6.37 (1H, dd), 3.92 (4H, q), 2.87 (2H, t), 2.62 (2H, t), 1.77 (4H, m), 1.44 (4H, m), 1.38-1.18 (73H, m), 0.88 (6H, t).

### 2-(3,5-bis(docosyloxy)phenyl)ethan-1-ol

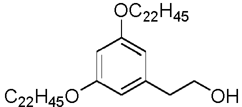

To a solution of 3-(2,4-bis(docosyloxy)phenyl)propanoic acid (2.5 mmol) THF (50 mL) stirred at 0 °C was added LiAlH₄ (1.7 eq, solid form). The resulting reaction mixture was stirred at 0 °C to rt for 24 h, quenched with water, and concentrated in vacuo. The collected solid was washed with MeOH and dried in vacuo to give 2-(3,5-bis(docosyloxy)phenyl)ethan-1-ol quantitatively (2.10 g) as white solid. ¹H NMR (CDCl₃, 300 MHz) *δ* 6.35 (2H, d), 6.34 (1H, d), 3.92 (4H, t), 3.84 (2H, q), 2.79 (2H, t), 1.76 (4H, m), 1.44 (4H, m), 1.38-1.20 (73H, m), 0.88 (6H, t).

### 3-(2,4-bis(docosyloxy)phenyl)propan-1-ol

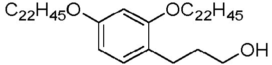

To a solution of 3-(2,4-bis(docosyloxy)phenyl)propanoic acid (1 mmol) THF (50 mL) stirred at 0 °C was added LiAlH₄ (1.7 eq). The resulting reaction mixture was stirred at 0 °C to rt for 24 h, quenched with water, and concentrated in vacuo. The collected solid was washed with MeOH and dried in vacuo to give 3-(2,4-bis(docosyloxy)phenyl)propan-1-ol quantitatively (900 mg) as white solid. ¹H NMR (CDCl₃, 500 MHz) *δ* 7.01 (1H, d), 6.44 (1H, d), 6.41 (1H, dd), 3.93 (4H, m), 3.57 (2H, q), 2.66 (2H, t), 1.78 (6H, m), 1.44 (4H, m), 1.39-1.19 (73H, m), 0.88 (6H, t).

### Example 19: Synthesis of DMTr-dT-Kc-C2 (18).

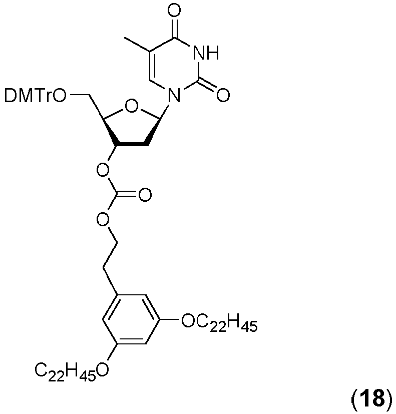

To a solution of DMTr-dT (0.30 mmol, 163 mg) and 1,1'-carbonyldiimidazole (0.60 mmol, 97.3 mg) in CH₂Cl₂ (10 mL) stirred at rt was added 4-dimethylaminopyridine (0.60 mmol, 73.3 mg). The resulting reaction mixture was stirred at rt for 14 h, diluted with NH₄Cl aq and extracted with CH₂Cl₂. The combined organic layers were dried over Na₂SO₄, filtered, and concentrated *in vacuo* to give DMTr-dT-1H-imidazole-1-carboxyl quantitatively (197 mg). To a solution of DMTr-dT-1H-imidazole-1-carboxyl (ca. 0.30 mmol) and DBU (0.23 mmol, 34.3 µL) in CH₂Cl₂ (15 mL) stirred at rt was added 2-(3,5-bis(docosyloxy)phenyl)ethan-1-ol, C₅₂H₉₈O₃ (0.15 mmol, 116 mg). The resulting reaction mixture was stirred at rt for 16 h and concentrated *in vacuo.* The precipitate was filtered and washed with MeOH to give *DMTr-dT-Kc-C2* in 89% (177 mg) as white solid. ¹H NMR (CDCl₃, 500 MHz) δ 7.99 (1H, s), 7.38 (2H, d), 7.30 (2H, q), 7.27 (2H, m), 7.25 (2H, m), 6.84 (4H, d), 6.44 (1H, dd), 6.34 (2H, m), 5.33 (1H, d), 4.32 (1H, t), 4.21 (1H, m), 3.90 (3H, t), 3.79 (6H, s), 3.79 (1H, m), 3.51 (1H, m), 3.44 (1H, m), 2.90 (1H, t), 2.55 (1H, m), 2.44 (1H, m), 1.75 (4H, quint), 1.43 (4H, m), 1.36 (3H, m), 1.34-1.22 (77H, m), 0.88 (6H, t); MS (ESI⁺): m/z calcd for [C₈₄H₁₂₈N₂O₁₁ + C₈H₁₉N + H]⁺: 1471.1, found: 1471.1.

### Example 20: Synthesis of DMTr-dT-Kb-C3 (19).

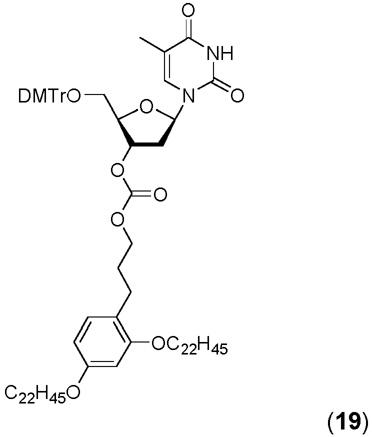

To a solution of DMTr-dT (0.30 mmol, 163 mg) and 1,1'-carbonyldiimidazole (0.60 mmol, 97.3 mg) in CH₂Cl₂ (10 mL) stirred at rt was added 4-dimethylaminopyridine (0.60 mmol, 73.3 mg). The resulting reaction mixture was stirred at rt for 14 h, diluted with NH₄Cl aq and extracted with CH₂Cl₂. The combined organic layers were dried over Na₂SO₄, filtered, and concentrated *in vacuo* to give DMTr-dT-1*H*-imidazole-1-carboxyl quantitatively (197 mg). To a solution of DMTr-dT-1*H*-imidazole-1-carboxyl (ca. 0.30 mmol) and DBU (0.23 mmol, 34.3 µL) in CH₂Cl₂ (15 mL) stirred at rt was added 3-(2,4-bis(docosyloxy)phenyl)propan-1-ol, C₅₃H₁₀₀O₃ (0.15 mmol, 118 mg). The resulting reaction mixture was stirred at rt for 16 h and concentrated *in vacuo.* The precipitate was filtered and washed with MeOH to give DMTr-dT-Kb-C3 in 73% yield (147 mg) as white solid. ¹H NMR (CDCl₃, 500 MHz) δ 8.11 (1H, s), 7.62 (1H, d), 7.38 (2H, m), 7.29 (4H, m), 7.27 (2H, m), 7.25 (2H, m), 6.98 (1H, d), 6.84 (5H, m), 6.46 (1H, dd), 6.41 (1H, d), 6.37 (1H, dd), 5.34 (1H, d), 4.23 (1H, d), 4.13 (2H, t), 3.91 (3H, t), 3.79 (6H, s), 3.78 (1H, m), 3.52 (1H, dd), 3.45 (1H, dd), 2.62 (2H, t), 2.56 (1H, dd), 2.45 (1H, m), 1.94 (2H, quint), 1.76 (4H, quint), 1.47-1.14 (77H, m), 0.88 (6H, t); MS (ESI⁺): m/z calcd for [C₈₅H₁₃₀N₂O₁₁ + C₈H₁₉N + H]⁺: 1485.1, found: 1485.1.

### Example 21: Synthesis of dT-Kc-C2 (20).

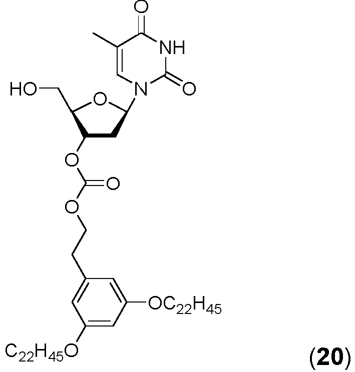

To a solution of DMTr-dT-Kc-C2 **18** (0.15 mmol) in CH₂Cl₂ (20 mL) stirred at rt was added dichloroacetic acid (7.46 mmol, 616.2 µl). The resulting reaction mixture was stirred at rt for 1 h, diluted with MeOH, neutralized with triethylamine (7.54 mmol, 1045.2 µl), and concentrated *in vacuo.* The precipitate was filtered and washed with MeOH to give dT-Kc-C2 in 68% yield (103 mg) as a white solid. ¹H NMR (CDCl₃, 500 MHz) *δ* 8.44 (1H, s), 7.43 (1H, s), 6.35 (3H, m), 6.16 (1H, dd), 5.27 (1H, dt), 4.35 (2H, t), 4.16 (1H, m), 3.95 (1H, m), 3.91 (3H, t), 3.88 (1H, m), 2.91 (2H, t), 2.53 (1H, m), 2.43 (1H, ddd), 2.18 (3H, s), 1.93 (3H, s), 1.76 (4H, quint), 1.44 (4H, quint), 1.37-1.19 (71H, m), 0.88 (6H, t); MS (ESI⁺): m/z calcd for [C₆₃H₁₁₀N₂O₉ + C₈H₁₉N + H]⁺: 1169.0, found: 1169.0.

### Example 22: Synthesis of DMTr-dA(Bz)-dT-Kc-C2 (21).

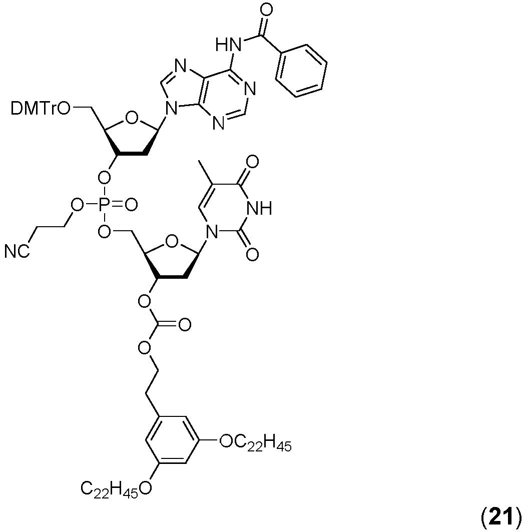

To a solution of dT-Kc-C2 **20** (1.00 mmol), DMTr-dA(Bz) phosphoramidite (2.00 mmol, 1.72 g) and in CH₂Cl₂ (100 mL) stirred at rt was added 0.25 M 5-benzylmercapto-1H-tetrazole solution in CH₃CN (2.00 mmol, 8 mL). After checking completion of coupling by TLC (hexane:EtOAc = 1:1), a solution of Ac₂O (1.31 mmol, 124 µL), pyridine (1.54 mmol, 124 µL) and 1-methylimidazole (1.56 mmol, 124 µL) in CH₂Cl₂ (12 mL) was added. After stirring at rt for 10 min, a solution of 70% t-butyl hydroperoxide solution (0.96 mmol, 130.8 µL) in CH₂Cl₂ (13.5 mL) was added. The resulting reaction mixture was stirred at rt for 30 min and concentrated *in vacuo.* The precipitate was filtered and washed with MeOH to give DMTr-dA(Bz)-dT-Kc-C2 in 68% yield (1.23 g) as a cream white solid. MS (ESI⁺): m/z calcd for [C₁₀₄H₁₄₇N₈O₁₇P + H] ⁺: 1812.1, found: 1812.1.

### Example 23: Synthesis of dA(Bz)-dT-Kc-C2 (22).

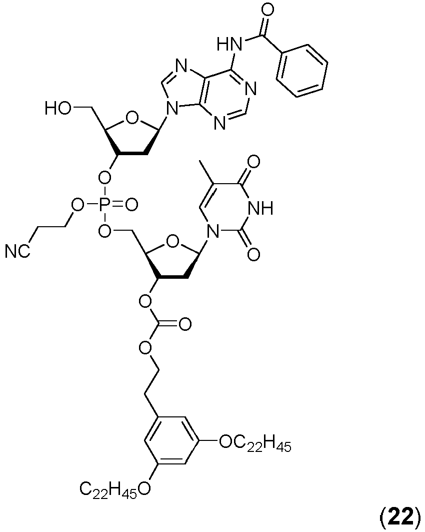

To a solution of DMTr-dA(Bz)-dT-Kc-C2 **21** (0.5 mmol) in CH₂Cl₂ (20 mL) stirred at rt was added dichloroacetic acid (7.46 mmol, 616.2 µl). The resulting reaction mixture was stirred at rt for 1 h, diluted with MeOH, neutralized with triethylamine (7.54 mmol, 1045.2 µl), and concentrated *in vacuo.* The precipitate was filtered and washed with MeOH to give dA(Bz)-dT-Kc-C2 in 84% yield (630 mg) as a cream white solid. MS (ESI⁺): m/z calcd for [C₈₃H₁₂₉N₈O₁₇P + H] ⁺: 1509.9, found: 1509.9.

### Example 24: Synthesis of dT-Kb-C3 (23).

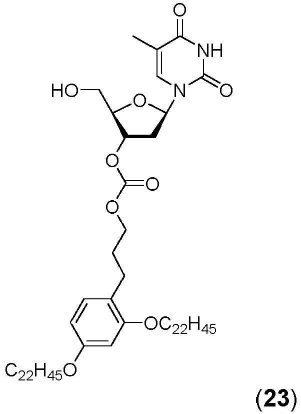

To a solution of DMTr-dT-Kb-C3 **19** (0.15 mmol) in CH₂Cl₂ (20 mL) stirred at rt was added dichloroacetic acid (7.46 mmol, 616.2 µl). The resulting reaction mixture was stirred at rt for 1 h, diluted with MeOH, neutralized with triethylamine (7.54 mmol, 1045.2 µl), and concentrated *in vacuo.* The precipitate was filtered and washed with MeOH to give dT-Kc-C3 in 40% yield (46 mg) as a white solid. ¹H NMR (CDCl₃, 500 MHz) *δ* 8.08 (1H, s), 7.71 (1H, m), 7.53 (1H, m), 6.99 (1H, d), 6.42 (1H, d), 6.38 (1H, dd), 6.18 (1H, m), 5.27 (1H, dt), 4.29 (1H, m), 4.15 (2H, m), 3.92 (5H, m), 3.66 (2H, m), 3.56 (1H, m), 3.38 (1H, s), 2.63 (2H, t), 2.53 (1H, m), 2.45 (1H, m), 1.94 (4H, m), 1.76 (4H, m), 1.44 (4H, m), 1.37-1.11 (70H, m), 0.88 (6H, t); MS (ESI⁺): m/z calcd for [C₆₄H₁₁₂N₂O₉ + C₈H₁₉N + H] ⁺: 1183.0, found: 1183.0.

### Example 25: Synthesis of DMTr-dA(Bz)-dT-Kb-C3 (24).

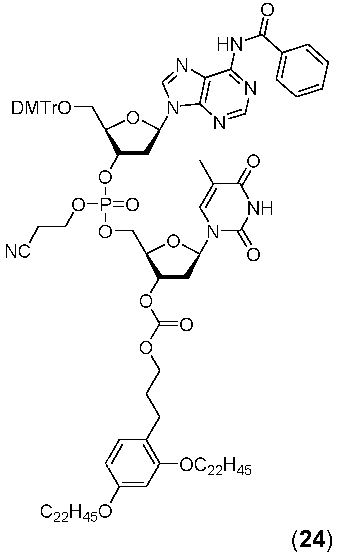

To a solution of dT-Kb-C3 **23** (1.00 mmol), DMTr-dA(Bz) phosphoramidite (2.00 mmol, 1.72 g) and in CH₂Cl₂ (100 mL) stirred at rt was added 0.25 M 5-benzylmercapto-1H-tetrazole solution in CH₃CN (2.00 mmol, 8 mL). After checking completion of coupling by TLC (hexane:EtOAc = 1:1), a solution of Ac₂O (1.31 mmol, 124 µL), pyridine (1.54 mmol, 124 µL) and 1-methylimidazole (1.56 mmol, 124 µL) in CH₂Cl₂ (12 mL) was added. After stirring at rt for 10 min, a solution of 70% t-butyl hydroperoxide solution (0.96 mmol, 130.8 µL) in CH₂Cl₂ (13.5 mL) was added. The resulting reaction mixture was stirred at rt for 30 min and concentrated *in vacuo.* The precipitate was filtered and washed with MeOH to give DMTr-dA(Bz)-dT-Kb-C3 in 93% yield (1.31 g) as a cream white solid. MS (ESI⁺): m/z calcd for [C₁₀₅H₁₄₉N₈O₁₇P + H] ⁺: 1826.1, found: 1826.1.

### Example 26: Synthesis of dA(Bz)-dT-Kb-C3 (25).

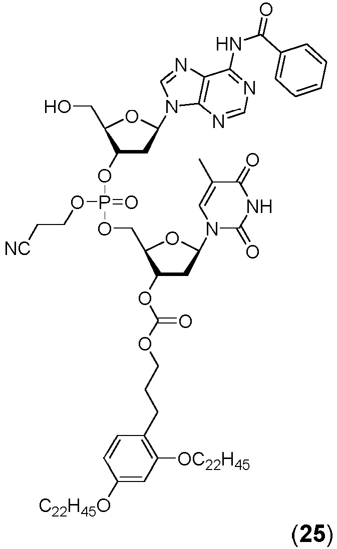

To a solution of DMTr-dA(Bz)-dT-Kb-C3 **24** (0.5 mmol) in CH₂Cl₂ (20 mL) stirred at rt was added dichloroacetic acid (7.46 mmol, 616.2 µl). The resulting reaction mixture was stirred at rt for 1 h, diluted with MeOH, neutralized with triethylamine (7.54 mmol, 1045.2 µl), and concentrated *in vacuo.* The precipitate was filtered and washed with MeOH to give dA(Bz)-dT-Kb-C3 in 87% yield (663 mg) as a cream white solid. MS (ESI⁺): m/z calcd for [C₈₄H₁₃₁N₈O₁₅P + H] ⁺: 1524.0, found: 1524.0.

### Example 27: Synthesis of DMTr-dT-Kb-C2 acid (26).

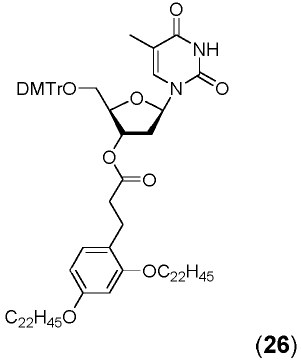

To a solution of DMTr-dT (0.30 mmol, 163 mg), DIPEA (0.45 mmol, 78.6 µL), COMU (0.3 mmol, 128.5 mg) and DMAP (0.02 mmol, 2.4 mg) in CH₂Cl₂ (20 mL) stirred at rt was added 3-(2,4-bis(docosyloxy)phenyl)propanoic acid, C₅₃H₉₈O₄ (Kb-C2 acid tag from Example 18, 0.15 mmol, 120 mg). The resulting reaction mixture was stirred at rt for 16 h and concentrated *in vacuo.* The precipitate was filtered and washed with MeOH to give DMTr-dT-Kc-C2 acid in 57% (114 mg) as white solid. ¹H NMR (CDCl₃, 500 MHz) δ 8.81 (1H, m), 7.61 (1H, s), 7.38 (2H, d), 7.27 (6H, m), 6.99 (1H, d), 6.84 (3H, d), 6.41 (1H, s), 6.37 (1H, dd), 5.44 (1H, s), 4.03 (1H, s), 3.90 (4H, dt), 3.79 (6H, s), 3.44 (1H, s), 3.41 (1H, m), 2.85 (2H, m), 2.61 (2H, t), 2.40 (1H, m), 1.76 (4H, dq), 1.44 (4H, m), 1.38-1.13 (79H, m), 0.88 (6H, t); MS (ESI⁺): m/z calcd for [C₈₃H₁₂₆N₂O₁₀ + C₈H₁₉N + H]⁺: 1455.1, found: 1455.1.

### Example 28: Synthesis of DMTr-dT-Kc-C1 acid (27).

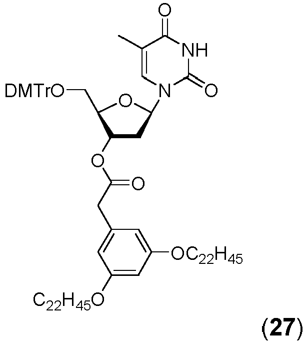

To a solution of DMTr-dT (0.30 mmol, 163 mg), DIPEA (0.45 mmol, 78.6 µL), COMU (0.3 mmol, 128.5 mg) and DMAP (0.02 mmol, 2.4 mg) in CH₂Cl₂ (20 mL) stirred at rt was added 2-(3,5-bis(docosyloxy)phenyl)acetic acid, C₅₂H₉₆O₄ (0.15 mmol, 118 mg). The resulting reaction mixture was stirred at rt for 16 h and concentrated *in vacuo.* The precipitate was filtered and washed with MeOH to give DMTr-dT-Kc-C1 acid in 80% (158 mg) as white solid. ¹H NMR (CDCl₃, 300 MHz) δ 9.10 (1H, s), 7.63 (1H, s), 7.37 (2H, m), 7.26 (5H, m), 6.83 (5H, d), 6.46 (2H, m), 6.37 (4H, m), 5.47 (1H, m), 4.11 (1H, m), 3.90 (6H, t), 3.78 (6H, s), 3.55 (2H, s), 3.46 (2H, s), 3.21 (1H, d), 2.97 (1H, d), 2.47 (2H, q), 1.74 (4H, quint), 1.42 (4H, m), 1.35-1.17 (70H, m), 0.88 (6H, t); MS m/z: [M + C₈H₁₉N + H]⁺ calcd for C₉₁H₁₄₆N₃O₁₀⁺, 1441.1; found 1441.1.

### Example 29: Synthesis of DMTr-2'O-Methyl-rU-Kb-C2 acid (28).

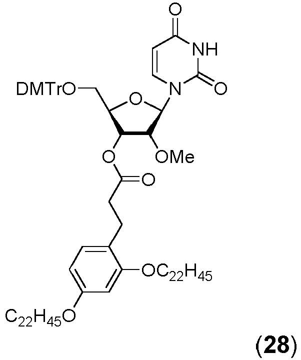

To a solution of DMTr-2'O-*Methyl*-rU (0.30 mmol, 163 mg), DIPEA (0.45 mmol, 78.6 µL), COMU (0.3 mmol, 128.5 mg) and DMAP (0.02 mmol, 2.4 mg) in CH₂Cl₂ (20 mL) stirred at rt was added 3-(2,4-bis(docosyloxy)phenyl)propanoic acid, C₅₃H₉₈O₄ (0.15 mmol, 120 mg). The resulting reaction mixture was stirred at rt for 16 h and concentrated *in vacuo.* The precipitate was filtered and washed with MeOH to give DMTr-2'O-*Methyl* -rU-Kb-C2 acid quantitatively (252 mg) as white solid. ¹H NMR (CDCl₃, 500 MHz) δ 9.07 (1H, s), 7.92 (1H, d), 7.36 (1H, m), 7.29 (2H, m), 7.25 (2H, m), 7.02 (1H, m), 6.84 (3H, m), 6.41 (2H, m), 6.37 (1H, dt), 6.34 (1H, dd), 6.01 (1H, d), 5.96 (1H, m), 5.31 (1H, d), 5.26 (1H, m), 3.92 (2H, m), 3.88 (2H, m), 3.79 (3H, s), 3.75 (6H, s), 3.69 (1H, t), 3.43 (1H, s), 2.95 (1H, d), 2.87 (2H, m), 2.85 (1H, s), 2.62 (2H, m), 1.76 (4H, m), 1.43 (4H, m), 1.36-1.17 (74H, m), 0.88 (6H, t); MS (ESI⁺): m/z calcd for [C₈₃H₁₂₆N₂O₁₀ + C₈H₁₉N + H] ⁺: 1441.1, found: 1441.1.

### Example 30: Synthesis of dT-Kb-C2 acid (29).

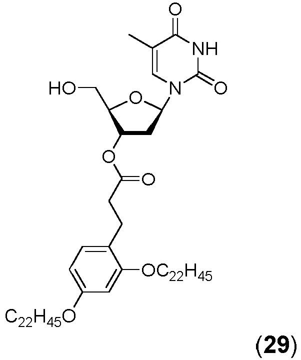

To a solution of DMTr-dT-Kb-C2 acid **26** (0.15 mmol) in CH₂Cl₂ (20 mL) stirred at rt was added dichloroacetic acid (7.46 mmol, 616.2 µl). The resulting reaction mixture was stirred at rt for 1 h, diluted with MeOH, neutralized with triethylamine (7.54 mmol, 1045.2 µl), and concentrated *in vacuo.* The precipitate was filtered and washed with MeOH to give dT-Kc-C2 in 46% yield (70 mg) as a white solid. ¹H NMR (CDCl₃, 500 MHz) *δ* 8.96 (1H, s), 7.48 (1H, d), 7.00 (1H, d), 6.43 (1H, d), 6.37 (1H, dd), 6.16 (1H, dd), 5.31 (1H, dt), 3.99 (1H, q), 3.92 (4H, dt), 3.87 (2H, m), 2.87 (2H, t), 2.75 (1H, m), 2.63 (2H, t), 2.41 (1H, m), 2.30 (1H, ddd), 1.92 (3H, s), 1.84 (1H, m), 1.77 (4H, dq), 1.46 (4H, m), 1.38-1.20 (71H, m), 0.88 (6H, t); MS (ESI⁺): m/z calcd for [C₆₃H₁₁₀N₂O₈ + C₈H₁₉N + H] ⁺: 1153.0, found: 1153.0.

### Example 31: Synthesis of DMTr-dA(Bz)-dT-Kb-C2 acid (30).

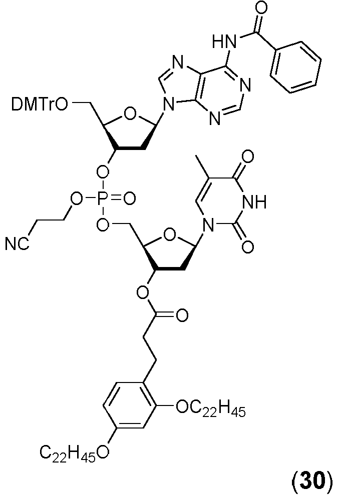

To a solution of dT-Kb-C2 acid **29** (1.00 mmol), DMTr-dA(Bz) phosphoramidite (2.00 mmol, 1.72 g) and in CH₂Cl₂ (100 mL) stirred at rt was added 0.25 M 5-benzylmercapto-1H-tetrazole solution in CH₃CN (2.00 mmol, 8 mL). After checking completion of coupling by TLC (hexane:EtOAc = 1:1), a solution of Ac₂O (1.31 mmol, 124 µL), pyridine (1.54 mmol, 124 µL) and 1-methylimidazole (1.56 mmol, 124 µL) in CH₂Cl₂ (12 mL) was added. After stirring at rt for 10 min, a solution of 70% t-butyl hydroperoxide solution (0.96 mmol, 130.8 µL) in CH₂Cl₂ (13.5 mL) was added. The resulting reaction mixture was stirred at rt for 30 min and concentrated *in vacuo.* The precipitate was filtered and washed with MeOH to give DMTr-dA(Bz)-dT-Kb-C2 acid in 67% yield (1.20 g) as a cream white solid. MS (ESI⁺): m/z calcd for [C₁₀₄H₁₄₇N₈O₁₆P + H] ⁺: 1796.1, found: 1796.1.

### Example 32: Synthesis of dA(Bz)-dT-Kb-C2 acid (31).

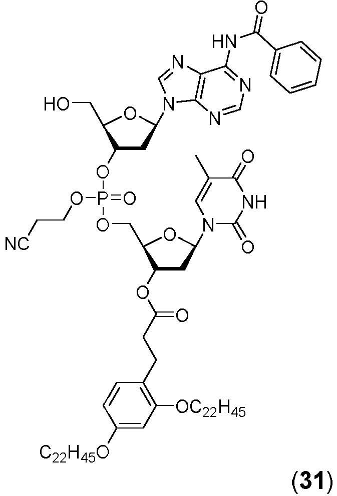

To a solution of DMTr-dA(Bz)-dT-Kb-C2 acid **30** (0.5 mmol) in CH₂Cl₂ (40 mL) stirred at rt was added dichloroacetic acid (14.9 mmol, 1.23 mL). The resulting reaction mixture was stirred at rt for 1 h, diluted with MeOH, neutralized with triethylamine (14.9 mmol, 2.09 mL), and concentrated *in vacuo.* The precipitate was filtered and washed with MeOH to give dA(Bz)-dT-Kb-C2 acid in 87% yield (601 mg) as a cream white solid. MS (ESI⁺): m/z calcd for [C₈₃H₁₂₉N₈O₁₄P + H] ⁺: 1493.9, found: 1493.9.

### Example 33: Synthesis of dT-Kc-C1 acid (32).

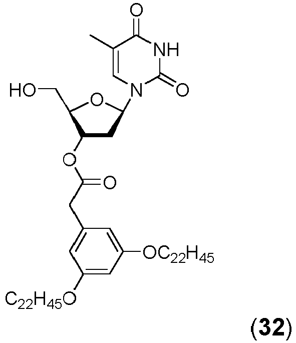

To a solution of DMTr-dT-Kc-C1 acid **27** (0.15 mmol) in CH₂Cl₂ (20 mL) stirred at rt was added dichloroacetic acid (7.46 mmol, 616.2 µl). The resulting reaction mixture was stirred at rt for 1 h, diluted with MeOH, neutralized with triethylamine (7.54 mmol, 1045.2 µl), and concentrated *in vacuo.* The precipitate was filtered and washed with MeOH to give dT-Kc-C1 acid in 75% yield (114 mg) as a white solid. ¹H NMR (CDCl₃, 500 MHz) *δ* 8.66 (1H, s), 7.49 (1H, d), 6.39 (2H, d), 6.35 (2H, m), 6.22 (1H, dd), 5.35 (1H, dt), 4.07 (1H, dt), 3.91 (6H, m), 3.56 (1H, s), 3.55 (1H, m), 2.97 (1H, d), 2.55 (1H, m), 2.44 (1H, m), 2.37 (1H, dd), 1.92 (3H, d), 1.75 (4H, m), 1.42 (4H, m), 1.38-1.19 (70H, m), 0.88 (6H, t); MS (ESI⁺): m/z calcd for [C₆₂H₁₀₈N₂O₈ + C₈H₁₉N + H] ⁺: 1139.0, found: 1139.0.

### Example 34: Synthesis of DMTr-dA(Bz)-dT-Kc-C1 acid (33).

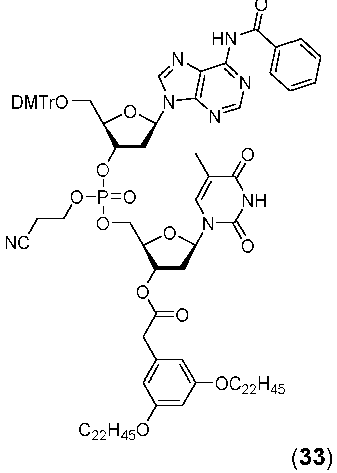

To a solution of dT-Kc-C1 acid **32** (1.00 mmol), DMTr-dA(Bz) phosphoramidite (2.00 mmol, 1.72 g) and in CH₂Cl₂ (100 mL) stirred at rt was added 0.25 M 5-benzylmercapto-*1H*-tetrazole solution in CH₃CN (2.00 mmol, 8 mL). After checking completion of coupling by TLC (hexane:EtOAc = 1:1), a solution of Ac₂O (1.31 mmol, 124 µL), pyridine (1.54 mmol, 124 µL) and 1-methylimidazole (1.56 mmol, 124 µL) in CH₂Cl₂ (12 mL) was added. After stirring at rt for 10 min, a solution of 70% t-butyl hydroperoxide solution (0.96 mmol, 130.8 µL) in CH₂Cl₂ (13.5 mL) was added. The resulting reaction mixture was stirred at rt for 30 min and concentrated *in vacuo.* The precipitate was filtered and washed with MeOH to give DMTr-dA(Bz)-dT-Kc-C1 acid in 80% yield (1.42 g) as a cream white solid. MS (ESI⁺): m/z calcd for [C₁₀₃H₁₄₅N₈O₁₆P + H] ⁺: 1782.1, found: 1782.0.

### Example 35: Synthesis of dA(Bz)-dT-Kc-C1 acid (34).

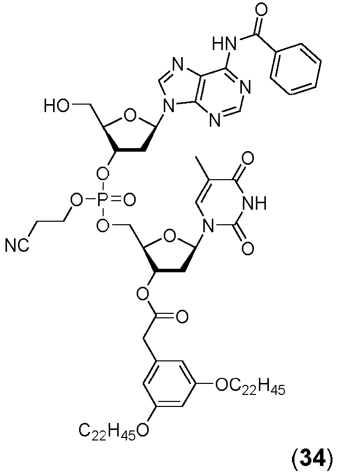

To a solution of DMTr-dA(Bz)-dT-Kc-C1 acid **33** (0.5 mmol) in CH₂Cl₂ (40 mL) stirred at rt was added dichloroacetic acid (14.9 mmol, 1.23 mL). The resulting reaction mixture was stirred at rt for 1 h, diluted with MeOH, neutralized with triethylamine (14.9 mmol, 2.09 mL), and concentrated *in vacuo.* The precipitate was filtered and washed with MeOH to give dA(Bz)-dT-Kc-C1 acid in 90% yield (665 mg) as a cream white solid. MS (ESI⁺): m/z calcd for [C₈₂H₁₂₇N₃O₁₄P + H] ⁺: 1479.9, found: 1480.0.

### Example 36: Synthesis of 2'-O-Methyl-rU(Me)-Kb-C2 acid (35).

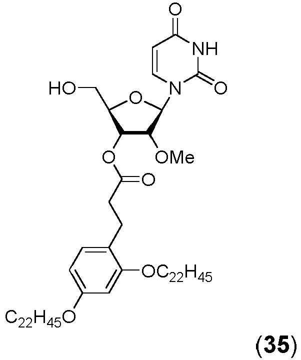

To a solution of DMTr-2'O-Methyl-rU-Kb-C2 acid **28** (0.15 mmol) in CH₂Cl₂ (20 mL) stirred at rt was added dichloroacetic acid (7.46 mmol, 616.2 µl). The resulting reaction mixture was stirred at rt for 1 h, diluted with MeOH, neutralized with triethylamine (7.54 mmol, 1045.2 µl), and concentrated *in vacuo.* The precipitate was filtered and washed with MeOH to give 2'O-Methyl-rU-Kb-C2 acid quantitatively (169 mg) as a white solid. ¹H NMR (CDCl₃, 500 MHz) *δ* 7.69 (1H, d), 7.01 (1H, d), 6.20 (1H, d), 6.36 (1H, dd), 5.74 (1H, d), 5.68 (1H, d), 5.24 (1H, t), 4.29 (1H, m), 4.19 (1H, t), 4.12 (1H, dt), 3.92 (4H, m), 3.70 (1H, d), 3.42 (3H, s), 2.94 (1H, d), 2.88 (3H, m), 2.70 (2H, t), 1.77 (4H, m), 1.44 (4H, m), 1.38-1.19 (72H, m), 0.88 (6H, t); MS (ESI⁺): m/z calcd for [C₆₃H₁₁₀N₂O₉ + C₈H₁₉N + H] ⁺: 1169.0, found: 1169.0.

### Example 37: Synthesis of DMTr-2'O-Methyl-rA(Bz)- 2'O-Methyl-rU-Kb-C2 acid (36).

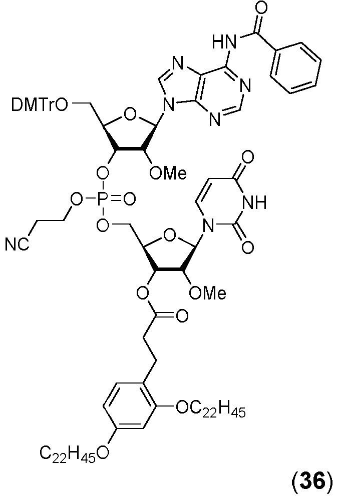

To a solution of 2'O-Methyl-rU-Kb-C2 acid **35** (1.00 mmol), DMTr-2'O-Methyl-rA(Bz) phosphoramidite (2.00 mmol, 1.72 g) and in CH₂Cl₂ (100 mL) stirred at rt was added 5-(Benzylthio)-1*H*-tetrazole (3.00 mmol, 577 mg). After checking completion of coupling by TLC (hexane:EtOAc = 1:1), a solution of Ac₂O (1.31 mmol, 124 µL), pyridine (1.54 mmol, 124 µL) and 1-methylimidazole (1.56 mmol, 124 µL) in CH₂Cl₂ (12 mL) was added. After stirring at rt for 10 min, a solution of 70% t-butyl hydroperoxide solution (0.96 mmol, 130.8 µL) in CH₂Cl₂ (13.5 mL) was added. The resulting reaction mixture was stirred at rt for 30 min and concentrated *in vacuo.* The precipitate was filtered and washed with MeOH to give DMTr-2'O-Methyl-rA(Bz)- 2'O-Methyl-rU-Kb-C2 acid in 79% yield (1.45 g) as a cream white solid. MS (ESI⁺): m/z calcd for [C₁₀₅H₁₄₉N₈O₁₈P + H] ⁺: 1842.1.0, found: 1842.1.

### Example 38: Synthesis 2'O-Methyl-rA(Bz)- 2'O-Methyl-rU-Kb-C2 acid (37).

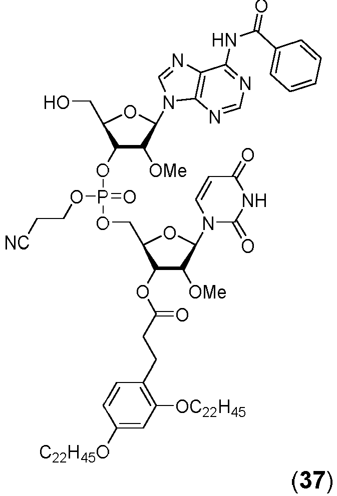

To a solution of DMTr-2'O-Methyl-rA(Bz)- 2'O-Methyl-rU-Kb-C2 acid 36 (0.5 mmol) in CH₂Cl₂ (20 mL) stirred at rt was added dichloroacetic acid (7.46 mmol, 616.2 µl). The resulting reaction mixture was stirred at rt for 1 h, diluted with MeOH, neutralized with triethylamine (7.54 mmol, 1045.2 µl), and concentrated *in vacuo.* The precipitate was filtered and washed with MeOH to give 2'O-Methyl-rA(Bz)- 2'O-Methyl-rU-Kb-C2 acid in 46% yield (358 mg) as a cream white solid. MS (ESI⁺): m/z calcd for [C₈₄H₁₃₁N₈O₁₆P + H] ⁺: 1539.9, found: 1540.0.

### Example 39: Synthesis of 2'O-Methyl-rA- 2'O-Methyl-rU (38).

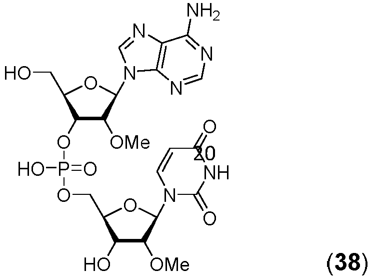

2'O-Methyl-rA(Bz, Me)- 2'*O-Methyl*-rU-Kb-C2 acid (0.0660 mmol) was added to 1 M NaOH water/tetrahydrofuran solution (13.4 mL, 1:3 v/v). After stirring at 80 °C for 90 min, the resulting reaction mixture was cooled to room temperature. The aqueous layer was separated, neutralized with 6N HCl, diluted with water, and lyophilized to give crude 2'*O-Methyl-rA-2'O-Methyl-rU* (176 mg). MS (ESI⁺): m/z calcd for [C₂₁H₂₈N₇O₁₂P + H] ⁺: 602.2, found: 602.2.

### Example 40: Synthesis of dA-dT (39).

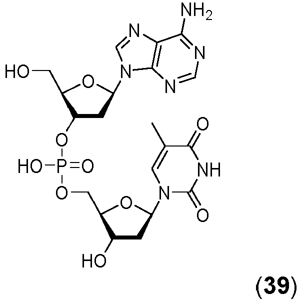

dA(Bz)-dT-Kb-C3 **25**(0.0676 mmol) was added to 1 M NaOH water/tetrahydrofuran solution (13.4 mL, 1:3 v/v). After stirring at 80 °C for 90 min, the resulting reaction mixture was cooled to room temperature. The aqueous layer was separated, neutralized with 6N HCl, diluted with water, and lyophilized to give crude *dA-dT* (231 mg). MS (ESI⁺): m/z calcd for [C₂₀H₂₆N₇O₁₀P + H] ⁺: 556.2, found: 556.2.

### Description of the Figures

**Figure 1** Non limiting examples of R⁶ moietes. * denotes the point of attachment to the oxygen atom of the R⁴, R⁵, R⁸, R⁹ or R¹⁰ moieties of the pseudo solid phase protecting groups or compounds according to any one of formulae I, I-a, I-b, Ic, I-b-1, II, 11-1, 11-2, II-a, II-a-1, II-a-2 , XIII, XIII-b to XIII-g, XIV, and XIV-b to XIV-g, or to the oxygen atoms denoted in any one of formulae III to VII, VIII to XII, VIII-1 to X-1.
**Figure 2** Non-limiting examples of building blocks suitable for the synthesis of oligonucleotides with a phosphoribose backbone.
   (a) Building block for synthesis of DNA by the phosphoramidite approach;
   (b) Building block for synthesis of RNA by the phosphoramidite approach;
   (c) Phosphoramidite building block with 2'-O-methoxy ribose;
   (d) Phosphoramidite building block with 2'-O-(2-methoxyethoxy) ribose;
   (e) Phosphoramidite building block with 2'-fluoro ribose.
**Figure 3** shows non-limiting examples of phosphoramidite building blocks for the synthesis of oligonucleotide analogs containing ribose surrogates (a) protected building block for obtaining arabinose nucleic acid (ANA), (b) building block for obtaining 2'-fluoroarabinose nucleic acid (FANA), (c) building block for obtaining threose nucleic acid (TNA).

The following types of building blocks can be obtained as stereoisomers, the displayed structures represent one of these compounds: (d) building block for obtaining 1',3'-anhydrothreitol nucleic acid (HNA), (e) building block for obtaining locked nucleic acid (LNA), (f) building block for obtaining ethylene-bridged nucleic acid (ENA), (g) building block for obtaining tricyclic nucleic acid (tc-DNA), (h) building block for obtaining cyclohexene nucleic acid (CeNA), (i) shows a building block in which ribose has been replaced by glycerol (GNA). The protected building block (j) for obtaining unlocked nucleic acid (UNA) is obtained by oxidative ring cleavage of ribose.

## Claims

1. A pseudo solid phase protecting group for protecting a nucleoside, a nucleotide, an oligonucleotide, or a conjugate of a nucleoside, a nucleotide or an oligonucleotide, wherein the pseudo solid phase protecting group is represented by formula II: 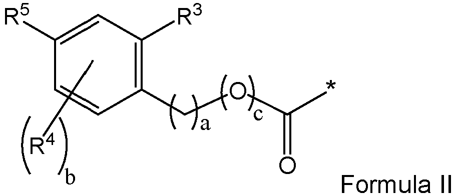 wherein:
* indicates the point of attachment to an oxygen atom of a hydroxyl moiety of the nucleoside, the nucleotide, the oligonucleotide, or of the conjugate of a nucleoside, a nucleotide or an oligonucleotide to be protected;
c is 0 or 1;
a is an integer of 1 to 3;
R³ is H;
R⁵ is O-R⁶ or H, where R⁶ is a C8-C40 aliphatic hydrocarbon group;
each of R⁴ is independently O-R⁶, where R⁶ is at each occurrence independently a C8-C40 aliphatic hydrocarbon group;
b is 1 to 3;
with the proviso that if b is 1, at least one of R³ and R⁵ is not H;
and with the further proviso that the sum of all carbon atoms contained in the R³, R⁴, and R⁵ moieties present is larger than 23 and smaller than 200.

2. The pseudo solid phase protecting group according to claim 1, wherein b is an integer of 1 to 2.

3. The pseudo solid phase protecting group according to any one of claims 1 and 2, which is represented by any one of formulae III to VII: 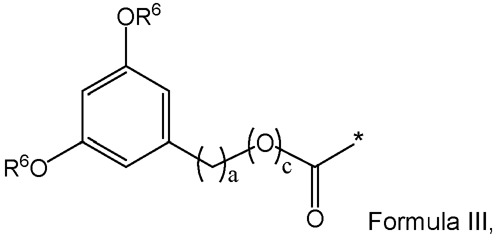 wherein in formula III, * indicates the point of attachment to the oxygen atom of a hydroxyl moiety to be protected; c is 0 or 1; a is an integer of 1 to 3; and each of R⁶ is independently a C12-C40 aliphatic hydrocarbon group; 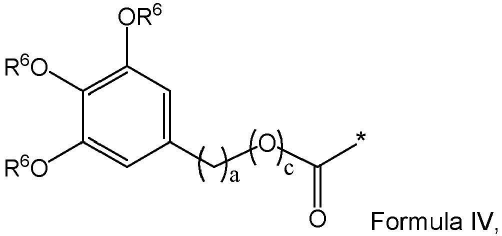 wherein in formula IV, * indicates the point of attachment to the oxygen atom of a hydroxyl moiety to be protected; c is 0 or 1; a is an integer of 1 to 3; and each of R⁶ is independently a C8-C40 aliphatic hydrocarbon group; 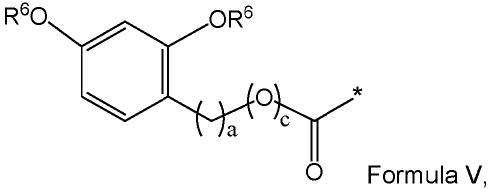 wherein in formula V, * indicates the point of attachment to the oxygen atom of a hydroxyl moiety to be protected; c is 0 or 1; a is an integer of 2 to 3; and each of R⁶ is independently a C12-C40 aliphatic hydrocarbon group; 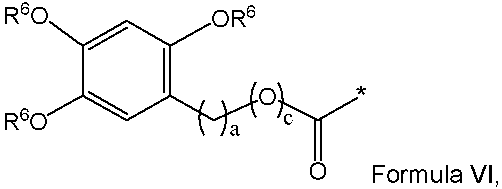 wherein in formula VI, * indicates the point of attachment to the oxygen atom of a hydroxyl moiety to be protected; c is 0 or 1; a is an integer of 2 to 3; and each of R⁶ is independently a C8-C40 aliphatic hydrocarbon group; and 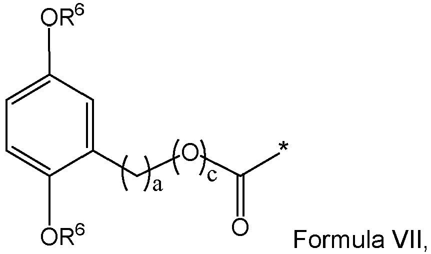 wherein in formula VII, * indicates the point of attachment to the oxygen atom of a hydroxyl moiety to be protected; c is 0 or 1; a is an integer of 1 to 3; and each of R⁶ is independently a C12-C40 aliphatic hydrocarbon group.

4. The pseudo solid phase protecting group according to any one of claims 1 to 3, wherein c is 1.

5. A compound according to formula I: 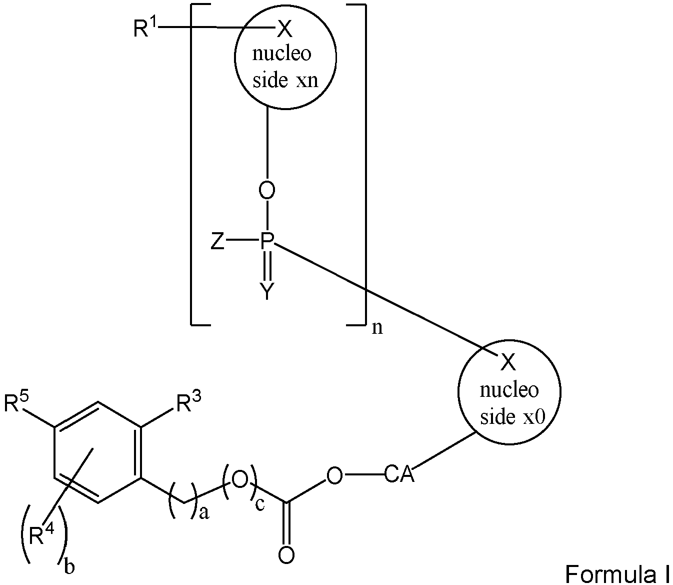 wherein:
R¹ is a protecting group;
X is independently at each position O or N;
n is an integer equal to or larger than 0;
Z is independently at each position an electron withdrawing group or H;
Y is independently for each repetitive unit n missing or O or S;
each of the nucleosides x0 to xn may be the same or different;
CA is a capping moiety or a single bond;
c is 0 or 1;
a is an integer of 1 to 3;
R³ is H;
R⁵ is O-R⁶ or H where R⁶ is a C8-C40 aliphatic hydrocarbon group;
each of R⁴ is independently O-R⁶, where R⁶ is at each occurrence independently a C8-C40 aliphatic hydrocarbon group;
b is 1 to 3;
with the proviso that if b is 1, at least one of R³ and R⁵ is not H;
and with the further proviso that the sum of all carbon atoms contained in the R³, R⁴, and R⁵ moieties present is larger than 23 and smaller than 200.

6. The compound according to claim 5, wherein b is an integer of 1 to 2.

7. The compound according to any one of claims 5 and 6, which is a compound according to any one of formulae VIII to XII: 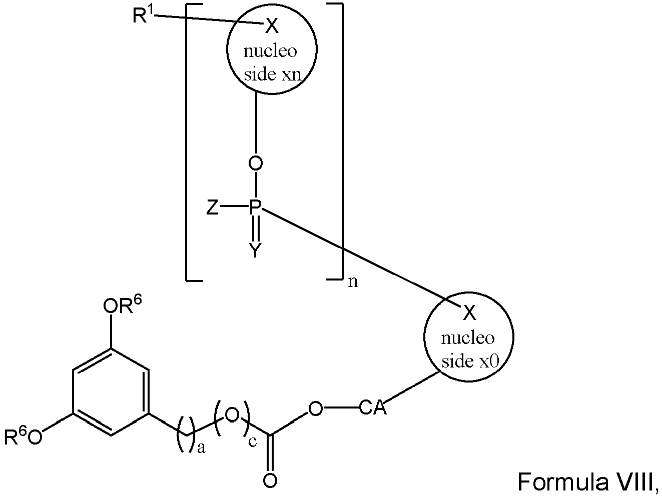
wherein in formula VIII, c is 0 or 1; a is an integer of 1 to 3; each of R⁶ is independently a C12-C40 aliphatic hydrocarbon group; R¹ is a protecting group;
X is independently at each position O or N; n is an integer equal to or larger than 0; Z is independently at each position H or an electron withdrawing group; Y is independently for each repetitive unit n missing or O or S; each of the nucleosides x0 to xn may be the same or different; and CA is a capping moiety or a single bond; 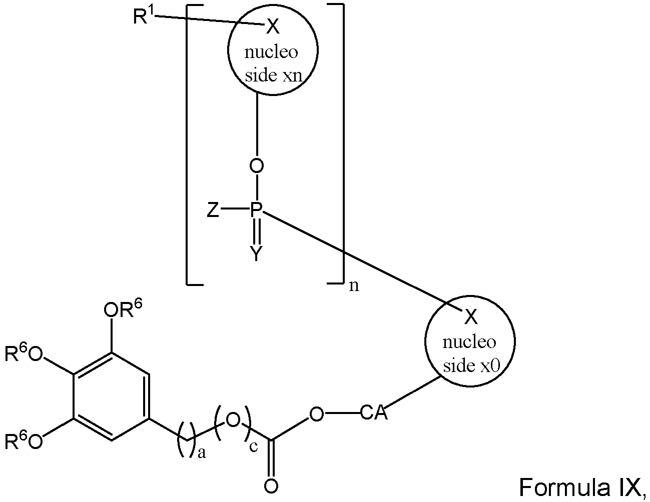
wherein in formula IX, c is 0 or 1; a is an integer of 1 to 3; each of R⁶ is independently a C8-C40 aliphatic hydrocarbon group; R¹ is a protecting group; X is independently at each position O or N; n is an integer equal to or larger than 0;
Z is independently at each position H or an electron withdrawing group; Y is independently for each repetitive unit n missing or O or S; each of the nucleosides x0 to xn may be the same or different; and CA is a capping moiety or a single bond; 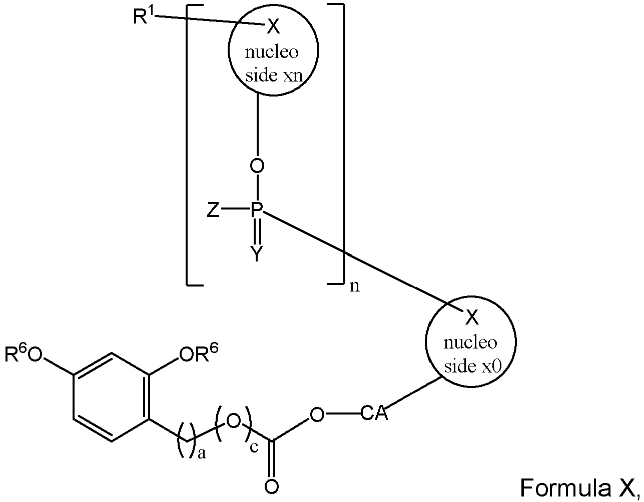
wherein in formula X, c is 0 or 1; a is an integer of 2 to 3; each of R⁶ is independently a C12-C40 aliphatic hydrocarbon group; R¹ is a protecting group;
X is independently at each position O or N; n is an integer equal to or larger than 0; Z is independently at each position H or an electron withdrawing group; Y is independently for each repetitive unit n missing or O or S; each of the nucleosides x0 to xn may be the same or different; and CA is a capping moiety or a single bond; 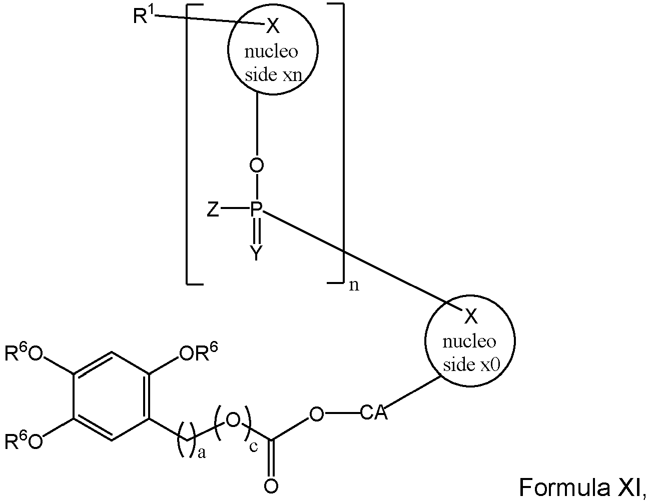
wherein in formula XI, c is 0 or 1; a is an integer of 2 to 3; each of R⁶ is independently a C8-C40 aliphatic hydrocarbon group; R¹ is a protecting group; X is independently at each position O or N; n is an integer equal to or larger than 0;
Z is independently at each position H or an electron withdrawing group; Y is independently for each repetitive unit n missing or O or S ; each of the nucleosides x0 to xn may be the same or different; and CA is a capping moiety or a single bond; and 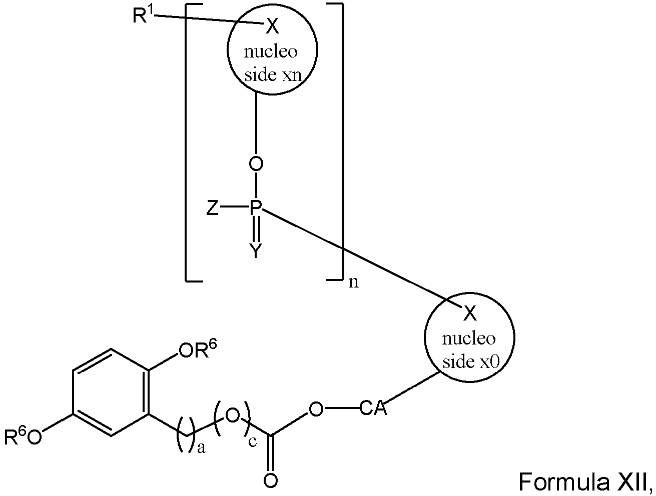
wherein in formula XII, c is 0 or 1; a is an integer of 1 to 3; each of R⁶ is independently a C12-C40 aliphatic hydrocarbon group, which optionally comprises one or more heteroatoms; R¹ is a protecting group; X is independently at each position O or N; n is an integer equal to or larger than 0; Z is independently at each position H or an electron withdrawing group; Y is independently for each repetitive unit n missing or O or S; each of the nucleosides x0 to xn may be the same or different; and CA is a capping moiety or a single bond.

8. The compound according to any one of claims 5 to 7, wherein c is 0.

9. A method for the synthesis of oligonucleotides, comprising steps a) through f):
a) providing a nucleoside, nucleotide, oligonucleotide, or conjugate of a nucleoside, nucleotide or oligonucleotide bound to a pseudo solid phase protecting group according to any one of claims 1 to 4, wherein the nucleoside, nucleotide, oligonucleotide, or conjugate thereof comprises a backbone hydroxyl or amine moiety, which is protected by a protecting group R¹;
b) cleaving the protecting group R¹ from the compound provided in the previous step, thereby generating a free backbone hydroxyl or amine group;
c) reacting the free backbone hydroxyl or amine group generated in step b) with a phosphorus moiety of a nucleoside or oligonucleotide building block, which building block further comprises a backbone hydroxyl or amine moiety protected by a protecting group R¹, thereby producing a covalent linkage between the oxygen or nitrogen atom of said free backbone hydroxyl or amine group and the phosphorus atom of said building block;
d) optionally modifying the phosphorus moiety;
e) optionally reiterating the sequence of steps b) to c) or b) to d); and
f) cleaving the oligonucleotide from the pseudo solid phase protecting group.

10. The method according to claim 9, wherein:
- the phosphorus moiety of the nucleoside or oligonucleotide building block used in step c) is a phosphorus (III) moiety;
- the method comprises a step d) of oxidizing or sulfurizing the phosphorus (III) atom to a phosphorus (V) atom, thereby creating the desired type of internucleoside linkage; and
- step e) comprises optionally either reiterating the sequence of steps b) through d) or reiterating the sequence of steps b) and c) before executing step d).

11. The method according to any one of claims 9 and 10, wherein step c) comprises reacting the free backbone hydroxyl group resulting from step b) with a phosphoramidite nucleoside or oligonucleotide building block so as to form a phosphite triester bond, and step d) involves oxidizing or sulfurizing the phosphite triester bond.

12. The method according to any one of claims 9 to 11, wherein step f) comprises incubating the conjugate of the pseudo solid phase protecting group and the oligonucleotide in a solution comprising a base, wherein said base is preferably selected from the group consisting of ammonia, a C1-C6-alkyl amine, and a source of hydroxide ions.

13. The method according to any one of claims 9 to 12, wherein the method further comprises the following step g):
g) isolating the support-cleaved oligonucleotide.

14. A method for preparing the compound according to claim 5, wherein c is 1, comprising the following steps i) to iii):
i) Providing a nucleoside or oligonucleotide comprising a first free backbone hydroxyl moiety, a second backbone hydroxyl or amine moiety, which is protected by a protecting group R¹, and optionally further protecting groups, which are orthogonal to R¹;
ii) Reacting the first free hydroxyl moiety with an activated carbonic acid derivative; and
iii) Reacting the product of step ii) with a compound of formula XIII 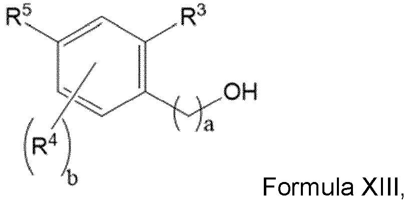 wherein:
a is an integer of 1 to 3;
R³ is H;
R⁵ is O-R⁶ or H where R⁶ is a C8-C40 aliphatic hydrocarbon group;
each of R⁴ is independently O-R⁶, where R⁶ is at each occurrence independently a C8-C40 aliphatic hydrocarbon group;
b is 1 to 3, preferably 1 to 2;
with the proviso that if b is 1, at least one of R³ and R⁵ is not H;
and with the further proviso that the sum of all carbon atoms contained in the R³, R⁴, and R⁵ moieties present is larger than 23 and smaller than 200.

15. A method for preparing the compound according to claim 5, wherein c is 0, comprising the following steps I) and II):
I) Providing a nucleoside, nucleotide, oligonucleotide, or conjugate thereof comprising a first free backbone hydroxyl moiety, a second backbone hydroxyl or amine moiety, which is protected by a protecting group R¹, and optionally further protecting groups, which are orthogonal to R¹; and
II) Reacting the compound provided in step I) with a compound of formula XIV, under conditions suitable to form an ester bond between the oxygen atom of said first free backbone hydroxyl moiety of the compound provided in step I) and the carboxyl or carbonyl carbon atom of formula XIV to which R^{L} is bonded: 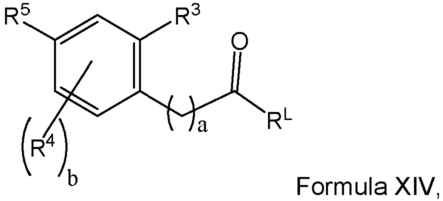 wherein in formula XIV:
R^{L} is selected from the group consisting of -OH and a leaving group capable of being substituted by a hydroxyl moiety during ester bond formation;
a is an integer of 1 to 3;
R³ is H;
R⁵ is O-R⁶ or H where R⁶ is a C8-C40 aliphatic hydrocarbon group;
each of R⁴ is independently O-R⁶, where R⁶ is at each occurrence independently a C8-C40 aliphatic hydrocarbon group;
b is 1 to 3, preferably 1 to 2;
with the proviso that if b is 1, at least one of R³ and R⁵ is not H;
and with the further proviso that the sum of all carbon atoms contained in the R³, R⁴, and R⁵ moieties present is larger than 23 and smaller than 200.

## Patentansprüche

1. Pseudo-Festphasen-Schutzgruppe zum Schutz eines Nukleosids, eines Nukleotids, eines Oligonukleotids oder eines Konjugats eines Nukleosids, eines Nukleotids oder eines Oligonukleotids, wobei die Pseudo-Festphasen-Schutzgruppe durch die Formel II dargestellt wird: 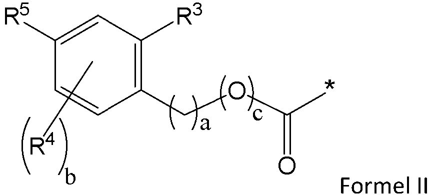 wobei:
* den Bindungspunkt an ein Sauerstoffatom einer Hydroxylgruppe des Nukleosids, des Nukleotids, des Oligonukleotids oder des Konjugats eines Nukleosids, eines Nukleotids oder eines Oligonukleotids, das geschützt werden soll, angibt;
c 0 oder 1 ist;
a eine ganze Zahl von 1 bis 3 ist;
R³ H ist;
R⁵ O-R⁶ oder H ist, wobei R⁶ eine aliphatische C8-C40-Kohlenwasserstoffgruppe ist;
jedes R⁴ unabhängig voneinander O-R⁶ ist, wobei R⁶ bei jedem Vorkommen unabhängig voneinander eine aliphatische C8-C40-Kohlenwasserstoffgruppe ist;
b 1 bis 3 ist;
mit der Maßgabe, dass, wenn b 1 ist, mindestens eines von R³ und R⁵ nicht H ist;
und mit der weiteren Maßgabe, dass die Summe aller in den vorhandenen R³ -, R⁴ - und R⁵ -Gruppen enthaltenen Kohlenstoffatome größer als 23 und kleiner als 200 ist.

2. Pseudo-Festphasenschutzgruppe gemäß Anspruch 1, wobei b eine ganze Zahl von 1 bis 2 ist.

3. Pseudo-Festphasen-Schutzgruppe gemäß einem der Ansprüche 1 und 2, die durch eine der Formeln III bis VII dargestellt wird: 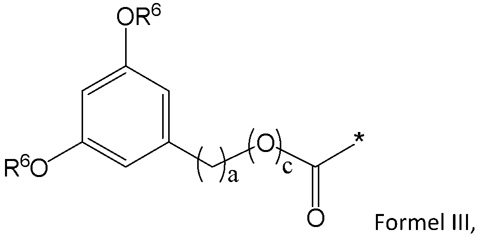 wobei in Formel III * den Bindungspunkt an das Sauerstoffatom einer zu schützenden Hydroxylgruppe angibt; c 0 oder 1 ist; a eine ganze Zahl von 1 bis 3 ist; und jedes R⁶ unabhängig voneinander eine aliphatische C12-C40-Kohlenwasserstoffgruppe ist; 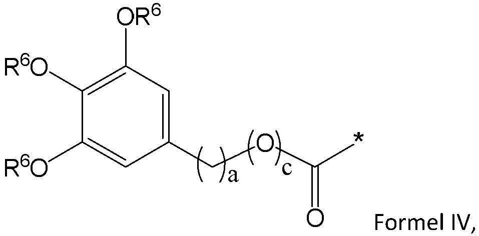 wobei in Formel IV * den Bindungspunkt an das Sauerstoffatom einer zu schützenden Hydroxylgruppe angibt; c 0 oder 1 ist; a eine ganze Zahl von 1 bis 3 ist; und jedes R⁶ unabhängig voneinander eine aliphatische C8-C40-Kohlenwasserstoffgruppe ist; 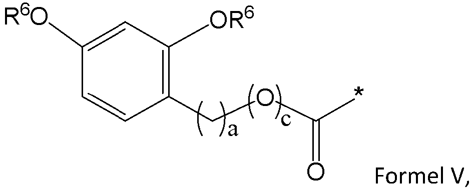 wobei in Formel V * den Bindungspunkt an das Sauerstoffatom einer zu schützenden Hydroxylgruppe angibt; c 0 oder 1 ist; a eine ganze Zahl von 2 bis 3 ist; und jedes R⁶ unabhängig voneinander eine aliphatische C12-C40-Kohlenwasserstoffgruppe ist; 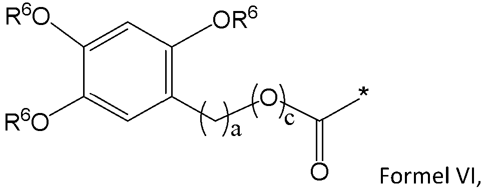 wobei in Formel VI * den Bindungspunkt an das Sauerstoffatom einer zu schützenden Hydroxylgruppe angibt; c 0 oder 1 ist; a eine ganze Zahl von 2 bis 3 ist; und jedes R⁶ unabhängig voneinander eine aliphatische C8-C40-Kohlenwasserstoffgruppe ist; und 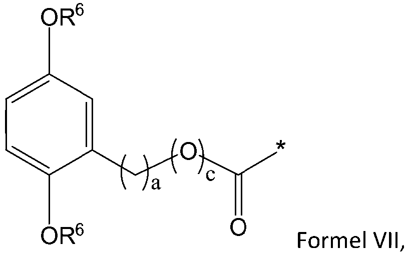 wobei in Formel VII * den Bindungspunkt an das Sauerstoffatom einer zu schützenden Hydroxylgruppe angibt; c 0 oder 1 ist; a eine ganze Zahl von 1 bis 3 ist; und jedes R⁶ unabhängig voneinander eine aliphatische C12-C40-Kohlenwasserstoffgruppe ist.

4. Pseudo-Festphasen-Schutzgruppe gemäß einem der Ansprüche 1 bis 3, wobei c 1 ist.

5. Verbindung gemäß Formel I: 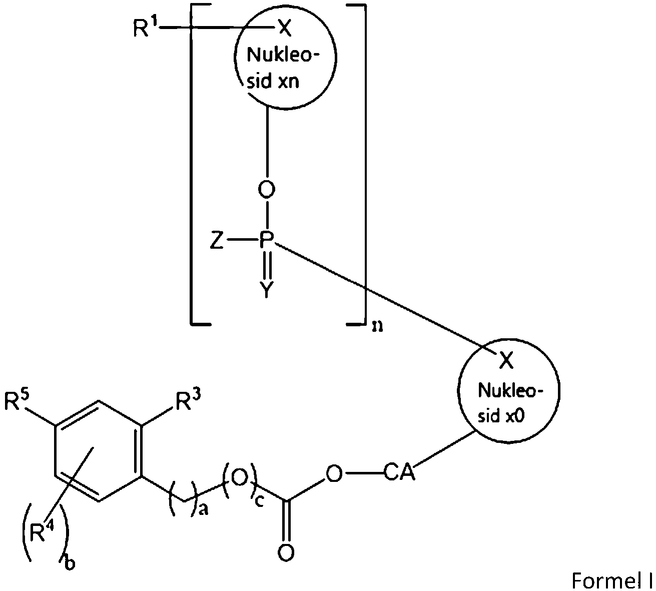 wobei:
R¹ eine Schutzgruppe ist;
X unabhängig an jeder Position O oder N ist;
n eine ganze Zahl größer oder gleich 0 ist;
Z unabhängig an jeder Position eine elektronenziehende Gruppe oder H ist;
Y unabhängig für jede sich wiederholende Einheit n fehlt oder O oder S ist;
jedes der Nukleoside x0 bis xn gleich oder unterschiedlich sein kann;
CA eine Kappengruppe oder eine Einfachbindung ist;
c 0 oder 1 ist;
a eine ganze Zahl von 1 bis 3 ist;
R³ H ist;
R⁵ O-R⁶ oder H ist, wobei R⁶ eine aliphatische C8-C40-Kohlenwasserstoffgruppe ist;
jedes R⁴ unabhängig voneinander O-R⁶ ist, wobei R⁶ bei jedem Vorkommen unabhängig voneinander eine aliphatische C8-C40-Kohlenwasserstoffgruppe ist;
b 1 bis 3 ist;
mit der Maßgabe, dass, wenn b 1 ist, mindestens eines von R³ und R⁵ nicht H ist;
und mit der weiteren Maßgabe, dass die Summe aller in den vorhandenen R³ -, R⁴ - und R⁵ -Gruppen enthaltenen Kohlenstoffatome größer als 23 und kleiner als 200 ist.

6. Verbindung gemäß Anspruch 5, wobei b eine ganze Zahl von 1 bis 2 ist.

7. Verbindung gemäß einem der Ansprüche 5 und 6, die eine Verbindung gemäß einer der Formeln VIII bis XII ist: 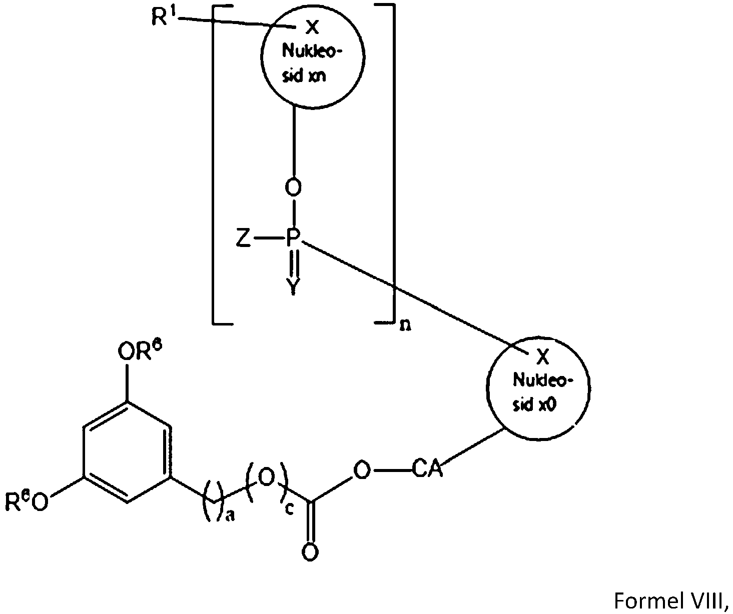
wobei in Formel VIII, c 0 oder 1 ist; a eine ganze Zahl von 1 bis 3 ist; jedes R⁶ unabhängig voneinander eine aliphatische C12-C40-Kohlenwasserstoffgruppe ist; R¹ eine Schutzgruppe ist; X unabhängig voneinander an jeder Position O oder N ist; n eine ganze Zahl größer oder gleich 0 ist; Z unabhängig an jeder Position H oder eine elektronenziehende Gruppe ist; Y unabhängig für jede sich wiederholende Einheit n fehlt oder O oder S ist; jedes der Nukleoside x0 bis xn gleich oder unterschiedlich sein kann;
und CA eine Kappengruppe oder eine Einfachbindung ist; 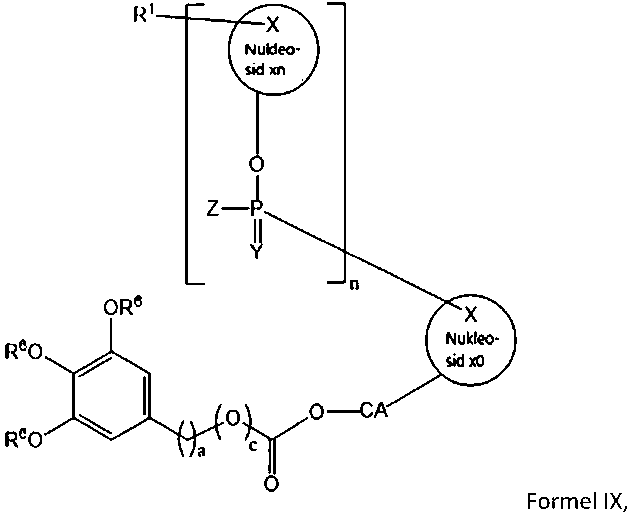
wobei in Formel IX c 0 oder 1 ist; a eine ganze Zahl von 1 bis 3 ist; jedes R⁶ unabhängig voneinander eine aliphatische C8-C40-Kohlenwasserstoffgruppe ist; R¹ eine Schutzgruppe ist; X unabhängig voneinander an jeder Position O oder N ist; n eine ganze Zahl größer oder gleich 0 ist; Z unabhängig an jeder Position H oder eine elektronenziehende Gruppe ist; Y unabhängig für jede sich wiederholende Einheit n fehlt oder O oder S ist; jedes der Nukleoside x0 bis xn gleich oder unterschiedlich sein kann;
und CA eine Kappengruppe oder eine Einfachbindung ist; 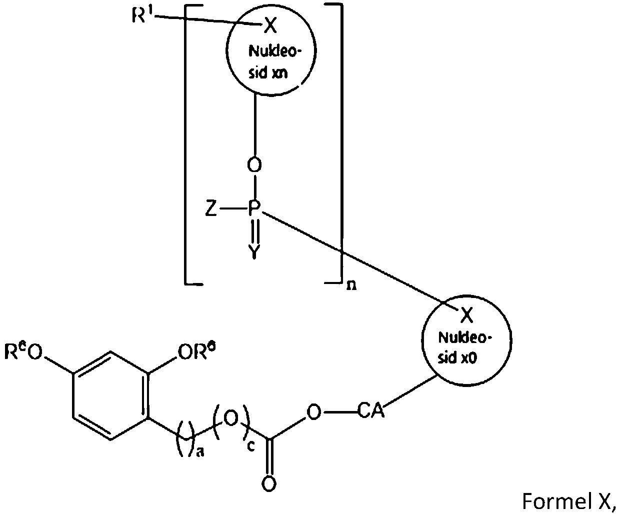
wobei in Formel X, c 0 oder 1 ist; a eine ganze Zahl von 2 bis 3 ist; jedes R⁶ unabhängig voneinander eine aliphatische C12-C40-Kohlenwasserstoffgruppe ist; R¹ eine Schutzgruppe ist; X unabhängig voneinander an jeder Position O oder N ist; n eine ganze Zahl größer oder gleich 0 ist; Z unabhängig an jeder Position H oder eine elektronenziehende Gruppe ist; Y unabhängig für jede sich wiederholende Einheit n fehlt oder O oder S ist; jedes der Nukleoside x0 bis xn gleich oder unterschiedlich sein kann;
und CA eine Kappengruppe oder eine Einfachbindung ist; 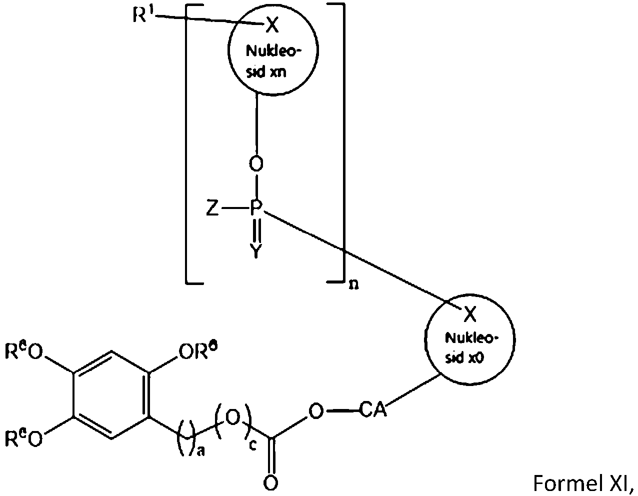
wobei in Formel XI, c 0 oder 1 ist; a eine ganze Zahl von 2 bis 3 ist; jedes R⁶ unabhängig voneinander eine aliphatische C8-C40-Kohlenwasserstoffgruppe ist; R¹eine Schutzgruppe ist; X unabhängig voneinander an jeder Position O oder N ist; n eine ganze Zahl größer oder gleich 0 ist; Z unabhängig an jeder Position H oder eine elektronenziehende Gruppe ist; Y unabhängig für jede sich wiederholende Einheit n fehlt oder O oder S ist; jedes der Nukleoside x0 bis xn gleich oder unterschiedlich sein kann;
und CA eine Kappengruppe oder eine Einfachbindung ist; und 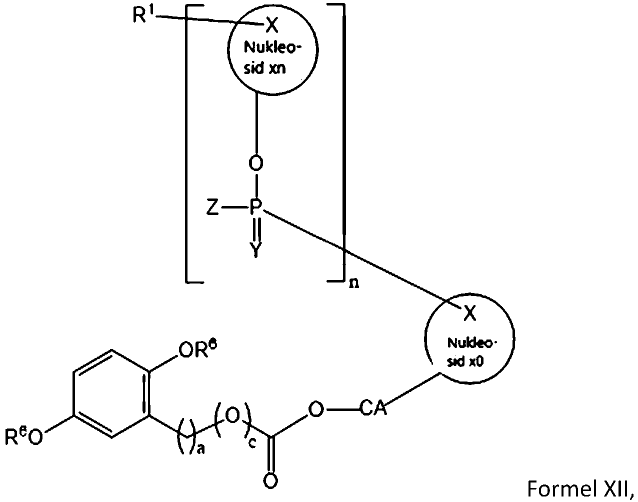
wobei in Formel XII, c 0 oder 1 ist; a eine ganze Zahl von 1 bis 3 ist; jedes R⁶ unabhängig voneinander eine aliphatische C12-C40-Kohlenwasserstoffgruppe ist, die optional ein oder mehrere Heteroatome umfasst; R¹ eine Schutzgruppe ist; X unabhängig voneinander an jeder Position O oder N ist; n eine ganze Zahl größer oder gleich 0 ist; Z unabhängig an jeder Position H oder eine elektronenziehende Gruppe ist; Y unabhängig für jede sich wiederholende Einheit n fehlt oder O oder S ist; jedes der Nukleoside x0 bis xn gleich oder unterschiedlich sein kann; und CA eine Kappengruppe oder eine Einfachbindung ist.

8. Verbindung gemäß einem der Ansprüche 5 bis 7, wobei c 0 ist.

9. Verfahren zur Synthese von Oligonukleotiden, umfassend die Schritte a) bis f):
a) Bereitstellen eines Nukleosids, Nukleotids, Oligonukleotids oder Konjugats eines Nukleosids, Nukleotids oder Oligonukleotids, das an eine pseudo-Festphasen-Schutzgruppe gemäß einem der Ansprüche 1 bis 4 gebunden ist, wobei das Nukleosid, Nukleotid, Oligonukleotid oder dessen Konjugat eine Rückgrat-Hydroxyl- oder Amin-Gruppe umfasst, die durch eine Schutzgruppe R¹ geschützt ist;
b) Abspalten der Schutzgruppe R¹ von der im vorherigen Schritt bereitgestellten Verbindung, wodurch eine freie Rückgrat-Hydroxyl- oder Amingruppe erzeugt wird;
c) Reagieren der in Schritt b) erzeugten freien Rückgrat-Hydroxyl- oder Amingruppe mit einer Phosphorgruppe eines Nukleosid- oder Oligonukleotid-Bausteins, wobei der Baustein ferner eine durch eine Schutzgruppe R¹ geschützte Rückgrat-Hydroxyl- oder Amingruppe umfasst, wodurch eine kovalente Bindung zwischen dem Sauerstoff- oder Stickstoffatom dieser freien Rückgrat-Hydroxyl- oder Amingruppe und dem Phosphoratom des Bausteins hergestellt wird;
d) optional Modifizieren der Phosphorgruppe;
e) optional Wiederholen der Schritte b) bis c) oder b) bis d); und
f) Spalten des Oligonukleotids von der pseudo-Festphasen-Schutzgruppe.

10. Verfahren nach Anspruch 9, wobei:
- die Phosphorgruppe des in Schritt c) verwendeten Nukleosid- oder Oligonukleotid-Bausteins eine Phosphor(III)-Gruppe ist;
- das Verfahren einen Schritt d) umfasst, bei dem das Phosphor(III)-Atom zu einem Phosphor(V)-Atom oxidiert oder sulfuriert wird, wodurch die gewünschte Art der Internukleosid-Bindung entsteht; und
- Schritt e) optional entweder die Wiederholung der Schritte b) bis d) oder die Wiederholung der Schritte b) und c) vor der Ausführung von Schritt d) umfasst.

11. Verfahren nach einem der Ansprüche 9 und 10, wobei Schritt c) das Umsetzen der aus Schritt b) resultierenden freien Rückgrat-Hydroxylgruppe mit einem Phosphoramidit-Nukleosid- oder Oligonukleotid-Baustein umfasst, um eine Phosphit-Triester-Bindung zu bilden, und Schritt d) das Oxidieren oder Sulfurieren der Phosphit-Triester-Bindung umfasst.

12. Verfahren nach einem der Ansprüche 9 bis 11, wobei Schritt f) das Inkubieren des Konjugats aus der pseudo-Festphasen-Schutzgruppe und dem Oligonukleotid in einer Lösung umfasst, die eine Base enthält, wobei die Base vorzugsweise aus der Gruppe ausgewählt ist, die aus Ammoniak, einem C1-C6-Alkylamin und einer Quelle für Hydroxidionen besteht.

13. Verfahren nach einem der Ansprüche 9 bis 12, wobei das Verfahren ferner den folgenden Schritt g) umfasst:
g) Isolieren des vom Träger abgespaltenen Oligonukleotids.

14. Verfahren zur Herstellung der Verbindung gemäß Anspruch 5, wobei c 1 ist, umfassend die folgenden Schritte i) bis iii):
i) Bereitstellen eines Nukleosids oder Oligonukleotids, das eine erste freie Rückgrat-Hydroxylgruppe, eine zweite Rückgrat-Hydroxyl- oder Amingruppe, die durch eine Schutzgruppe R¹ geschützt ist, und gegebenenfalls weitere Schutzgruppen, die orthogonal zu R¹ sind, umfasst;
ii) Reagieren der ersten freien Hydroxylgruppe mit einem aktivierten Carbonsäurederivat; und
iii) Reagieren des Produkts aus Schritt ii) mit einer Verbindung der Formel XIII 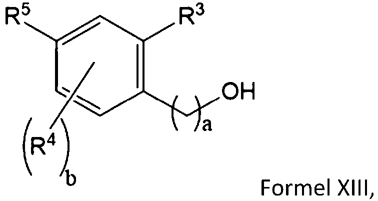 wobei:
a eine ganze Zahl von 1 bis 3 ist;
R³ H ist;
R⁵ O-R⁶ oder H ist, wobei R⁶ eine aliphatische C8-C40-Kohlenwasserstoffgruppe ist;
jedes R⁴ unabhängig voneinander O-R⁶ ist, wobei R⁶ bei jedem Vorkommen unabhängig voneinander eine aliphatische C8-C40-Kohlenwasserstoffgruppe ist;
b 1 bis 3 ist, vorzugsweise 1 bis 2;
mit der Maßgabe, dass, wenn b 1 ist, mindestens eines von R³ und R⁵ nicht H ist;
und mit der weiteren Maßgabe, dass die Summe aller in den vorhandenen R³ -, R⁴ - und R⁵ -Gruppen enthaltenen Kohlenstoffatome größer als 23 und kleiner als 200 ist.

15. Verfahren zur Herstellung der Verbindung gemäß Anspruch 5, wobei c 0 ist, umfassend die folgenden Schritte I) und II):
I) Bereitstellen eines Nukleosids, Nukleotids, Oligonukleotids oder Konjugats davon, das eine erste freie Rückgrat-Hydroxylgruppe, eine zweite Rückgrat-Hydroxyl- oder Amingruppe, die durch eine Schutzgruppe R¹ geschützt ist, und optional weitere Schutzgruppen, die orthogonal zu R¹ sind, umfasst; und
II) Reagieren der in Schritt I) bereitgestellten Verbindung mit einer Verbindung der Formel XIV unter Bedingungen, die zur Bildung einer Esterbindung zwischen dem Sauerstoffatom dieser ersten freien Rückgrat-Hydroxylgruppe der in Schritt I) bereitgestellten Verbindung und dem Carboxyl- oder Carbonylkohlenstoffatom der Formel XIV, an das R^{L} gebunden ist, geeignet sind: 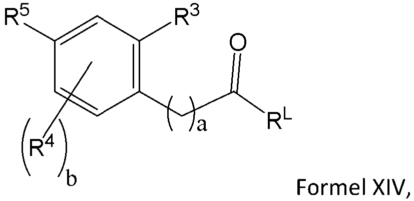 wobei in Formel XIV:
R^{L} aus der Gruppe ausgewählt ist, die aus -OH und einer Abgangsgruppe besteht, die während der Esterbindungsbildung durch eine Hydroxylgruppe substituiert werden kann;
a eine ganze Zahl von 1 bis 3 ist;
R³ H ist;
R⁵ O-R⁶ oder H ist, wobei R⁶ eine aliphatische C8-C40-Kohlenwasserstoffgruppe ist;
jedes R⁴ unabhängig voneinander O-R⁶ ist, wobei R⁶ bei jedem Vorkommen unabhängig voneinander eine aliphatische C8-C40-Kohlenwasserstoffgruppe ist;
b 1 bis 3 ist, vorzugsweise 1 bis 2;
mit der Maßgabe, dass, wenn b 1 ist, mindestens eines von R³ und R⁵ nicht H ist;
und mit der weiteren Maßgabe, dass die Summe aller in den R³, R⁴ und R⁵ -Gruppen enthaltenen Kohlenstoffatome größer als 23 und kleiner als 200 ist.

## Revendications

1. Groupe protecteur de phase pseudo solide destiné à protéger un nucléoside, un nucléotide, un oligonucléotide ou un conjugué d'un nucléoside, d'un nucléotide ou d'un oligonucléotide, dans lequel le groupe protecteur de phase pseudo solide est représenté par la formule II : 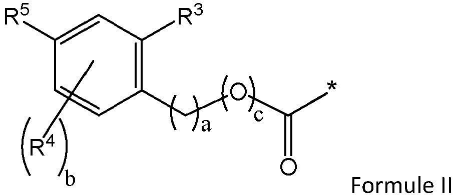 dans laquelle :
* indique le point de fixation à un atome d'oxygène d'un groupement hydroxyle du nucléoside, du nucléotide, de l'oligonucléotide, ou du conjugué d'un nucléoside, d'un nucléotide ou d'un oligonucléotide à protéger ;
c est 0 ou 1 ;
a est un entier de 1 à 3 ;
R³ est H ;
R⁵ est O-R⁶ ou H, dans lequel R⁶ est un groupement hydrocarboné aliphatique en C8-C40 ;
chacun parmi R⁴ est indépendamment O-R⁶, dans lequel R⁶ est en chaque occurrence indépendamment un groupe hydrocarboné aliphatique en C8-C40 ;
b est 1 à 3 ;
à condition que si b est 1, au moins l'un parmi R³ et R⁵ ne soit pas H ;
et à condition de plus que la somme de tous les atomes de carbone contenus dans les groupements R³, R⁴ et R⁵ présents soit supérieure à 23 et inférieure à 200.

2. Groupe protecteur de phase pseudo solide selon la revendication 1, dans lequel b est un entier de 1 à 2.

3. Groupe protecteur de phase pseudo solide selon l'une quelconque des revendications 1 et 2, qui est représenté par l'une quelconque des formules III à VII : 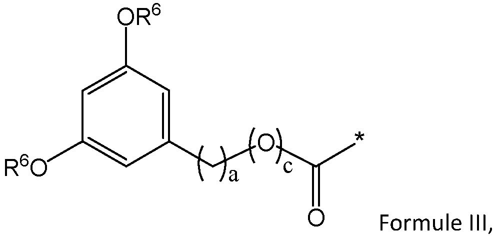 dans laquelle, dans la formule III, * indique le point de fixation à l'atome d'oxygène d'un groupement hydroxyle à protéger ; c est 0 ou 1 ; a est un entier de 1 à 3 ; et chacun parmi R⁶ est indépendamment un groupe hydrocarboné aliphatique en C12-C40 ; 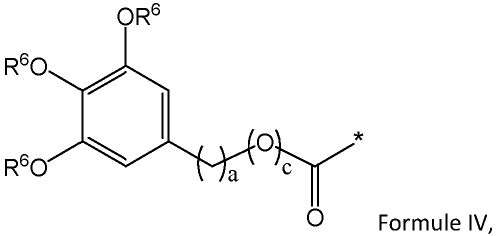 dans laquelle, dans la formule IV, * indique le point de fixation à l'atome d'oxygène d'un groupement hydroxyle à protéger ; c est 0 ou 1 ; a est un entier de 1 à 3 ; et chacun parmi R⁶ est indépendamment un groupe hydrocarboné aliphatique en C8-C40 ; 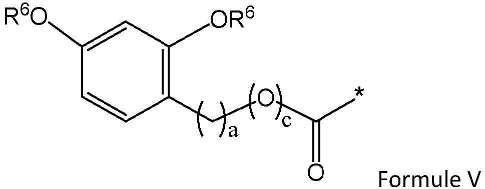 dans laquelle, dans la formule V, * indique le point de fixation à l'atome d'oxygène d'un groupement hydroxyle à protéger ; c est 0 ou 1 ; a est un entier de 2 à 3 ; et chacun parmi R⁶ est indépendamment un groupe hydrocarboné aliphatique en C12-C40 ; 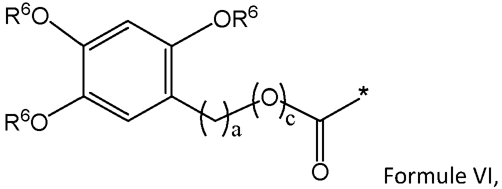 dans laquelle, dans la formule VI, * indique le point de fixation à l'atome d'oxygène d'un groupement hydroxyle à protéger ; c est 0 ou 1 ; a est un entier de 2 à 3 ; et chacun parmi R⁶ est indépendamment un groupe hydrocarboné aliphatique en C8-C40 ; et 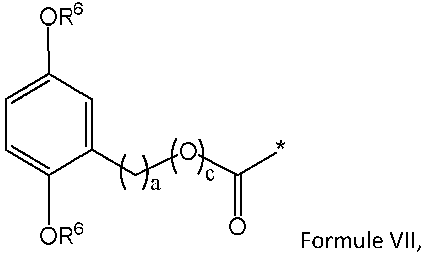 dans laquelle, dans la formule VII, * indique le point de fixation à l'atome d'oxygène d'un groupement hydroxyle à protéger ; c est 0 ou 1 ; a est un entier de 1 à 3 ; et chacun parmi R⁶ est indépendamment un groupe hydrocarboné aliphatique en C12-C40.

4. Groupe protecteur de phase pseudo solide selon l'une quelconque des revendications 1 à 3, dans lequel c est 1.

5. Composé selon la formule I : 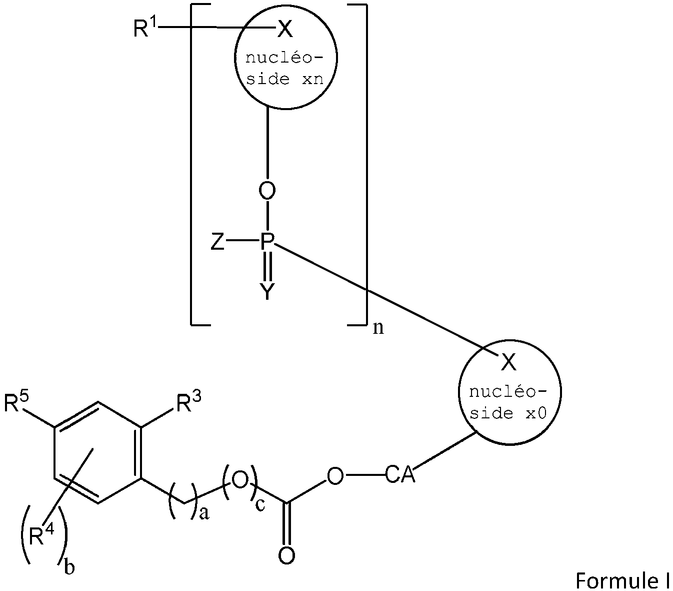 dans laquelle :
R¹ est un groupe protecteur;
X est indépendamment à chaque position O ou N ;
n est un entier égal ou supérieur à 0 ;
Z est indépendamment à chaque position un groupe électroattracteur ou H ;
Y est indépendamment, pour chaque motif répétitif n, manquant ou O ou S ;
chacun des nucléosides x0 à xn peut être identique ou différent ;
CA est un groupement coiffant ou une simple liaison ;
c est 0 ou 1 ;
a est un entier de 1 à 3 ;
R³ est H ;
R⁵ est O-R⁶ ou H, dans lequel R⁶ est un groupe hydrocarboné aliphatique en C8-C40 ;
chacun parmi R⁴ est indépendamment O-R⁶, dans lequel R⁶ est en chaque occurrence indépendamment un groupe hydrocarboné aliphatique en C8-C40 ;
b est 1 à 3 ;
à condition que si b est 1, au moins l'un parmi R³ et R⁵ ne soit pas H ;
et à condition de plus que la somme de tous les atomes de carbone contenus dans les groupements R³, R⁴ et R⁵ présents soit supérieure à 23 et inférieure à 200.

6. Composé selon la revendication 5, dans lequel b est un entier de 1 à 2.

7. Composé selon l'une quelconque des revendications 5 et 6, qui est un composé selon l'une quelconque des formules VIII à XII : 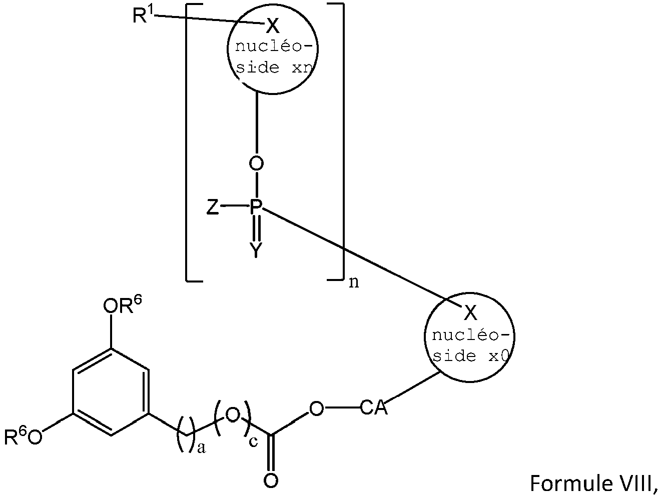 dans laquelle, dans la formule VIII, c est 0 ou 1 ; a est un entier de 1 à 3 ; chacun de R⁶ est indépendamment un groupe hydrocarboné aliphatique en C12-C40 ; R¹ est un groupe protecteur ; X est indépendamment à chaque position O ou N ; n est un entier égal ou supérieur à 0 ; Z est indépendamment à chaque position H ou un groupe électroattracteur ; Y est indépendamment, pour chaque motif répétitif n, manquant ou O ou S; chacun des nucléosides x0 à xn peut être identique ou différent ; et CA est un groupement coiffant ou une simple liaison ; 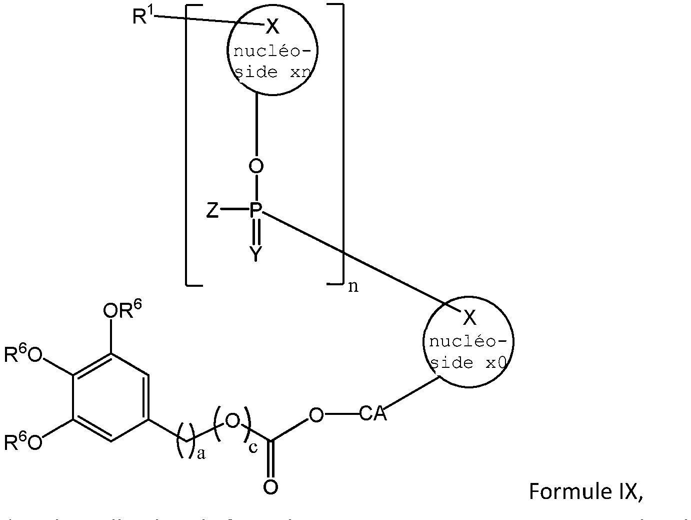 dans laquelle, dans la formule IX, c est 0 ou 1 ; a est un entier de 1 à 3 ; chacun de R⁶ est indépendamment un groupe hydrocarboné aliphatique en C8-C40 ; R¹ est un groupe protecteur ; X est indépendamment à chaque position O ou N ; n est un entier égal ou supérieur à 0 ; Z est indépendamment à chaque position H ou un groupe électroattracteur ; Y est indépendamment, pour chaque motif répétitif n, manquant ou O ou S ; chacun des nucléosides x0 à xn peut être identique ou différent ; et CA est un groupement coiffant ou une simple liaison ; 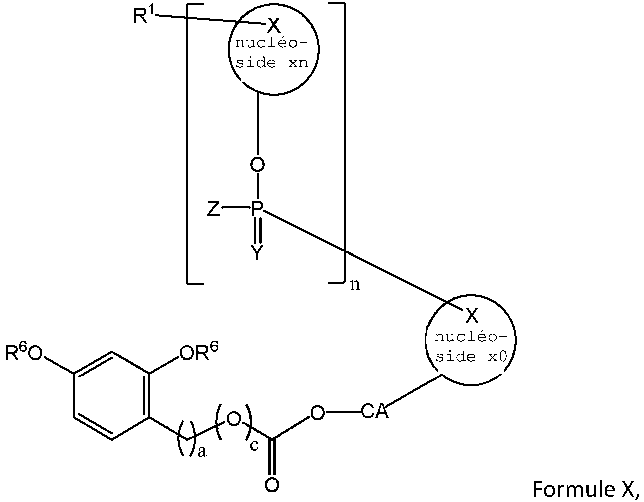 dans laquelle, dans la formule X, c est 0 ou 1 ; a est un entier de 2 à 3 ; chacun des R⁶ est indépendamment un groupe hydrocarboné aliphatique en C12-C40 ; R¹ est un groupe protecteur ; X est indépendamment à chaque position O ou N ; n est un entier égal ou supérieur à 0 ; Z est indépendamment à chaque position H ou un groupe électroattracteur ; Y est indépendamment, pour chaque motif répétitif n, manquant ou O ou S ; chacun des nucléosides x0 à xn peut être identique ou différent ; et CA est un groupement coiffant ou une simple liaison ; 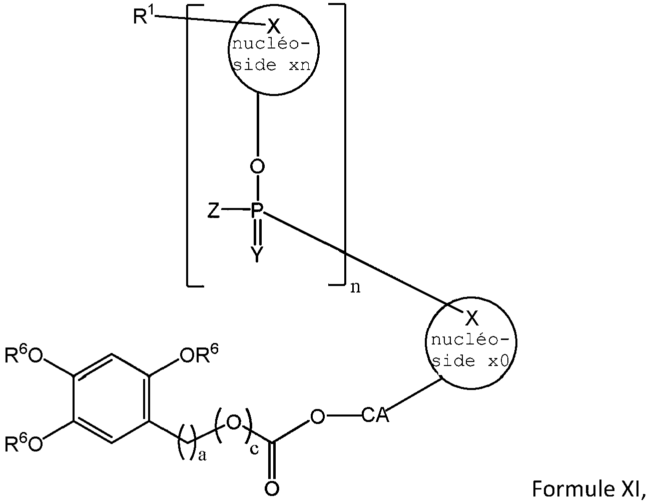 dans laquelle, dans la formule XI, c est 0 ou 1 ; a est un entier de 2 à 3 ; chacun des R⁶ est indépendamment un groupe hydrocarboné aliphatique en C8-C40 ; R¹ est un groupe protecteur ; X est indépendamment à chaque position O ou N ; n est un entier égal ou supérieur à 0 ; Z est indépendamment à chaque position H ou un groupe électroattracteur ; Y est indépendamment, pour chaque motif répétitif n, manquant ou O ou S; chacun des nucléosides x0 à xn peut être identique ou différent ; et CA est un groupement coiffant ou une simple liaison ; et 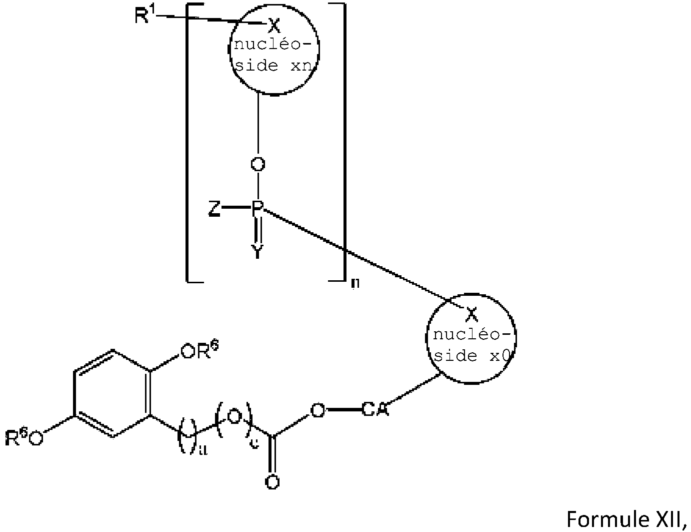 dans laquelle, dans la formule XII, c est 0 ou 1 ; a est un entier de 1 à 3 ; chacun des R⁶ est indépendamment un groupe hydrocarboné aliphatique en C12-C40, qui comprend éventuellement un ou plusieurs hétéroatomes ; R¹ est un groupe protecteur ; X est indépendamment à chaque position O ou N ; n est un entier égal ou supérieur à 0 ; Z est indépendamment à chaque position H ou un groupe électroattracteur; Y est indépendamment, pour chaque motif répétitif n, manquant ou O ou S ; chacun des nucléosides x0 à xn peut être identique ou différent ; et CA est un groupement coiffant ou une simple liaison.

8. Composé selon l'une quelconque des revendications 5 à 7, dans lequel c est 0.

9. Procédé de synthèse d'oligonucléotides comprenant les étapes a) à f) :
a) fourniture d'un nucléoside, nucléotide, oligonucléotide ou conjugué d'un nucléoside, nucléotide ou oligonucléotide lié à un groupe protecteur de phase pseudo solide selon l'une quelconque des revendications 1 à 4, dans lequel le nucléoside, nucléotide, oligonucléotide ou conjugué correspondant comprend un groupement hydroxyle ou amine de squelette, qui est protégé par un groupe protecteur R¹ ;
b) clivage du groupe protecteur R¹ du composé fourni dans l'étape précédente, de façon à générer un groupe hydroxyle ou amine de squelette libre ;
c) mise en réaction du groupe hydroxyle ou amine de squelette libre généré dans l'étape b) avec un groupement phosphore d'un bloc de construction de nucléoside ou oligonucléotide, lequel bloc de construction comprend en outre un groupement hydroxyle ou amine de squelette protégé par un groupe protecteur R¹, produisant ainsi une liaison covalente entre l'atome d'oxygène ou d'azote dudit groupe hydroxyle ou amine de squelette libre et l'atome de phosphore dudit bloc de construction ;
d) éventuellement modification du groupement phosphore ;
e) éventuellement réitération de la séquence des étapes b) à c) ou b) à d) ; et
f) clivage de l'oligonucléotide du groupe protecteur de phase pseudo solide.

10. Procédé selon la revendication 9, dans lequel :
- le motif groupement du bloc de construction de nucléoside ou d'oligonucléotide utilisé à l'étape c) est un groupement phosphore (III) ;
- le procédé comprend une étape d) d'oxydation ou de sulfuration de l'atome de phosphore (III) en un atome de phosphore (V), ce qui crée le type souhaité de liaison internucléosidique ; et
- l'étape e) comprend optionnellement soit la réitération de la succession d'étapes b) à d), soit la réitération de la succession d'étapes b) et c) avant d'exécuter l'étape d).

11. Procédé selon l'une quelconque des revendications 9 et 10, dans lequel l'étape c) comprend la mise en réaction du groupe hydroxyle de squelette libre résultant de l'étape b) avec un bloc de construction de nucléoside ou oligonucléotide phosphoramidite de manière à former une liaison triester de phosphite, et l'étape d) implique l'oxydation ou la sulfuration de la liaison triester de phosphite.

12. Procédé selon l'une quelconque des revendications 9 à 11, dans lequel l'étape f) comprend l'incubation du conjugué du groupe protecteur de phase pseudo solide et de l'oligonucléotide dans une solution comprenant une base, ladite base étant de préférence choisie dans le groupe constitué par l'ammoniac, une C1-C6-alkylamine et une source d'ions hydroxyde.

13. Procédé selon l'une quelconque des revendications 9 à 12, dans lequel le procédé comprend en outre l'étape g) suivante :
g) isolement de l'oligonucléotide clivé par un support.

14. Procédé de préparation du composé selon la revendication 5, dans lequel c est 1, comprenant les étapes i) à iii) suivantes :
i) mise à disposition d'un nucléoside ou oligonucléotide comprenant un premier groupement hydroxyle de squelette libre, un deuxième groupement hydroxyle ou amine de squelette, qui est protégé par un groupe protecteur R¹, et éventuellement d'autres groupes protecteurs, qui sont orthogonaux par rapport à R¹ ;
ii) mise en réaction du premier groupement hydroxyle libre avec un dérivé d'acide carbonique activé ; et
iii) mise en réaction du produit de l'étape ii) avec un composé de formule XIII 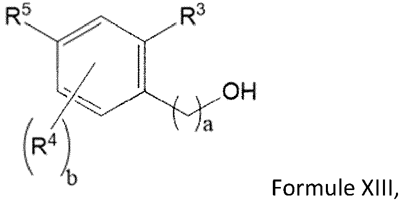 dans laquelle :
a est un entier de 1 à 3 ;
R³ est H ;
R⁵ est O-R⁶ ou H, dans lequel R⁶ est un groupe hydrocarboné aliphatique en C8-C40 ;
chacun parmi R⁴ est indépendamment O-R⁶, dans lequel R⁶ est en chaque occurrence indépendamment un groupe hydrocarboné aliphatique en C8-C40 ;
b est 1 à 3, de préférence 1 à 2 ;
à condition que si b est 1, au moins l'un parmi R³ et R⁵ ne soit pas H ;
et à condition de plus que la somme de tous les atomes de carbone contenus dans les groupements R³, R⁴ et R⁵ présents soit supérieure à 23 et inférieure à 200.

15. Procédé de préparation du composé selon la revendication 5, dans lequel c est 0, comprenant les étapes I) et II) suivantes :
I) fourniture d'un nucléoside, nucléotide, oligonucléotide ou conjugué correspondant comprenant un premier groupement hydroxyle de squelette libre, un deuxième groupement hydroxyle ou amine de squelette, qui est protégé par un groupe protecteur R¹, et éventuellement d'autres groupes protecteurs, qui sont orthogonaux par rapport à R¹ ; et
II) mise en réaction du composé fourni dans l'étape i) avec un composé de formule XIV, dans des conditions adaptées pour former une liaison ester entre l'atome d'oxygène dudit premier groupement hydroxyle de squelette libre du composé fourni dans l'étape I) et l'atome de carbone carboxyle ou carbonyle de formule XIV auquel R^{L} est lié : 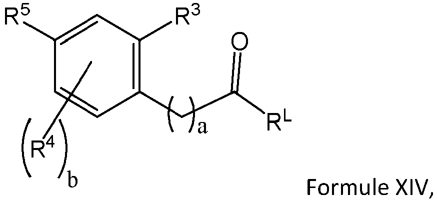 dans laquelle dans la formule XIV :
R^{L} est choisi dans le groupe constitué par -OH et un groupe partant pouvant être substitué par un groupement hydroxyle pendant la formation de liaison ester;
a est un entier de 1 à 3 ;
R³ est H ;
R⁵ est O-R⁶ ou H, dans lequel R⁶ est un groupe hydrocarboné aliphatique en C8-C40 ;
chacun parmi R⁴ est indépendamment O-R⁶, dans lequel R⁶ est en chaque occurrence indépendamment un groupe hydrocarboné aliphatique en C8-C40 ;
b est 1 à 3, de préférence 1 à 2 ;
à condition que si b est 1, au moins l'un parmi R³ et R⁵ ne soit pas H ;
et à condition de plus que la somme de tous les atomes de carbone contenus dans les groupements R³, R⁴ et R⁵ présents soit supérieure à 23 et inférieure à 200.
